# EUROPEAN PATENT APPLICATION

(11) **EP 3 881 830 A1**
(43) Date of publication of application: **22.09.2021**
(21) Application number: 21154438.2
(22) Date of filing: 03.04.2015
(51) Int. Cl.: A61K 9/14, A61K 38/08, A61P 3/00

(54) **METHODS OF TREATING CELIAC DISEASE WITH LARAZOTIDE**

(30) Priority: 04.04.2014 US 201461975128 P
(62) Divisional of application: 15772716.5
(71) Applicant: Alba Therapeutics Corporation, Baltimore, MD 21202 (US)
(72) Inventor: PERROW, Wendy, Baltimore, MD Maryland 21202 (US); PEACOCK, Bruce, Baltimore, MD Maryland 21202 (US)
(74) Representative: Grund, Martin

(57) **Abstract**

The present invention provides compositions comprising Larazotide. The invention further provides methods of using the Larazotide compositions for treating celiac disease in symptomatic celiac disease patients.

## Description

### RELATED APPLICATIONS

This application claims the benefit of U.S. Provisional Patent Application No. 61/975,128, filed April 4, 2014, the entire disclosure of which is hereby incorporated by reference in its entirety.

### FIELD OF THE INVENTION

The present invention provides compositions and methods for treating celiac disease. Particularly, the present invention provides methods for treating symptomatic or active celiac disease, including patients that are non-responsive to gluten free diet or patients having refractory celiac disease.

### BACKGROUND

Celiac disease is a genetic autoimmune disease triggered by the ingestion of gluten (a protein in wheat, rye, and barley). Individuals with celiac disease have increased intestinal permeability, which allows gluten break-down products (the triggering antigens of celiac disease) to reach gut-associated lymphoid tissue, thus initiating an inflammatory response including inflammatory cytokine release and T-cell recruitment. Celiac disease is characterized by chronic inflammation of the small intestinal mucosa that may result in atrophy of the small intestinal villi and diverse symptoms, such as malabsorption, diarrhea, abdominal pain, bloating, and nausea. In addition, celiac disease is also associated with neurological and psychiatric disorders including cerebella ataxia, brain atrophy, peripheral neuropathy, epilepsy, cognitive-function deterioration, depression, and anxiety. Altogether, these symptoms result in a significant reduction in the quality of life for celiac disease patients.

No pharmacological therapies are currently available for the treatment of celiac disease. Rather, the only treatment option for celiac disease patients is a life-long adherence to a gluten-free diet (GFD). However, changes in dietary habits are difficult to maintain, and entirely gluten-free food products are not widely available. As such, inadequate dietary compliance is common among celiac disease patients. Long-term consequences for poor dietary compliance include the risk of developing osteoporosis, stomach, esophageal, or colon cancer, and T cell lymphoma. In addition, the continuous gastrointestinal symptoms often result in significant morbidity with a substantial reduction in the quality of life for celiac disease patients. Further still, about 10% of patients diagnosed with celiac disease do not get better on a gluten-free diet, and thus are non-responsive to GFD, or have refractory celiac disease.

Accordingly, there remains an urgent need for effective pharmacological therapies for the treatment of celiac disease.

### SUMMARY OF THE INVENTION

The present invention provides methods for treating celiac disease, including in conjunction with a gluten-free diet (GFD). The invention provides methods for treating symptomatic or active celiac disease, despite reasonable or substantial compliance with a GFD. In some embodiments, the invention provides methods of treating patients having refractory celiac disease or celiac disease that is non-reponsive to a GFD. The methods comprise administering a pharmaceutical composition comprising Larazotide or salt thereof. Larazotide is a peptide agent that promotes GI tight junction integrity. When administered to the GI, for example, in an amount in the range of about 0.25 mg to about 1 mg of Larazotide, symptomatic, active, refractory, and/or non-responsive celiac disease is reduced or ameliorated.

As disclosed herein, Larazotide is safe and effective for prolonged use, and in various embodiments, celiac disease symptoms (including GI symptoms, abdominal symptoms, and non-GI symptoms) continue to decline with prolonged use of Larazotide. For example, in various embodiments, the composition is administered to the celiac disease patient about 2 or 3 times per day for at least about 8 weeks. In an embodiment, the composition is administered for at least about 9 weeks, at least about 10 weeks, or at least about 12 weeks, or more, such as for at least about 6 months or for at least about 1 year. The composition may be administered prior to meals, to promote tight junction integrity in the GI in case of unintentional exposure to gluten or intentional exposure of small amounts of gluten.

In various embodiments, at the start of treatment with Larazotide the celiac disease patient may be experiencing one or more classes of symptoms, such as abdominal domain symptoms, diarrhea and loose stools domain symptoms, nausea domain symptoms, gastrointestinal domain symptoms, and non-GI domain symptoms, despite being on a GFD. In some embodiments, the patient experiences non-GI domain symptoms together with abdominal domain symptoms, diarrhea and loose stools symptoms, nausea symptoms, and/or GI domain symptoms, despite reasonable or substantial compliance with a GFD. Exemplary celiac symptoms include a plurality of abdominal cramping, abdominal pain, bloating, gas, diarrhea, loose stools, nausea, vomiting, indigestion, reflux, constipation, vomiting, headache, and tiredness. In some embodiments, the celiac disease patient is experiencing dermatitis herpetiformis. Prior to treatment, the patient may experience at least about 2, or at least about 3, at least about 4, at least about 5, at least about 6, or at least about 7 CeD PRO symptomatic days per week, which is reduced by one or more days upon treatment with Larazotide for at least about 8 weeks. For example, upon treatment with Larazotide the patient experiences one, two or more CeD PRO improved symptom days (or non-symptomatic days) per week, and over time symptoms may substantially or entirely subside.

Other aspects and embodiments of the invention will be apparent from the following detailed description.

### DESCRIPTION OF THE FIGURES

Figure 1 shows the timeline for a clinical study involving administration of 0.5 mg, 1.0 mg, or 2.0 mg of Larazotide acetate three times daily (TID). The study was conducted in 3 phases: a 4-week single-blind, placebo run-in phase; a 12-week double-blind, randomized phase; and a 4-week single-blind, run-out phase.
Figure 2 shows that a significant percentage of celiac disease patients on a gluten-free diet reported CeD PRO symptoms during the placebo run-in period (week 3).
Figures 3A (MITT population) and 3B (Per-Protocol population) show that Larazotide acetate significantly reduced gastrointestinal symptoms in celiac disease patients as measured by the CeD GSRS score.
Figure 4 shows that Larazotide acetate significantly reduced gastrointestinal symptoms in celiac disease patients as measured by a change from baseline to the end of treatment in CeD GSRS score.
Figures 5A (MITT population) and 5B (Per-Protocol population) show the CeD Pro Abdominal Domain score of celiac patients treated with placebo, 0.5 mg, 1.0 mg, or 2.0 mg TID of Larazotide acetate.
Figures 6A (MITT population) and 6B (Per-Protocol population) show the CeD Pro Gastrointestinal Domain score of celiac patients treated with placebo, 0.5 mg, 1.0 mg, or 2.0 mg TID of Larazotide acetate.
Figure 7 shows that Larazotide acetate significantly reduced gastrointestinal symptoms in celiac disease patients as measured by the total GSRS score.
Figure 8 shows that 0.5 mg of Larazotide acetate TID significantly reduced gastrointestinal symptoms as measured by individual domains of the total GSRS score (*i.e.,* Diarrhea syndrome, Indigestion syndrome, Constipation syndrome, Abdominal Pain syndrome, and Reflux Pain syndrome).
Figure 9 shows that Larazotide acetate reduced the number of bowel movements in celiac disease patients.
Figure 10 shows that Larazotide acetate reduced the number of diarrhea/loose stools BSFS scores of 5-7.
Figure 11 shows that Larazotide acetate reduced the baseline of weekly average of CeD PRO abdominal domain score for ≥ 6 out of 12 weeks of treatment.
Figure 12 shows that Larazotide acetate reduced the baseline of weekly average of CeD GSRS score for ≥ 6 out of 12 weeks of treatment.
Figures 13A (MITT population) and 13B (Per Protocol population) show that Larazotide acetate increased the average weekly number of CeD PRO improved symptom days in celiac disease patients.
Figures 14A (MITT population), 14B (Per Protocol population), and 14C (MITT and Per Protocol populations) show that Larazotide acetate reduced the average weekly number of CeD PRO symptomatic days in celiac disease patients.
Figure 15 shows that Larazotide acetate decreased the average CeD PRO non-gastrointestinal domain score in celiac disease patients.
Figures 16A (MITT population) and 16B (Per Protocol population) show the effects of Larazotide acetate treatment on the CeD PRO overall wellbeing score.

### DETAILED DESCRIPTION

The present invention provides methods for treating celiac disease, including patients on gluten-free diet (GFD), including patients that are significantly symptomatic despite reasonable or substantial compliance with a GFD. In accordance with the invention, patients are treated with Larazotide or salt thereof (e.g., acetate salt). Larazotide is a peptide agent that promotes tight junction integrity in the GI. Larazotide has the amino acid sequence: Gly Gly Val Leu Val Gln Pro Gly (SEQ ID NO:1), and can be formulated for targeted release in affected portions of the GI (e.g., small intestine and/or large intestine), including one or more of the duodenum, jejunum, and ileum.

Celiac disease patients can be identified, diagnosed or confirmed, for example, by measuring the serum levels of anti-endomysial antibody, anti-tissue transglutaminase antibody (anti-tTG), and/or anti-deamidated gliadin peptide (anti-DGP). Celiac patients may also be diagonosed, for example, by small bowel biopsy and/or capsule endoscopy. In addition, patients can be screened for genes encoding the human leukocyte antigens HLA-DQ2 and HLA-DQ8, which are statistically associated with celiac disease.

In various embodiments, the celiac disease patient may not be reasonably or substantially compliant with a gluten-free diet, meaning that gluten exposure is not merely of an unintentional nature. In accordance with the invention, the celiac symptoms of these patients can be reduced, ameliorated, or prevented, thereby allowing for some GFD non-compliance. In alternative embodiments, the celiac disease patient is reasonably or substantially compliant with a gluten-free diet, meaning that any significant gluten exposure is inadvertent or infrequent. For example, in some embodiments, prior to treatment with Larazotide the celiac disease patient has been on a GFD for at least about 1 week, at least about 2 weeks, at least about 3 weeks, at least about 4 weeks, at least about 1 month, at least about 2 months, at least about 3 months, at least about 4 months, at least about 5 months, or at least about 6 months. In some embodiments, the celiac disease patient has been on a GFD for at least about 1 year, at least about 2 years, at least about 3 years, at least about 4 years, or at least about 5 years. Despite being on a GFD for a length of time, and despite reasonable compliance with the GFD, the patient may still experience celiac symptoms as described herein. Larazotide in conjunction with the GFD can reduce these symptoms, even when these symptoms are significant or substantial, and/or when even non-GI symptoms are experienced or prevalent.

In various embodiments, the celiac disease is non-responsive to GFD. Patients with non-responsive celiac disease do not exhibit a histological response to a gluten-free diet. Such patients continue to exhibit small-bowel mucosal villous atrophy during a gluten-free diet which is diagnosed by intestinal biopsy. See Pulido et al., (2013) Can J. Gastroenterol, 27(8):449-453 and Spatoloa et al., (2014) Aliment Pharmacol Ther. 39(4): 407-417, the entire contents of which are hereby incorporated by reference. The most common reason for this lack of recovery despite being on gluten-free diet is continuing gluten ingestion (intentional and unintentional), because gluten is present in many foods and medications. For example, the patient may exhibit a high sensitivity to gluten. Other potential reasons include, for example, the development of other complications such as irritable bowel syndrome, small bowel bacterial overgrowth, other food intolerances such as lactose intolerance, microscopic colitis, Crohn's disease, ulcerative colitis, and pancreative digestive enzyme insufficiency.

Accordingly, in some embodiments, the present invention treats celiac disease patients who continue to be exposed to gluten, intentionally and/or unintentionally. In some embodiments, the celiac disease patient is non-responsive to a GFD. In some embodiments, the patient is determined to have irritable bowel syndrome, small bowel bacterial overgrowth, or other food intolerances such as lactose intolerance, gastroesophageal reflux, microscopic colitis, Crohn's disease, ulcerative colitis, and pancreative digestive enzyme insufficiency. In further embodiments, the present invention reduces symptoms in celiac disease patients having, for example, extra-intestinal diseases and/or conditions including, but not limited to, dermatitis herpetiformis, diabetes (Type 1 and 2), autoimmune thyroid disease, anemia, dental-enamel hypoplasia/tooth discoloration, osteopenia or osteoporosis, abnormal liver function tests, joint pain and/or join disease, and recurrent miscarriages or fertility problems. These non-GI conditions may be ameliorated, avoided, or managed in part with a Larazotide regimen as described herein.

In various embodiments, the present invention provides methods for treating celiac disease patients having refractory celiac disease. Refractory celiac disease is defined by persistent or recurrent malabsorptive symptoms and damaged intestinal architecture despite strict adherence to a gluten-free diet for at least six to twelve months in the absence of other causes of non-responsive celiac disease and overt malignancy. See Spatoloa et al., (2014) Aliment Pharmacol Ther. 39(4): 407-417, Rubio-Tapia et al., Gut, 59(4):547-557, and Semrad (2008) Impact, 8(3): 1-3, the entire contents of which are hereby incorporated by reference. Some of these patients never respond to a gluten-free diet while others initially respond but have a recurrence of symptoms and intestinal inflammation. Most patients with refractory celiac disease have persistent diarrhea, abdominal pain, malabsorption, and involuntary weight loss in addition to vitamin and mineral deficiencies, anemia, fatigue, and malaise. Refractory celiac disease is divided into two types. Type I patients exhibit normal T cell population in the intestinal lining and are conventionally treated with aggressive nutritional support as well as pharmacologic therapies including steroids. In contrast, type II patients show abnormal T-cell population in the intestinal lining. These patients have a poor prognosis as they respond poorly to steroid treatment and have a high chance of developing severe complications such as enteropathy-associated T-cell lymphoma (EATL) and ulcerative jejunitis. Accordingly, in some embodiments, the present invention provides methods for treating celiac disease patient with type I refractory celiac disease. In embodiments where the patient has non-responsive or refractory celiac disease, the patient may undergo an adjunct therapy. Exemplary adjunct therapy includes treatment with any of the additional therapeutic agents as described herein. For example, the celiac disease patient may undergo an adjunct therapy involving an anti-inflammatory such as steroid treatment (e.g., prednisone, budesonide, prednisolone, etc.) or NSAID treatment. Alternatively, the celiac disease patient may undergo adjunct therapy with immunosuppressants and other biological modifiers such as azathioprine, cyclosporin, infliximab and alemtuzumab treatment. Alternatively, the celiac patient may undergo therapy with an antibiotic to control bacterial overgrowth in the GI. Alternatively or in addition, the patient may undergo treatment with a probiotic.

In various embodiments, the celiac disease patient is experiencing symptoms, despite substantial compliance with GFD, or due to non-compliance. While symptoms may be determined by the attending physician through examination/interview of the patient, there are various tools for quantifying or evaluating a patient's symptoms, well-being, and GFD compliance, which may also be employed. These include, but are not limited to, Celiac Disease Patient Reported Outcome (CeD PRO), Gastrointestinal Symptom Rating Scale (GSRS), Celiac Disease Gastrointestinal Symptom Rating Scale (CeD GSRS), Bristol Stool Form Scale (BSFS), General Well-Being Questionnaire, Short Form 12 Health Survey Version 2 (SF12V2), Celiac Disease Quality of Life Questionnaire (CeD-QoL), and Clinician Global Assessment of Disease Activity (CGA), all of which are further described in Example 1. Adherence to gluten-free diet may be assessed by, for example, Celiac Dietary Adherence Test (CDAT) and Gluten-Free Diet Compliance Questionnaire (GFDCQ), both of which are further described in Example 1. Accordingly, in some embodiments, the celiac disease patients are experiencing one or more symptoms as measured by one of the scales described herein.

In an embodiment, the celiac disease patient is experiencing one or more symptoms as measured by CeD PRO at the start of treatment with Larazotide. The CeD PRO questionnaire was developed to assess symptom severity in clinical trials in subjects with celiac disease. Items in the questionnaire were formulated based on one-on-one interviews with subjects with celiac disease and thus reflect the symptoms that subjects consider part of their celiac disease experience. The CeD PRO includes 12 items asking participants about the severity of celiac disease symptoms they experience each day. Subjects rate their symptom severity on an 11-point, 0 to 10 scale; from "not experiencing the symptom" to "the worst possible symptom experience". The CeD PRO included a Gastrointestinal Domain scale consisting of all the gastrointestinal symptoms (abdominal cramping, abdominal pain, bloating, constipation, diarrhea, gas, loose stools, nausea, vomiting), and a Non-gastrointestinal Domain scale which include items such as headache and tiredness. Each domain score is calculated by summing the value of the individual items within a scale and averaging across the number of items within the scale. Higher scores reflect greater symptom severity.

In an embodiment, the celiac disease patient is experiencing one or more CeD PRO abdominal domain symptoms selected from abdominal pain, abdominal cramping, bloating or gas. In an embodiment, the celiac disease patient is experiencing one or more CeD PRO Gastrointestinal (GI) domain symptoms selected from abdominal cramping, abdominal pain, bloating, gas, diarrhea, loose stools, nausea, indigestion, reflux, constipation, and vomiting. In an embodiment, the celiac disease patient is experiencing one or more of diarrhea and loose stools. In an embodiment, the celiac disease patient is experiencing nausea. In an embodiment, the celiac disease patient is experiencing constipation. In an embodiment, the celiac disease patient is experiencing vomiting. In various embodiments, the celiac disease patient is experiencing one or more CeD PRO Non-Gastrointestinal domain symptoms including, but not limited to, headaches and tiredness. The patient may be experiencing one or more CeD PRO Non-GI domain symptoms, with one or more CeD PRO abdominal domain or CeD PRO GI domain symptoms.

In various embodiments, the celiac disease patient is experiencing one or more significant or severe symptomatic days (based on CeD PRO) at the start of treatment with Larazotide. A CeD PRO Symptomatic day can include abdominal domain and GI domain symptoms, and/or non-GI domain symptoms. A CeD PRO Symptomatic day is defined as a day where the mean of the set of symptoms is scored as ≥ 2.5 out of a 0 to 10 scale, or ≥ 3 out of a 0 to 10 scale. In an embodiment, the celiac disease patient is experiencing one or more CeD PRO Symptomatic days of at least 2, 3, 4, 5, 6, or 7. CeD PRO abdominal domain symptoms are abdominal pain, abdominal cramping, bloating or gas. CeD PRO GI domain symptoms and/or one or more CeD PRO GI domain symptoms are abdominal cramping, abdominal pain, bloating, gas, diarrhea, loose stools, nausea, indigestion, reflux, constipation, and vomiting. CeD PRO non-GI domain symptoms include headache or tiredness. An additional non-GI symptom can include dermatitis herpetiformis.

In various embodiments, the celiac disease patient is experiencing one or more symptoms as rated by the Gastrointestinal Symptom Rating Scale (GSRS) at the start of treatment with Larazotide. The GSRS, as further described in Example 1, is a 15-question, 7-scale questionnaire to assess 5 dimensions of gastrointestinal syndromes: diarrhea, indigestion, constipation, abdominal pain, and reflux. The questionnaire was originally constructed to measure symptoms in subjects with irritable bowel syndrome and peptic ulcer. In some embodiments, the patient scores at least 2, or at least 3, or at least 4, or at least 5, or at least 6, or 7 on the Gastrointestinal Symptom Rating Scale (GSRS) at the start of treatment with Larazotide using the 1-7 Likert scale, with 1 representing the most positive option and 7 the most negative option.

In various embodiments, the celiac disease patient is experiencing one or more symptoms as rated by the Celiac Disease Gastrointestinal Symptom Rating Scale (CeD GSRS) at the start of treatment with Larazotide. The CeD GSRS dimensions, as described in the Example 1, measures a subset of the GSRS with dimensions more applicable to celiac disease. The CeD GSRS dimensions include 10 questions in the following domains: Diarrhea syndrome; Indigestion syndrome; and Abdominal Pain syndrome. In some embodiments, the patient scores at least 2, or at least 3, or at least 4, or at least 5, or at least 6 or at least 7 on the CeD Gastrointestinal Symptom Rating Scale (CeD GSRS) at the start of treatment with Larazotide using the 1-7 Likert scale, with 1 representing the most positive option and 7 the most negative option.

In various embodiments, the celiac disease patient is experiencing diarrhea and loose bowel movements at the start of treatment with Larazotide. In an embodiment, the celiac disease patient is experiencing at least about 3, about 4, about 5, about 6, about 7, about 8, about 9, about 10, or more bowel movements per day at the start of treatment with Larazotide. In an embodiment, the celiac disease patient is experiencing more than about 12, more than about 14, more than about 16, more than about 18 bowel movements per day at the start of treatment with Larazotide.

As described in Example 1, the Bristol Stool Form Scale (BSFS) is a pictorial aid to help patients identify the shape and consistency of their bowel movements. For example, the BSFS differentiates stools into seven types:
- Type 1: Separate hard lumps, like nuts (hard to pass)
- Type 2: Sausage-shaped, but lumpy
- Type 3: Like a sausage but with cracks on its surface
- Type 4: Like a sausage or snake, smooth and soft
- Type 5: Soft blobs with clear cut edges (passed easily)
- Type 6: Fluffy pieces with ragged edges, a mushy stool
- Type 7: Watery, no solid pieces, entirely liquid

Types 1-2 indicate constipation, with 3 and 4 being the ideal stools (especially the latter), as they are easy to defecate while not containing any excess liquid, and 5, 6 and 7 tending towards diarrhea. In various embodiments, the celiac disease patient is experiencing at least about 4, at least about 5, at least about 6, at least about 7, or at least about 8, or at least about 9, or at least about 10 diarrhea or loose stools per day on the Bristol Form Scale (BSFS) at the start of treatment Larazotide. In an embodiment, the celiac disease patient is experiencing ≥ 3 diarrhea or loose stools per day with a score of 5-7 as measured by the Bristol Form Scale (BSFS) at the start of treatment with Larazotide.

In various embodiments, administration of Larazotide effectively improves the symptoms and sense of well-being of celiac disease patients. Improvements can be assessed using the various scales as described herein, or be determined by the attending physician by patient evaluation. For example, improvements in symptoms and sense of well-being may be evaluated by, but not limited to, CeD PRO, GSRS, CeD GSRS, BSFS, General Well-Being Question, SF12V2, CeD-QoL, and CGA scores.

In various embodiments, administration of Larazotide results in a reduction of the CeD GSRS score, that is, administration of Larazotide results in a reduction in symptoms as measured by a change from baseline in CeD GSRS score. For example, administration of Larazotide may reduce the CeD GSRS score by at least about 20 %, at least about 30 %, at least about 40 %, at least about 50 %, at least about 60 %, at least about 70 %, at least about 75 %, at least about 80 %, at least about 85 %, at least about 90 %, at least about 95 %, or about 100 %.

In various embodiments, administration of Larazotide results in a reduction of the CeD PRO abdominal domain score. In an embodiment, administration of Larazotide results in a reduction in symptoms as measured by a change from baseline in CeD PRO abdominal domain score. For example, administration of Larazotide may reduce the CeD PRO abdominal domain score by at least about 20 %, at least about 30 %, at least about 40 %, at least about 50 %, at least about 60 %, at least about 70 %, at least about 75 %, at least about 80 %, at least about 85 %, at least about 90 %, at least about 95 %, or about 100 %.

In various embodiments, administration of Larazotide results in a reduction of the CeD PRO gastrointestinal domain score. For example, administration of Larazotide may reduce the CeD PRO gastrointestinal domain score by at least about 20 %, at least about 30 %, at least about 40 %, at least about 50 %, at least about 60 %, at least about 70 %, at least about 75 %, at least about 80 %, at least about 85 %, at least about 90 %, at least about 95 %, or about 100 %.

In various embodiments, administration of Larazotide results in a reduction of the CeD PRO Non-Gastrointestinal domain score (headache and tiredness). For example, administration of Larazotide may reduce the CeD PRO Non-Gastrointestinal domain score by at least about 20 %, at least about 30 %, at least about 40 %, at least about 50 %, at least about 60 %, at least about 70 %, at least about 75 %, at least about 80 %, at least about 85 %, at least about 90 %, at least about 95 %, or about 100 %.

In various embodiments, administration of Larazotide results in an increase in CeD PRO Improved Symptom days. A CeD PRO Improved Symptom day is a day where the mean of abdominal cramping, abdominal pain, bloating, and gas is scored as ≤ 1.5 out of a 0 to 10 scale, and a day where the mean of diarrhea and loose stool is scored as ≤ 1.5 out of a 0 to 10 scale, and a day where nausea is scored as ≤ 1 out of a 0 to 1 0 scale. For example, administration of Larazotide increases the number of (e.g., average weekly number of) CeD PRO Improved Symptom days by at least about 1, 2, 3, 4, 5, 6, or 7 days.

In various embodiments, administration of Larazotide results in the patient experiencing a reduction in CeD PRO Symptomatic days (e.g., average weekly number of) CeD PRO Symptomatic days by at least about 1, 2, 3, 4, 5, 6, or 7 days.

In various embodiments, administration of Larazotide results in a reduction of the total GSRS score. In an embodiment, administration Larazotide results in a reduction of the the GSRS score in one or more of the individual domains including diarrhea syndrome, indigestion syndrome, constipation syndrome, abdominal pain syndrome, and reflux syndrome. For example, administration of Larazotide may reduce the total GSRS score (including one or more of the individual domain scores) by at least about 20 %, at least about 30 %, at least about 40 %, at least about 50 %, at least about 60 %, at least about 70 %, at least about 75 %, at least about 80 %, at least about 85 %, at least about 90 %, at least about 95 %, or about 100 %.

In various embodiments, administration of Larazotide results in a reduction of the number of bowel movements. In an embodiment, administration of the pharmaceutical composition of the invention results in a reduction of the average on-treatment number of weekly bowel movements with BSFS scores of 5 to 7 (diarrhea and loose stools). For example, administration of Larazotide may reduce the number of bowel movements by at least about 20 %, at least about 30 %, at least about 40 %, at least about 50 %, at least about 60 %, at least about 70 %, at least about 75 %, at least about 80 %, at least about 85 %, at least about 90 %, at least about 95 %, or about 100 %. In another example, administration of Larazotide may reduce the number of bowel movements per week by at least about 2, at least about 3, at least about 5, at least about 10, at least about 15, or at least about 20.

In various embodiments, administration of Larazotide results in an improvement of symptoms and well-being in the celiac disease patient as measured by the average on-treatment General Well-Being Question score.

In various embodiments, administration of Larazotide results in an improvement of symptoms and well-being in the celiac disease patient as measured by the SF12V2 Questionnaire.

In various embodiments, administration of Larazotide results in an improvement of symptoms and well-being in the celiac disease patient as measured by the Celiac Disease - Quality of Life Questionnaire (CeD-QoL).

In various embodiments, administration of Larazotide results in an improvement of symptoms and well-being in the celiac disease patient as measured by the Clinician Global Assessment of Disease Activity (CGA).

Larazotide may be administered in any suitable form, including as a salt. For example, Larazotide may be administered as the acetate salt. Salts of Larazotide, including the acetate salt and hydrochloride salt, are described in US 2013/0281384, which is hereby incorporated by reference in its entirety. Alternative salts may be employed, including any pharmaceutically acceptable salt of the peptide such as those listed in Journal of Pharmaceutical Science, 66, 2-19 (1977) and The Handbook of Pharmaceutical Salts; Properties, Selection, and Use. P. H. Stahl and C. G. Wermuth (eds.), Verlag, Zurich (Switzerland) 2002, which are hereby incorporated by reference in their entirety.

Larazotide can be administered in unit dosage forms (e.g., tablets or capsules). As shown herein, patients experiencing substantial celiac disease symptoms, as well as patients identified as being non-responsive to GFD or having refractory celiac disease, can show considerable improvement even on low doses of Larazotide. For example, Larazotide (or salt thereof) can be administered at from about 0.1 mg to about 2 mg, or at from about 0.25 mg to about 1 mg, or at from about 0.5 mg to about 1 mg, or at from about 0.25 to about 0.75 mg. Exemplary unit doses include about 0.1 mg, about 0.2 mg, about 0.25 mg, about 0.3 mg, about 0.4 mg, about 0.5 mg, about 0.6 mg, about 0.7 mg, about 0.75 mg, about 0.8 mg, about 0.9 mg, about 1 mg, about 1.25 mg, about 1.5 mg, about 1.75 mg, or about 2 mg.

In accordance with certain embodiments of the invention, Larazotide is administered more than once daily to promote GI tight junction integrity. For example, Larazotide may be administered about two times daily, about three times daily, about four times daily, or about five times daily. In an embodiment, the pharmaceutical composition is administered about three times daily. In some embodiments, Larazotide is administered prior to meals, simultaneously with meals, or after meals, to reduce the effects of gluten exposure. In an embodiment, Larazotide is administered prior to meals, such as about 15 minutes prior to meals. In such embodiments, Larazotide is administered about 2 hours, about 90 minutes, about 60 minutes, about 55 minutes, about 45 minutes, about 40 minutes, about 35 minutes, about 30 minutes, about 25 minutes, about 20 minutes, about 15 minutes, about 10 minutes, about 9 minutes, about 8 minutes, about 7 minutes, about 6 minutes, about 5 minutes, about 4 minutes, about 3 minutes, about 2 minutes, or about 1 minute prior to meals.

In another embodiment, Larazotide is administered after meals. In such embodiments, Larazotide is administered about 2 hours, about 90 minutes, about 60 minutes, about 55 minutes, about 45 minutes, about 40 minutes, about 35 minutes, about 30 minutes, about 25 minutes, about 20 minutes, about 15 minutes, about 10 minutes, about 9 minutes, about 8 minutes, about 7 minutes, about 6 minutes, about 5 minutes, about 4 minutes, about 3 minutes, about 2 minutes, or about 1 minute after meals.

In some embodiments, Larazotide may be administered for a prolonged period. Continuous Larazotide regimens can exhibit improving symptoms over time. For example, Larazotide may be administered as described herein for at least about 1 week, at least about 2 weeks, at least about 3 weeks, at least about 4 weeks, at least about 5 weeks, at least about 6 weeks, at least about 7 weeks, at least about 8 weeks, at least about 9 weeks, at least about 10 weeks, at least about 11 weeks, at least about 12 weeks, or at least about 26 weeks. For example, Larazotide may be administered for at least about 8 weeks, at least about 9 weeks, at least about 10 weeks, at least about 11 weeks, or at least about 12 weeks. In some embodiments, the Larazotide is administered for at least about 1 month, at least about 2 months, at least about 3 months, at least about 4 months, at least about 5 months, at least about 6 months, at least about 7 months, at least about 8 months, at least about 9 months, at least about 10 months, at least about 11 months, or at least about 12 months. For example, the Larazotide is administered for at least about 6 months. In some embodiments, the Larazotide may be administered for at least about 1 year, at least about 2 years, at least about 3 years, at least about 4 years, or at least about 5 years. For example, the Larazotide may be administered for at least about 1 year.

In some embodiments, Larazotide compositions are administered to a subject by contacting the mucosal tissues of the gastrointestinal tract. For example, Larazotide may be formulated for delivery to one or more of the small intestine and large intestine. By targeting release of Larazotide in the affected region(s) (e.g. duodenum, jejunum and ileum, colon transversum, colon descendens, colon ascendens, colon sigmoidenum and cecum), tight junction integrity at any portion of the GI can be improved.

In various embodiments, the pharmaceutical composition may be formulated to have a delayed-release profile, i.e. not immediately release the active ingredient(s) upon ingestion; rather, postponement of the release of the active ingredient(s) until the composition is lower in the gastrointestinal tract; for example, for release in the small intestine (e.g., one or more of duodenum, jejunum, ileum) or the large intestine (e.g., one or more of cecum, ascending, transverse, descending or sigmoid portions of the colon). In an embodiment, the pharmaceutical composition is formulated to have a delayed-release profile as described in, for example, U.S. Patent No. 8,168,594, the entire contents of which are hereby incorporated by reference.

For example, Larazotide may be administered to the duodenum of the patient, as an oral dosage, delayed-release composition that contains Larazotide-coated beads that are stable in gastric fluid and unstable in intestinal fluid so as to substantially release the peptide in the duodenum. The composition may further comprise a second population of beads with a pH-dependent coating to affect release of the peptide in the jejunum of the patient. For example, the second population of beads may release the Larazotide about 30 minutes after the beads releasing peptide in the duodenum. The oral dosage composition can be in the form of a capsule or tablet. The pH-dependent coating in some embodiments is a 1:1 co-polymer of methacrylic acid and ethyl acrylate, wherein the thickness of the layer determines the release profile of each bead. The beads may have one or more additional coatings such as a base coat, a separating layer, and an overcoat layer.

In an exemplary oral dosage composition, an effective amount of Larazotide (e.g., as the acetate salt) is provided in first delayed-release particles that are capable of releasing Larazotide in the duodenum of a patient, and second delayed release particles that are capable of releasing Larazotide in the jejunum of a patient. Each particle has a core particle, a coat comprising Larazotide over the core particle, and a delayed-release coating (e.g., a 1:1 co-polymer of acrylate and methacrylate) outside the coat comprising Larazotide. Whereas the first delayed-release particles release at least 70% of the Larazotide in the first delayed-release particles by about 60 minutes of exposure to simulated intestinal fluid having a pH of greater than 5; the second delayed-release particles release at least 70% of the Larazotide by about 30 and about 90 minutes of exposure to simulated intestinal fluid having a pH of greater than 5.

For patients that may have additional symptoms of ulcerative colitis and/or Crohn's disease. Or other large intestinal symptoms, beads may further be formulated for segments of the large intestine, including the colon. See US Patent 8,796,203, which is hereby incorporated by reference in its entirety.

Generally, the delayed-release coating may degrade as a function of time without regard to the pH and/or presence of enzymes. Such a coating may comprise, for example, a water insoluble polymer. Its solubility is therefore independent of the pH. The term "pH independent" as used herein means that the permeability of the polymer and its ability to release pharmaceutical ingredients is not a function of pH and/or is only very slightly dependent on pH. Such coatings may be used to prepare, for example, sustained release formulations. Suitable water insoluble polymers include, but are not limited to, cellulose ethers, cellulose esters, or cellulose ether-esters, i.e., a cellulose derivative in which some of the hydroxy groups on the cellulose skeleton are substituted with alkyl groups and some are modified with alkanoyl groups. Examples include ethyl cellulose, acetyl cellulose, nitrocellulose, and the like. Other examples of insoluble polymers include, but are not limited to, lacquer, and acrylic and/or methacrylic ester polymers, polymers or copolymers of acrylate or methacrylate having a low quaternary ammonium content, or mixture thereof and the like. Other examples of insoluble polymers include EUDRAGIT RS®, EUDRAGIT RL®, and EUDRAGIT NE®. Insoluble polymers useful in the present invention include, for example, polyvinyl esters, polyvinyl acetals, polyacrylic acid esters, butadiene styrene copolymers, and the like.

In some embodiments, such as for patients having non-responsive or refractory celiac disease or IBS, the patient may receive adjunct therapy, which in some embodiments is synergistic with Larazotide treatment. In some embodiments, the additional therapeutic agent is an anti-inflammatory agent such as steroidal anti-inflammatory agents or nonsteroidal anti-inflammatory agents (NSAIDs). Steroids, particularly the adrenal corticosteroids and their synthetic analogues, are well known in the art. Examples of corticosteroids include, without limitation, hydroxyltriamcinolone, alpha-methyl dexamethasone, beta-methyl betamethasone, beclomethasone dipropionate, clobetasol valerate, desonide, desoxymethasone, dexamethasone, diflorasone diacetate, diflucortolone valerate, fluadrenolone, fluclorolone acetonide, flumethasone pivalate, fluosinolone acetonide, fluocinonide, flucortine butylester, fluocortolone, fluprednidene (fluprednylidene) acetate, flurandrenolone, halcinonide, methylprednisolone, triamcinolone acetonide, cortisone, cortodoxone, flucetonide, fludrocortisone, difluorosone diacetate, fluradrenolone acetonide, medrysone, amcinafel, amcinafide, betamethasone and the balance of its esters, chloroprednisone, clocortelone, clescinolone, dichlorisone, difluprednate, flucloronide, flunisolide, fluoromethalone, fluperolone, fluprednisolone, hydrocortisone, meprednisone, paramethasone, prednisolone, prednisone, and budesonide. (NSAIDS) that may be used in the present invention, include but are not limited to, salicylic acid, acetyl salicylic acid, methyl salicylate, glycol salicylate, salicylmides, benzyl-2,5-diacetoxybenzoic acid, ibuprofen, fulindac, naproxen, ketoprofen, etofenamate, phenylbutazone, and indomethacin.

In an embodiment, the additional therapeutic agent is an immunosuppressive agent such as azathioprine, cyclosporin, infliximab, and alemtuzumab.

In some embodiments, the additional therapeutic agent is an antidiarrheal agent. Antidiarrheal agents suitable for use in the present invention include, but are not limited to, DPP-IV inhibitors, natural opioids, such as tincture of opium, paregoric, and codeine, synthetic opioids, such as diphenoxylate, difenoxin and loperamide, bismuth subsalicylate, lanreotide, vapreotide and octreotide, motiln antagonists, COX2 inhibitors like celecoxib, glutamine, thalidomide and traditional antidiarrheal remedies, such as kaolin, pectin, berberine and muscarinic agents.

In some embodiments, the additional therapeutic agent is an antibacterial agent such as an antibiotic. Antibiotics suitable for use in the present invention include, but are not limited to, cephalosporin antibiotics (cephalexin, cefuroxime, cefadroxil, cefazolin, cephalothin, cefaclor, cefamandole, cefoxitin, cefprozil, and ceftobiprole); fluoroquinolone antibiotics (cipro, Levaquin, floxin, tequin, avelox, and norflox); tetracycline antibiotics (tetracycline, minocycline, oxytetracycline, and doxycycline); penicillin antibiotics (amoxicillin, ampicillin, penicillin V, dicloxacillin, carbenicillin, vancomycin, and methicillin); monobactam antibiotics (aztreonam); and carbapenem antibiotics (ertapenem, doripenem, imipenem/cilastatin, and meropenem).

In some embodiments, the additional therapeutic agent is a probiotic. Probiotics suitable for use in the present invention include, but are not limited to, Saccharomyces boulardii; Lactobacillus rhamnosus GG; Lactobacillus plantarum 299v; Clostridium butyricum M588; Clostridium difficile VP20621 (non-toxigenic C. difficile strain); combination of Lactobacillus casei, Lactobacillus acidophilus (Bio-K + CL1285); combination of Lactobacillus casei, Lactobacillus bulgaricus, Streptococcus thermophilus (Actimel); combination of Lactobacillus acidophilus, Bifidobacterium bifidum (Florajen3); combination of Lactobacillus acidophilus, Lactobacillus bulgaricus delbrueckii subsp. bulgaricus, Lactobacillus bulgaricus casei, Lactobacillus bulgaricus plantarum, Bifidobacterium longum, Bifidobacterium infantis, Bifidobacterium breve, and Streptococcus salivarius subsp.thermophilus (VSL#3)). I

### EXAMPLES

### Example 1. A Phase IIb Study for Evaluating the Efficacy and Safety of Larazotide Acetate in Treating Celiac Disease

This example describes an outpatient, randomized, parallel-group, double-blind, placebo controlled, multicenter, phase IIb study that was conducted to evaluate the efficacy and safety of Larazotide acetate for the treatment of subjects with celiac disease. Table 1 below provides a list of the abbreviations used in this example.

**Table 1. List of Abbreviations**

| **Abbreviation** | **Term** |
|---|---|
| AE(s) | adverse event(s) |
| ALT | alanine aminotransferase |
| ANCOVA | analysis of covariance |
| anti-DGP | anti-deamidated gliadin peptide |
| anti-tTG | anti-tissue transglutaminase |
| AST | aspartate aminotransferase |
| AT-1001 | previous identification code name for Larazotide acetate |
| βhCG | beta human chorionic gonadotropin |
| BMI | body mass index |
| BSFS | Bristol Stool Form Scale |
| BUN | blood urea nitrogen |
| CDAT | Celiac Dietary Adherence Test |
| CeD GSRS | Celiac Disease domains of the Gastrointestinal Symptoms Rating Scale |
| CeD PRO | Celiac Disease Patient Reported Outcome |
| CeD-QoL | Celiac Disease Quality of Life |
| CGA | Clinical Global Assessment |
| EATL | enteropathy-associated T cell lymphoma |
| ECG | electrocardiogram |
| eCRF | electronic case report form |
| FDA | Food and Drug Administration |
| GFD | gluten-free diet |
| GFDCQ | Gluten-Free Diet Compliance Questionnaire |
| GSRS | Gastrointestinal Symptoms Rating Scale |
| HIV | human immunodeficiency virus |
| ICH | International Conference on Harmonisation |
| IEC | Independent Ethics Committee |
| IgA | immunoglobulin A |
| IgG | immunoglobulin G |
| iDMC | independent Data Monitoring Committee |
| IRB | Institutional Review Board |
| IV | intravenous(ly) |
| IVRS | Interactive Voice Response System |
| IWRS | Interactive Web Response System |
| LLN | lower limit of normal |
| MedDRA | Medical Dictionary for Regulatory Activities |
| MITT | modified intent-to-treat |
| MMRM | mixed model for repeated measures |
| NSAID | nonsteroidal anti-inflammatory drug |
| RCD | refractory celiac disease |
| SF12V2 | Short Form 12 health survey, Version 2 |
| TEAE | Treatment-emergent adverse event |
| TID | three times daily |
| ULN | upper limit of normal |
| US | United States |
| WHO-DD | World Health Organization - Drug Dictionary |

### Study Objectives:

The primary objectives of the study were to assess the efficacy of 3 different doses of Larazotide acetate (0.5 mg, 1 mg, and 2 mg three times daily (TID)) versus placebo for the treatment of celiac disease in adults as an adjunct to a gluten-free diet (GFD).

The secondary objectives of the study were to validate a Celiac Disease Patient Reported Outcome (CeD PRO) diary instrument in subjects with celiac disease (Abdominal Domain, Gastrointestinal Domain, Diarrhea and Loose Stools Domain, Constipation Domain, Nausea Domain, Vomiting Domain, and Non-Gastrointestinal Domain); to compare various efficacy endpoints during 12 weeks of double-blind treatment with different doses of Larazotide acetate and placebo relative to baseline (start of treatment); and to assess the safety and tolerability of Larazotide acetate in subjects with active celiac disease.

### Study Endpoints:

The primary endpoint was the average on-treatment score of the Celiac Disease Gastrointestinal Symptom Rating Scale (CeD GSRS). Baseline was defined as the CeD GSRS score collected at randomization (Day 0/Week 0; CeD GSRS completion date for Week 0). A comparison was made between each of the 3 doses of Larazotide acetate versus placebo throughout the 12 treatment weeks.

Secondary endpoints included:
- Change from baseline to the end of treatment in the CeD GSRS score
- Average on-treatment scores of the CeD PRO Abdominal Domain (abdominal pain, abdominal cramping, bloating, and gas)
- Change from baseline to the end of treatment in the CeD PRO Abdominal Domain
- Average on-treatment scores of the CeD PRO Gastrointestinal Domain (abdominal pain, abdominal cramping, bloating, gas, diarrhea, and loose stools)
- Change from baseline to the end of treatment in the CeD PRO Gastrointestinal Domain

Exploratory endpoints included:
- Average on-treatment total GSRS score
- Average on-treatment score for individual domains (scores) of the total GSRS
- Average on-treatment number of weekly bowel movements (all Bristol Stool Form Scale [BSFS] scores) as recorded in the BSFS daily diary
- Average on-treatment number of weekly bowel movements with BSFS scores of 5 to 7
- Subjects with ≥ 10%, ≥ 20%, ≥ 30%, and ≥ 50% reduction from baseline in the weekly averages of the CeD PRO Abdominal Domain scores for ≥ 6 weeks out of 12 weeks of treatment
- Subjects with ≥ 10%, ≥ 20%, ≥ 30%, and ≥ 50% reduction from baseline in the weekly averages of the CeD PRO Gastrointestinal Domain scores for ≥ 6 weeks out of 12 weeks of treatment
- Subjects with ≥ 10%, ≥ 20%, ≥ 30%, and ≥ 50% reduction from baseline in the weekly on-treatment CeD GSRS scores for ≥ 6 weeks out of 12 weeks of treatment
- Subjects with ≥ 1, ≥ 2, or ≥ 3 bowel movement reductions from baseline in the weekly number of diarrhea and loose stools (defined as a BSFS score of 5 to 7) for ≥ 6 weeks out of 12 weeks of treatment
- Average on-treatment weekly number of CeD PRO Improved Symptom days (defined as the Abdominal Domain score (mean of abdominal cramping, abdominal pain, bloating, gas) ≤ 1.5; AND Diarrhea and Loose Stools Domain score (mean of diarrhea, loose stools) ≤ 1.5)
- Average on-treatment weekly number of CeD PRO Symptomatic days (defined as the Abdominal Domain score (mean of abdominal cramping, abdominal pain, bloating, gas) ≥ 2.5 or ≥ 3; OR Diarrhea and Loose Stools Domain score (mean of diarrhea, loose stools) ≥ 2.5 or ≥ 3)
- Average on-treatment scores of the CeD PRO Non-Gastrointestinal Domain (defined as headache and tiredness)
- Average on-treatment scores of the CeD PRO Nausea Domain (nausea), Constipation Domain (constipation), and Vomiting Domain (vomiting)
- Clinician Global Assessment (CGA Assessment 1 and Assessment 2)
- General Well-Being Question
- Anti-tTG (IgA and IgG)
- Anti-deamidated gliadin peptide (DGP; IgA and IgG)
- Short Form 12 health survey, Version 2 (SF12V2)
- Celiac Disease Quality of Life Questionnaire (CeD-QoL)

Safety endpoints included:
- Frequency and intensity of treatment and non-treatment-emergent adverse events and serious adverse events
- Vital signs
- Physical examination
- Electrocardiogram (ECG)
- Clinical laboratory test results
- Concomitant treatment

### Methods:

The clinical study included 4 arms: Larazotide acetate 0.5 mg, 1 mg, 2 mg, or placebo administered TID, 15 minutes prior to each meal.

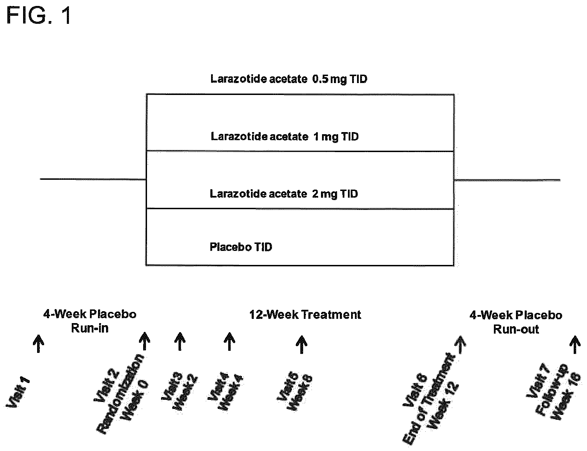

The study was conducted in 3 phases after screening: a 4-week single-blind, placebo run-in phase; a 12-week double-blind, randomized phase; and a 4-week single-blind, run-out phase (see Figure 1). The 4-week single-blind, placebo run-in phase was included to evaluate subjects' baseline symptoms in the absence of Larazotide acetate but in the presence of single-blind placebo to minimize the impact of placebo during the double-blind phase. The 12-week double-blind, randomized phase was followed by a 4-week single-blind, placebo run-out phase to evaluate recurrence of symptoms in the absence of Larazotide acetate. A comparative study was not possible because no pharmaceutical treatments are available for patients with celiac disease.

Efficacy was assessed by a variety of known scales used for evaluating subjects with celiac disease, including the GSRS, CeD GSRS, BSFS, SF12V2, CeD-QoL, and CGA. In addition, a new scale developed to assess patient reported outcomes, the CeD PRO, was also included. The GFDCQ and the CDAT assessed compliance with the GFD.

After signing the informed consent, subjects were screened for the study. At Visit 1 (Day -28), eligible subjects were to enter a single-blind, placebo run-in phase for 4 weeks. During the single-blind, placebo run-in phase, subjects were to maintain their current diet and complete the weekly GSRS, daily CeD PRO and daily BSFS diaries. At the end of the 4-week single-blind, placebo run-in phase, subjects were to return to the site for Visit 2 (Baseline, Week 0/Day 0) and be re-evaluated based on the inclusion/exclusion criteria.

At Visit 2, eligible subjects were randomized to enter a 12-week double-blind phase to receive 1 of 3 doses of Larazotide acetate or placebo. Subjects were to maintain their current diet during the double-blind, randomized phase and complete the weekly GSRS, daily CeD PRO and daily BSFS diaries. Subjects were to be followed by a phone call 2 weeks after the start of the double-blind, randomized phase (Visit 3, Week 2/Day 14) to ensure adherence to the study protocol. Subjects were to return to the clinic for Visit 4 (Week 4/Day 28), Visit 5 (Week 8/Day 56) and Visit 6 (Week 12/Day 84) after the start of the double-blind, randomized phase for assessment. All subjects were to participate in a 4-week single-blind, run-out phase with placebo until Visit 7 (Week 16/Day 112).

Subjects were instructed how to record information for the weekly GSRS responses, daily CeD PRO responses, and daily BSFS responses using an electronic diary (eDiary). Questionnaires for SF12V2 and CeD-QoL were to be administered at the beginning of the placebo run-in phase (Visit 1, Day -28), at the end of the 12-week double-blind, randomized phase (Visit 6, Week 12/Day 84) and at the follow-up visit (Visit 7, Week 16/Day 112). The CGA was to be assessed at screening, randomization (Visit 2, Week 0/Day 0), at the end of the 12-week double-blind, randomized phase (Visit 6, Week 12/Day 84) and at the follow-up visit (Visit 7, Week 16/Day 112). At the end of the follow-up phase, subjects were to complete the Gluten-Free Diet Compliance Questionnaire (GFDCQ) and the Celiac Dietary Adherence Test (CDAT).

A blood sample was to be obtained for haplotype testing of HLA-DQ2/HLA-DQ8 for subjects who consented to pharmacogenetic sampling.

Safety assessments including adverse events, clinical laboratory testing, vital signs, and electrocardiograms were assessed throughout the study.

### Selection of Study Population:

Subjects were eligible for the study if all of the following inclusion criteria were met at the beginning of the single-blind, placebo run-in period and at randomization (Visit 2, Week 0/Day 0):
1. Provided written informed consent.
2. Male or female, age 18 to 75 years, inclusive.
3. Body mass index (BMI) between 16 and 45, inclusive.
4. Female subjects either post-menopausal (amenorrhea for at least 24 consecutive months), surgically sterile, or women of childbearing potential with a negative serum beta human chorionic gonadotropin (βhCG) pregnancy test prior to entering the study who agreed to use acceptable methods of contraception for the duration of the study. Acceptable contraceptives included intrauterine devices, hormonal contraceptives (oral, patch, or injectable) in use for 1 month prior to screening, and double-barrier methods such as condoms or diaphragms with spermicidal gel or foam. All methods were to be used from at least 2 weeks (4 weeks in the case of hormonal contraceptives) before randomization until the end of the follow-up period.
5. Diagnosis of celiac disease confirmed by duodenal/jejunal biopsy or by capsule endoscopy plus a positive celiac serology test result ≥ 12 months before study entry.
6. Measureable serum anti-tTG (IgA or IgG) or anti-DGP (IgA or IgG) antibodies as determined by screening serology.
7. A CeD GSRS score of ≥ 2.0 at screening and at the end of the single-blind, placebo run-in phase prior to randomization.
8. During the month prior to the screening visit, experienced 1 of the following symptoms at least once per week or 4 times during the month: diarrhea, abdominal pain, bloating, nausea, or stomachache.
9. Attempted a GFD for at least 12 consecutive months prior to screening and willing to maintain their current diet for the duration of the study.
10. Willing to refrain from taking the following types of prohibited medications: systemic or intestinal immune suppressants, chronic or continuous oral and intravenous (IV) antibiotics (> 2 weeks' administration), chronic or continuous nonsteroidal anti-inflammatory drugs (NSAIDs) (> 2 weeks' administration) as outlined in the permitted and prohibited medications section of the protocol, medications altering gastric pH, substances that alter intestinal permeability, laxatives and colonic therapies, pancreatic enzyme replacements, fibrates, probiotics, ezetimibe, and herbal remedies.
11. Willing to return for all scheduled study visits.
12.Willing to refrain from illicit drug use and alcohol abuse throughout the duration of the study.

Subjects were not eligible for the study if any of the following exclusion criteria applied at the beginning of the single-blind, placebo run-in period and/or at randomization (Visit 2, Week 0/Day 0):
1. Identified as having Refractory celiac disease (RCD) type 1 or type 2 or severe complications of celiac disease, including, but not limited to, enteropathy-associated T cell lymphoma (EATL), ulcerative jejunitis, perforation, severe osteoporosis or malnutrition, or any other severe complication of celiac disease.
2. Suspected lymphoma or any other serious complications of celiac disease. Special caution was to be exercised when including diagnosed subjects > 50 years to exclude RCD and EATL, by the standard methods used at the site.
3. Diagnosed with any chronic active gastrointestinal disease other than celiac disease (e.g., Crohn's disease, ulcerative colitis, active irritable bowel syndrome, microscopic colitis, active lactose intolerance, active gastroesophageal reflux disease not controlled by H2 blockers), following diagnostic procedures that were standard at the site.
4. Diabetes (type 1 or type 2) or autoimmune diseases that might interfere with the conduct of the study, such as autoimmune hepatitis, primary biliary cirrhosis, multiple sclerosis, and systemic lupus erythematosus.
5. Known untreated small intestinal bacterial overgrowth.
6. Significant comorbid disease that the Investigator determined would make the subject unsuitable for enrollment, including unstable medical conditions (eg, chronic obstructive pulmonary disease, angina, severe cardio-respiratory conditions).
7. Smokers who were using nicotine or nicotine-containing products. Ex-smokers must have stopped smoking or must have stopped using tobacco and/or nicotine-containing products for at least 6 months prior to entry into the study.
8. Symptomatic neurological or psychiatric disease that would interfere with the conduct of the study.
9. Any condition or clinically significant abnormal laboratory test result at the screening visit, including ECG, as determined by the Investigator or designee physician, and confirmed by the Medical Monitor.
10. Pregnant or breastfeeding, or wishing to become pregnant during the study period, or unwilling to use birth control if a woman of childbearing potential.
11. Hemoglobin value < 8.5 g/dL, blood donation within the last 56 days, or donation of a unit of plasma in the last 7 days.
12. History of alcohol or drug abuse in the past 2 years.
13. Currently taking an excluded medication.
14. Positive urine drug test at screening suspected to be indicative of substance abuse. Subjects who tested positive on the urine drug screen parameters secondary to the use of prescribed or over-the-counter substances were not excluded, provided abuse was not suspected.
15. Positive human immunodeficiency virus (HIV), hepatitis B surface antigen, or hepatitis C tests.
16. History of cancer, other than non-melanoma skin cancer or cervical carcinoma-in-situ, which was treated successfully with curative therapy, as demonstrated by medical records.
17. Participation in any clinical drug study within the past 30 days or subjects who intended to participate in another clinical study while participating in the CLIN1001-012 study.
18. Deemed inappropriate by the Investigator.
19. Previous exposure to Larazotide acetate (AT1001).

### Withdrawal of Subjects from Study:

Reasons for withdrawal (subjects refused to return for any remaining study visits) or discontinuation (subjects who prematurely stopped treatment) at any time during the study may have included but were not limited to the following:
- Safety reasons, either at the discretion of the Investigator or the subject. In particular, subjects were to be discontinued from the study if they developed severe complications of celiac disease that required intensive treatment (eg, EATL, jejunitis, perforation).
- Protocol violations at the discretion of the Sponsor.
- Concomitant therapy that could have interfered with the results of the study (the Investigator was to report all such information on the eCRFs and decide, in accordance with the Sponsor, whether the subject was to be withdrawn).
- Subject's decision to withdraw at any time.

All premature discontinuations and their causes were carefully documented by the Investigator on the end-of-study eCRF page, and, if applicable, on the adverse event form.

If, for any reason, a subject was withdrawn before completing the final visit, the reason for termination was entered on the end-of-study eCRF page. All data gathered on the subject prior to termination were made available to the Sponsor. Randomized subjects not completing the entire study were fully evaluated when possible. The appropriate eCRFs were completed in addition to the end-of-study eCRF.

All subjects were free to withdraw from the study at any time and for whatever reason, specified or unspecified, without prejudice.

Subjects who were randomized and started double-blind study medication, but who chose to withdraw before completing the study, were to return to complete all safety and efficacy assessments as outlined for the early termination visit.

Subjects who began single-blind medication but withdrew before randomization were to return any unused run-in medication and other study-related supplies to the clinic. Safety and efficacy assessments were to be completed as appropriate.

### Treatments:

At Visit 1 (Day -28), subjects entered a single-blind, placebo run-in phase for 4 weeks. During the 12-week randomized, double-blind period, study drug was taken orally TID based on randomization to placebo, or to 0.5, 1, or 2 mg Larazotide acetate. Capsules were to be ingested 15 minutes before meals (morning, mid-day, and in the evening), preferably at the same time of the day, with water. The study finished with a 4-week single-blind, placebo run-out phase.

Larazotide acetate as 0.5 mg, 1 mg, and 2 mg was provided in 1 white opaque capsule containing approximately 1% Larazotide acetate enteric-coated multi-particulate beads. Placebo was provided in 1 white opaque capsule, identical in appearance to the Larazotide acetate drug capsules, containing enteric-coated multi-particulate beads.

The Investigator or designee was responsible for maintaining accurate study drug accountability records for the secure storage of the study medication.

### Method of Assigning Subjects to Treatment Groups:

Randomization was centralized using a stratified randomization scheme. Subjects were prospectively stratified into 4 groups according to gender (85 ± 10% female or 15 ± 10% male) and a baseline CeD GSRS score of < 3 or ≥ 3.

Three randomization cohorts were specified in the study protocol. The primary cohort, into which the vast majority of subjects were randomized, used the 1:1:1:1 allocation (placebo, Larazotide acetate 0.5 mg, 1 mg, or 2 mg). However, as the 0.5 mg drug supplies were not available until after study initiation, a separate randomization cohort allowing for only 3 groups and a 1:1:1 allocation within each stratum was used employing the same stratification factors as in the final 4-arm randomization detailed above. A third randomization cohort was to be considered when the 0.5 mg drug supplies became available, using a 1:2:1:1 allocation to allow for a "rebalancing" of the overall treatment group allocation by the end of study enrollment.

The pharmacist or designee was responsible for dispensing the study medication according to the randomization code provided by the Interactive Voice Response System/Interactive Web Response System (IVRS/IWRS).

### Selection of Doses in the Study:

The doses of Larazotide acetate selected for this study were based on results of previous clinical studies. Larazotide acetate has been studied at doses ranging from 0.25 mg to 8 mg TID and up to 36 mg in a single dose with an acceptable safety profile.

### Selection and Timing of Dose for Each Subject:

During the 12-week randomized, double-blind period, study drug was taken orally TID based on randomization to placebo, or to 0.5 mg, 1 mg, or 2 mg Larazotide acetate. Capsules were to be ingested 15 minutes before meals (morning, mid-day, and in the evening), preferably at the same time of the day, with water.

### Blinding:

The placebo run-in/run-out phases were single-blind, during which only the subjects were blinded. The treatment phase was double-blind.

If there was a need to unblind a subject's treatment assignment for emergency medical management, the Investigator was to contact the Medical Monitor. The Medical Monitor, in consultation with the Sponsor, was to decide whether to unblind the subject's treatment assignment. If the decision was made to unblind, a prompt written notification was provided to the Investigator.

If reporting of an adverse event was to be performed unblinded as per a competent regulatory authority's guidelines, Sponsor personnel unrelated to the study were to unblind the individual subject's treatment group and perform the unblinded reporting. No treatment group information was to be shared with study personnel. No unblinding reporting was necessary for this study.

### Prior and Concomitant Therapy:

As a general guidance, medications that alter gastrointestinal symptoms, in particular symptoms of diarrhea and gastrointestinal pain, were excluded from use during the placebo run-in and run-out periods as well as the double-blind treatment phase.

Use of the following medications was permitted during the study:
- Multivitamin complex (may have included calcium and iron)
- Fish oil supplements
- Omega-3 supplements
- Calcium supplements
- H₂ blockers (eg, cimetidine, ranitidine)
- Hormone replacement therapy
- Oral contraceptives or injectable birth control
- Sleep aids (non-herbal)
- Antidepressants (non-herbal)
- NSAIDs < 2 weeks
- Aspirin or NSAIDs at dose levels intended for cardiovascular prophylaxis (ie, 81 mg or low-dose aspirin)
- Inhaled, topical, or nasal corticosteroids
- Oral and IV antibiotics < 2 weeks

Subjects taking any of the following medications were not eligible for participation in the study. After enrollment into the single-blind, placebo run-in phase, use of these medications was permitted if required for treatment of an adverse event and such use was to be documented as a protocol deviation. If the medication was determined to interfere with the results of the study, the subject was to be withdrawn at the discretion of the Investigator and the Sponsor.
- Systemic or intestinal immune suppressants. The Medical Monitor was to be consulted on other immune therapies, excluding those listed as permitted, to assess their potential interference with study conduct.
- Chronic or continuous oral and IV antibiotics (> 2 weeks' use). Topical antibiotic use was allowed.
- Chronic or continuous use of NSAIDs for purposes other than cardiovascular prophylaxis purposes (> 2 weeks' use).
- Medications that alter gastric pH: proton pump inhibitors (eg, Prilosec®, Nexium®), chewable, liquid, or other antacids (eg, Maalox®, Mylanta®). Subjects could have switched to H₂ blockers before beginning the single-blind, placebo run-in phase since these medications do not alter pH directly (no washout period required).
- Substances that decrease intestinal permeability, such as diphenoxylate-containing medications (eg, Lomotil®) or bismuth-containing medications (eg, Pepto-Bismol®).
- Laxatives and colonic therapies of any type.
- Pancreatic enzyme replacement.
- Fibrates.
- Herbal remedies,
- Probiotics as therapeutics (yogurts containing probiotics were acceptable).
- Ezetimibe (Zetia®).

### Treatment Compliance:

Subjects were given bottles of either Larazotide acetate or placebo to take home with them, along with directions on when and how to take each oral dose. At scheduled clinic visits, subjects returned unused (excess) drug that was accounted for on the individual subject record.

### Efficacy and Safety Variables:

The schedule of evaluations and procedures that were performed are presented in Table 2 below.

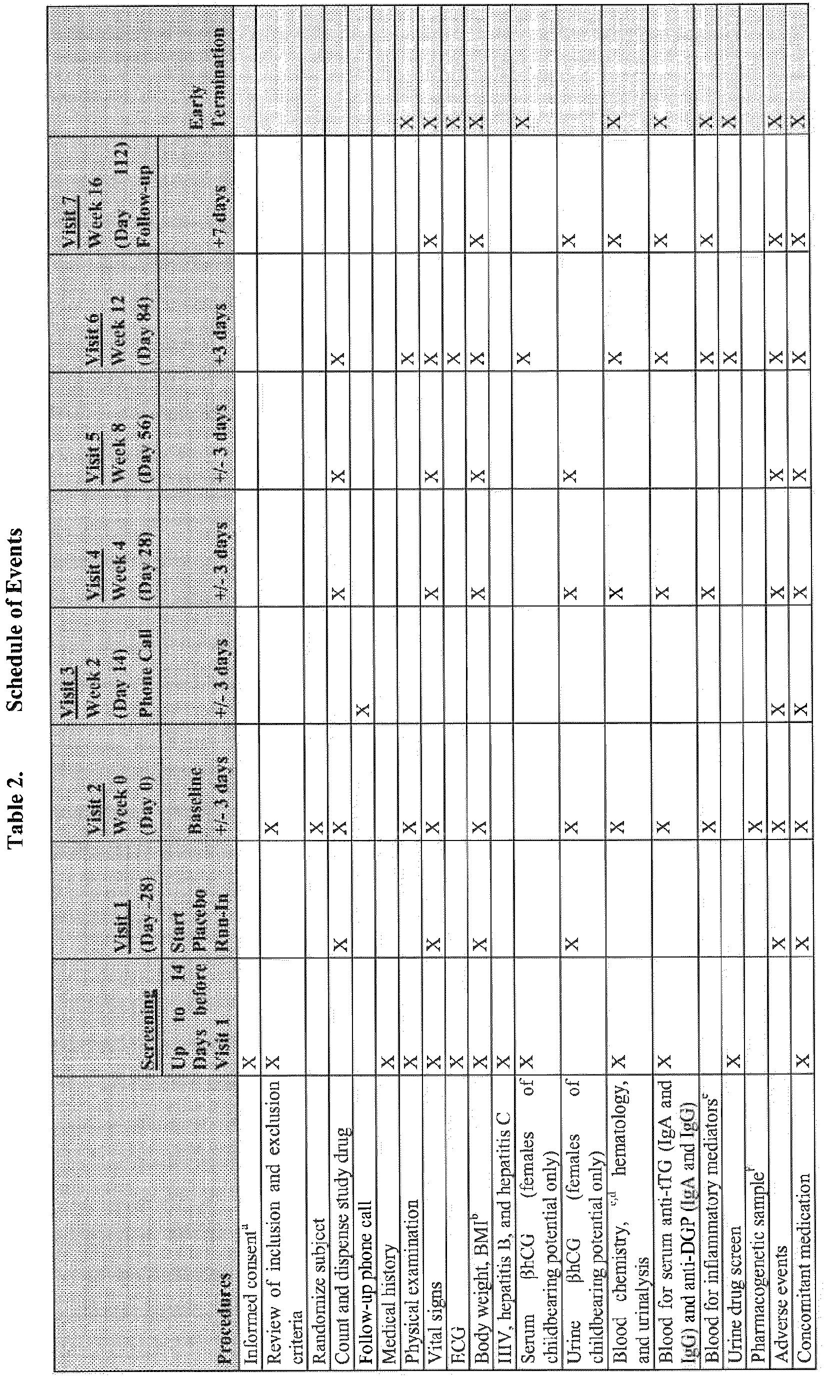

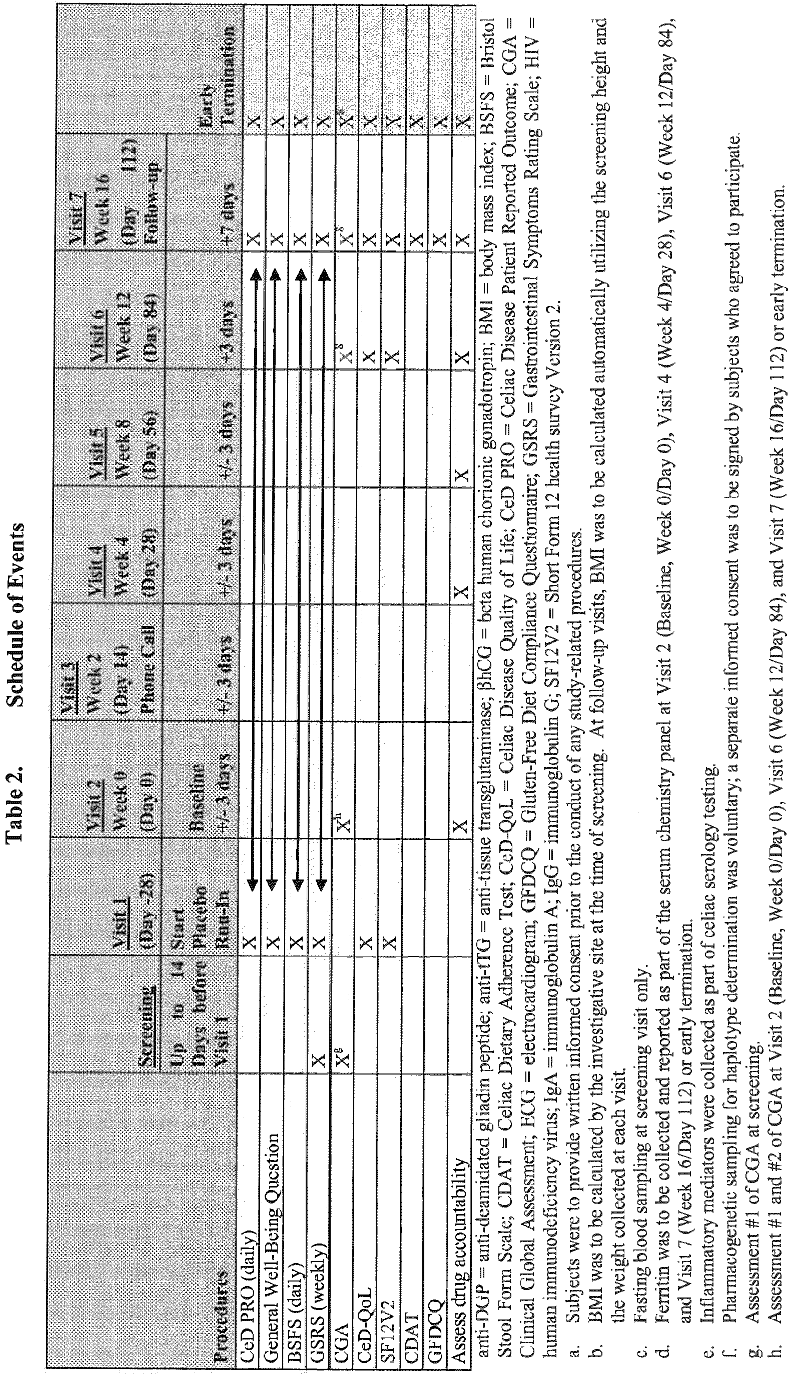

### Efficacy Evaluations:

### 1. Gastrointestinal Symptom Rating Scale (GSRS)/Celiac Disease Gastrointestinal Symptom Rating Scale (CeD GSRS)

The GSRS is a 15-question, 7-scale questionnaire to assess 5 dimensions of gastrointestinal syndromes: diarrhea, indigestion, constipation, abdominal pain, and reflux. The questionnaire was originally constructed to measure symptoms in subjects with irritable bowel syndrome and peptic ulcer and is well validated. The CeD GSRS measures a subset of the GSRS with dimensions more applicable to celiac disease. The CeD GSRS dimensions include 10 questions in the following domains: Diarrhea syndrome; Indigestion syndrome; and Abdominal Pain syndrome. The timings for completion of the CeD GSRS are presented in Table 2.

### 2. Celiac Disease Patient Reported Outcome (CeD PRO)

The CeD PRO questionnaire (see Appendix P of the protocol) was developed to assess symptom severity in clinical trials in subjects with celiac disease. Items in the questionnaire were formulated based on one-on-one interviews with subjects with celiac disease and thus reflect the symptoms that subjects consider part of their celiac disease experience. The questionnaire was designed as a self-administered daily diary, to be completed at the same time each day, and required < 10 minutes to complete. The CeD PRO includes 12 items asking participants about the severity of celiac disease symptoms they experience each day. Subjects were asked to rate their symptom severity on an 11-point, 0 to 10 scale; from "not experiencing the symptom" to "the worst possible symptom experience". Symptoms included abdominal cramping, abdominal pain, bloating, constipation, diarrhea, gas, loose stools, nausea, vomiting, headache, and tiredness.

The CeD PRO included a Gastrointestinal Domain scale consisting of all the gastrointestinal symptoms, Abdominal Domain, Diarrhea and Loose Stool Domain, Nausea Domain, Symptomatic days, Improved Symptom days, and a Non-gastrointestinal Domain scale consisting of the items headache and tiredness. Each domain score was calculated by summing the value of the individual items within a scale and averaging across the number of items within the scale. Aggregation occurred over a 7-day period starting from the day after the first dose of double-blind study drug. Higher domain scores reflected greater symptom severity. The General Well-Being Question was scored similarly as a stand-alone item. The timings for completion of the CeD PRO are presented in Table 2.

### 3. Bristol Stool Form Scale (BSFS)

The BSFS is a pictorial aid to help subjects identify the shape and consistency of their bowel movements during the study. The BSFS was developed to help physicians in diagnosing digestive conditions [Lewis 1997]. The timings for completion of the BSFS are presented in Table 2.

### 4. Clinician Global Assessment of Disease Activity (CGA)

The Investigator or qualified health care provider (physician, physician assistant, or nurse practitioner) with celiac disease expertise, completed Assessment #1 of the CGA at screening, identifying the subject's disease activity (complete remission, mild disease, moderate disease, or severe disease) by placing an "X" on a scale using all of the information normally available in their clinical practice. At all subsequent times at which the CGA was completed, the Investigator or designated health care provider completed Assessment #1 and Assessment #2 of the CGA. The same person was to complete both assessments for each subject, if possible. The timings for completion of the CGA are presented in Table 2.

### 5. General Well-Being Question

Subjects were asked to rate their general well-being on a daily basis in an eDiary on an 11-point, 0 to 10 scale; from "poor" to "excellent". Subjects completed the General Well-Being Question after completing the CeD PRO daily. The timings for completion of the General Well-Being Question are presented in Table 2.

### 6. Short Form 12 Health Survey Version 2 (SF12V2)

The SF12V2 is a health survey for monitoring outcomes in general. The study used a 4-week recall period and was to be completed by the subject. The SF12V2 offers greater comparability with translations and cultural adaptations widely used in the US and other countries. The timings for completion of the SF12V2 are presented in Table 2.

### 7. Celiac Disease Quality of Life Questionnaire (CeD-QoL)

The CeD-QoL is a 20-question instrument that measures a subject's quality of life in the past 30 days across 4 clinically relevant subscales (limitations, dysphoria, health concerns, and inadequate treatment) (see Appendix M of the protocol). The timings for completion of the CeD-QoL are presented in Table 2.

### 8. Celiac Dietary Adherence Test (CDAT)

The CDAT is a 7-item questionnaire to measure adherence to a GFD that was completed by the subject (see Appendix N of the protocol). Scores on the CDAT range from 7 to 35, with higher scores denoting worse adherence with a GFD. The timings for completion of the CDAT are presented in Table 2.

### 9. Gluten-Free Diet Compliance Questionnaire (GFDCQ)

The GFDCQ is a 13-item questionnaire to measure compliance with a GFD. The study staff interviewed each subject and completed the GFDCQ (see Appendix O of the protocol). The timings for completion of the GFDCQ are presented in Table 2.

### 10. Anti-Tissue Transglutaminase (Anti-tTG IgA and IgG) and Anti-Deamidated Gliadin Peptide (Anti-DGP IgA and IgG)

Blood was collected for determination of serum anti-tTG (IgA and IgG) and anti-DGP (IgA and IgG) at the timings specified in Table 2. These antibodies have proven sensitivity and specificity for celiac disease and exposure to gluten.

### 11. Body Weight, Height and BMI

Body weight was measured and BMI was calculated based upon screening height and weight at the timings specified in Table 2.

### Safety Evaluations:

An adverse event was any untoward medical occurrence in a study subject that was temporally associated with the use of a medicinal product, regardless of its potential relationship to the medicinal product. An adverse event, therefore, could have been any unfavorable or unintended sign, including an abnormal laboratory finding, symptom, or disease (new or exacerbated), whether or not related to study drug.

The Investigator and study staff were responsible for collecting and recording adverse events and serious adverse events during scheduled safety evaluations and whenever such information was brought to their attention. During each visit, the Investigator questioned the subject about adverse events using an open question, taking care not to influence the subject's answers, eg, "have you noticed any change in your health?"

All adverse events occurring after signing the informed consent form and on or before the final visit were to be reported as adverse events. All adverse events were to be recorded irrespective of whether they were considered drug-related.

At each visit/assessment, adverse events were to be evaluated by the Investigator and recorded. Any adverse events already documented at a previous assessment and designated as ongoing were to be reviewed at subsequent visits as necessary, and their resolution documented. Changes in intensity or frequency of adverse events were to be recorded as separate events (ie, a new record started).

All adverse events, including either observed or volunteered problems, complaints, or symptoms, were to be recorded on the adverse event pages of the eCRF. The need to capture this information was not dependent upon whether the adverse event was associated with study treatment. Adverse events resulting from concurrent illnesses or reactions to concurrent medications were also to be recorded. To avoid vague, ambiguous, or colloquial expressions, the adverse event was to be recorded in standard medical terminology rather than the subject's own words.

Each adverse event was to be evaluated for duration, intensity, and whether the event may have been associated with the study drug or other causes. Start and stop dates, relationship to study drug, medical management, and alternative causality of event were to be recorded in the adverse event section of the eCRF.

The severity of an adverse event was to be scored according to the following scale:

| | |
|---|---|
| Mild: | Awareness of sign or symptom, but easily tolerated |
| Moderate: | Discomfort enough to cause interference with usual activity |
| Severe: | Incapacitating, with inability to work or perform usual activity |

The relationship of an adverse event to study drug was to be assessed according to the following definitions:
- Definitely Not Related:: The adverse event was definitely not related to study drug. This designation was reserved for those events that occurred prior to study treatment or for those events that could not be even remotely related to study participation (eg, injuries sustained in an automobile accident).
- Unlikely to be Related:: There was no reasonable association between the study drug and the suspected event, and the event could have been produced by the subject's clinical state or other modes of therapy administered to the subject.
- Possibly Related:: The suspected adverse event may or may not have followed a reasonable temporal sequence from study drug administration but could not be dismissed as unlikely. The event could have been produced or mimicked by the subject's clinical state or by other modes of therapy concomitantly administered to the subject.
- Probably Related:: The suspected adverse event followed a reasonable temporal sequence from study drug administration, abated upon discontinuation of the treatment, and could not be reasonably explained by the known characteristics of the subject's clinical state.
- Definitely Related:: This designation was reserved for those events that had no uncertainty in their relationship to study drug administration.

All adverse events considered possibly related, probably related or definitely related to study drug and those adverse events considered clinically significant were to be followed until resolution, or for 28 days after the last dose of the drug, or a diagnosis of chronicity could be made, provided it was within the 28-day follow-up period.

A serious adverse event was any untoward medical occurrence that, at any dose:
- resulted in death.
- was life-threatening. The term life-threatening in the definition of serious referred to an event in which the subject was at risk of death at the time of the event. It did not refer to an event that, hypothetically, might have caused death, if it were more severe.
- required hospitalization or prolongation of existing hospitalization. In general, hospitalization signified that the subject had been detained (usually involving at least an overnight stay) at the hospital or emergency ward for observation and/or treatment that would not have been appropriate in the physician's office or outpatient setting. Complications that occurred during hospitalization were adverse events. If a complication prolonged hospitalization or fulfilled any other serious criteria, the event was serious. When in doubt as to whether "hospitalization" occurred or was necessary, the adverse event was considered serious. Hospitalization for elective treatment of a pre-existing condition that did not worsen from baseline was not considered an adverse event.
- resulted in disability/incapacity. The term disability meant a substantial disruption of a person's ability to conduct normal life functions. This definition was not intended to include experiences of relatively minor medical significance such as uncomplicated headache, nausea, vomiting, diarrhea, influenza, and accidental trauma (eg, sprained ankle), which may have interfered or prevented everyday life functions but did not constitute a substantial disruption.
- was a congenital anomaly/birth defect.
- medical or scientific judgment was to be exercised in deciding whether reporting a serious adverse event was appropriate in other situations, such as important medical events that may not have been immediately life-threatening or resulted in death or hospitalization but may have jeopardized the subject or may have required medical or surgical intervention to prevent 1 of the other outcomes listed in the above definitions. These were also considered serious. Examples of such events included invasive or malignant cancers, intensive treatment in an emergency room or at home for allergic bronchospasm, blood dyscrasias or convulsions that did not result in hospitalization, or development of drug dependency or drug abuse.

Serious adverse events were classified as "not related" or "related" (ie, there was a reasonable possibility that the event was caused by the study drug). If the relationship of the serious adverse event to the study drug was not known, it was assumed to be related.

Any serious adverse event, occurring in a subject receiving treatment or if the Investigator became aware of any serious adverse event post-treatment during the follow-up period, was to have been reported by the Investigator to the Medical Monitor within 24 hours of becoming aware of the event, even if the serious adverse event did not appear to be drug-related. This was done by telephone and by sending a faxed copy of the serious adverse event report form plus other related information. Additionally, it may have been necessary for the Sponsor to directly communicate with the Investigator if additional information was required.

All serious adverse events were followed until the outcome was determined by the Investigator as either resolved, ongoing, chronic/stable, death, or unknown.

The Sponsor evaluated expectedness of the serious adverse event as per the safety information contained in the current Investigator's Brochure.

The Sponsor was responsible for completing the safety report and for notifying the relevant authorities of any serious adverse event as outlined in the ICH guidelines and per local regulatory requirements. The Investigator was to notify the appropriate IRB/IEC/Research Ethics Board of the serious adverse event.

After the initial serious adverse event report, the Investigator was required to follow each subject proactively and provide further information to the Sponsor on the subject's condition within 24 hours. All additional follow-up evaluations were to have been reported to the Medical Monitor. Such data was to have been sent to the Sponsor within 10 calendar days (or as specified by national legislation) using the serious adverse event report form.

New or updated information was to be recorded on the serious adverse event eCRF. The updated serious adverse event eCRF was to have been sent to the Sponsor within 24 hours.

The Investigator or designee obtained vital signs including temperature, blood pressure (sitting), and pulse at screening and all study visits.

The Investigator or qualified designee performed a complete physical examination at the times indicated in Table 2. The physical exam included, at a minimum, assessment of general appearance, head/eyes/ears/nose/throat/neck, lymph nodes, respiratory system, cardiovascular system, gastrointestinal system, musculoskeletal system (including extremities), neurological, psychological, and dermatological systems.

Twelve-lead ECGs were obtained at screening and at Visit 6 or early termination (Table 2).

Clinical laboratory evaluations were performed centrally. Handling and shipment of clinical laboratory samples were outlined in the Study Manual.

Blood and urine samples were collected at the times indicated in Table 2.; clinical laboratory parameters assessed during the study are listed in Table 3.

**Table 3 Laboratory Parameters**

| **Blood Chemistry** | **Urinalysis** |
|---|---|
| Alanine aminotransferase (ALT) | Protein |
| Aspartate aminotransferase (AST) | Glucose |
| Alkaline phosphatase | Ketones |
| Blood urea nitrogen (BUN) | Blood cells |
| Creatinine | Leukocyte esterase |
| Glucose | Nitrite |
| Ferritin | Bilirubin |
| Sodium | Urobilinogen |
| Potassium | Microscopy |
| Calcium | Urine pregnancy test (βhCG) |
| Chloride | |
| Total bilirubin | **Urine Drug Screen** |
| Albumin | Amphetamines |
| Serum pregnancy test (βhCG) | Barbiturates |
| Human Immunodeficiency Virus antibody | Cocaine metabolites |
| Hepatitis B surface antigen | Opiates |
| Hepatitis C antibody | Benzodiazepines Cannabinoids |

| **Hematology** | |
|---|---|
| Hemoglobin | |
| Hematocrit | |
| Platelet count | |
| White blood cell count with differential | |

A subject with a clinically significant laboratory finding identified between screening and Visit 2 (Week 0/Day 0) was not to have participated in the study. All clinically significant findings during the study were to have been followed until resolution or until the finding was clinically stable. Subjects were to have been withdrawn from the study if the Investigator or Sponsor deemed that the clinically significant finding compromised subject safety.

Women of childbearing potential were to have urine or serum pregnancy tests at the times indicated in Table 2. Subjects who became pregnant during the study were to be withdrawn from participation and the outcome of the pregnancy followed.

Blood for serum was collected at the times indicated in Table 2 for potential future determination of inflammatory mediators that may be involved in the response of celiac disease to Larazotide acetate, such as cytokines (e.g., TNF-α or IFN-γ) [Fasano 2000a, Fasano 2000b, Wang 2000, Drago 2006]. The sera are stored at the Sponsor's designated facility for 5 years for this purpose.

At Visit 2 (randomization, Week 0/Day 0) or any time thereafter, blood was collected from subjects who consented to HLA typing (DQ2 and DQ8). Samples were coded in order to trace information back to the study subject, which was necessary to confirm the diagnosis of celiac disease and to understand how the HLA haplotype determines the response to therapy. This substudy was voluntary, and a subject's willingness to provide this additional sample was optional and did not affect his/her participation in the study. Subjects signed an appropriate consent, separate from the main study consent, before sample collection.

The HLA haplotype confirms the diagnosis of celiac disease. It has a > 99% negative predictive value, indicating that a subject is extremely unlikely to have celiac disease if the HLA-DQ2/DQ8 haplotype is not present. The HLA haplotype is also an exploratory genetic marker to determine the possible association between HLA-DQ2 and HLA-DQ8 and the clinical features of celiac disease, and its potential response to Larazotide acetate in a given subject.

Efficacy was assessed by a variety of known scales used for evaluating subjects with celiac disease, including the GSRS, CeD GSRS, BSFS, SF12V2, CeD-QoL, and CGA. In addition, a new scale developed to assess celiac disease patient reported outcomes (CeD PRO) was also included. Standard assessments of safety were utilized in the study.

The primary efficacy endpoint of the study was the average on-treatment score of the CeD GSRS. A comparison was made between each of the 3 doses of Larazotide acetate versus placebo throughout the 12 treatment weeks. Secondary efficacy endpoints included change from baseline to the end of treatment in the CeD GSRS score, average on-treatment scores of the CeD PRO Abdominal Domain (abdominal pain, abdominal cramping, bloating, and gas); change from baseline to the end of treatment in the CeD PRO Abdominal Domain, average on-treatment scores of the CeD PRO Gastrointestinal Domain (abdominal pain, abdominal cramping, bloating, gas, diarrhea, and loose stools), and change from baseline to the end of treatment in the CeD PRO Gastrointestinal Domain. In addition, a number of exploratory efficacy variables were also included as specified in the Statistical Analysis Plan.

Measurements of drug concentration were not performed in this study.

### Data Quality Assurance:

The Investigator was required to prepare and maintain adequate and accurate case histories (ie, source documents and/or Medical Record Supplement) designed to record all observations and other data pertinent to the study for each subject. This included accurate documentation of accountability of study drugs. The medical records were to contain adequate information to allow for verification of a subject's identity throughout the study.

An eCRF was to be completed for each subject enrolled in the study. All information recorded on the eCRFs for this study was to have been consistent with the subject's source documentation (ie, source documents and/or Medical Record Supplement). The source documents may have included the hospital and/or the physician's chart, biopsies, or laboratory test documentation.

During the course of the study, a monitor made routine site visits to review protocol compliance, check informed consent forms of newly enrolled subjects, assess drug accountability, and ensure that the study was being conducted according to pertinent regulatory requirements. In addition, the original eCRF for each subject was to be periodically checked by the monitor against the subject's source documents at the study site; these reviews were to be performed in a manner to ensure that subject confidentiality was maintained. Instances of missing or unclear data were to be discussed with appropriate site personnel for resolution. The eCRF data were entered into a computerized database and a quality-assurance audit was performed on the database.

Quality-assurance audits may also have been performed by the Sponsor during the course of this study. These audits were performed in a manner to ensure that subject confidentiality was maintained.

### Statistical Methods Utilized in the Protocol and Determinal of Sample Size:

The set of all randomized subjects was used only for displays of subject disposition, protocol deviations, and listings.

The following 3 specific analysis populations were defined for various analyses:
1. Modified intent-to-treat (MITT) Population: All randomized and treated subjects included in the assigned treatment groups if they had a baseline measure and at least 1 on-study measure. This was the primary population used to assess efficacy.
2. Safety Population: All subjects who received at least 1 dose of study drug with treatment assignments designated according to the actual study treatment initially received. This was the primary population for evaluating safety.
3. Per-Protocol Population: This subset excluded subjects with major protocol deviations thought to impact the ability to assess the efficacy of the treatment. Exclusion of subjects from the Per-Protocol Population was reviewed, documented, and approved before the study was unblinded. The criteria for excluding subjects from the Per-Protocol Population were specified in a Per-Protocol Analysis Set Plan. The Per-Protocol Population was used for selected efficacy analyses.

In general, data were summarized by scheduled nominal visit. For the purposes of data listings and summaries, study day was calculated relative to the first dose of study drug (*i.e.,* date of interest - date of first dose of double-blind study medication).

Baseline values for both safety and efficacy variables were selected as the last non-missing observation before administration of the first dose of double-blind study medication. Assessments falling exactly on the date of the first dose of study medication were considered pre-dose, with the exception of adverse events and concomitant medications, which were considered on-therapy.

Data from subjects who were randomized and subjects who completed or were withdrawn from the double-blind treatment and follow-up periods of the study were summarized using subject counts and percentages. Data from subjects who were withdrawn from the double-blind treatment and follow-up periods of the study were also summarized by reason for withdrawal using subject counts and percentages.

Subject demographics at the screening visit were summarized for the MITT and Safety Populations. All medical histories were coded using the Medical Dictionary for Regulatory Activities (MedDRA) Version 14.1. Medical history and diagnostic biopsy data at the screening visit were summarized by the number and percentage of subjects (eg, with each history type).

All previous and concomitant therapies were coded using the World Health Organization's Drug Dictionary (WHO-DD Sep 2011). All concomitant nondrug treatments or procedures were coded using MedDRA Version 14.1.

Previous drug treatments included all drug treatments taken prior to the first day of study drug (ie, before or on the date of medication dispensing at Visit 2). Generally, the previous drug treatments were captured if the subject had been taking them within 30 days of the screening visit. Concomitant drug treatments and concomitant nondrug treatments included all drug treatments and nondrug treatments taken while the subject was on study drug (ie, after Week 0).

The incidences of previous and concomitant drug treatments were summarized using subject counts and percentages by anatomic therapeutic class and preferred term. Subjects were counted only once in each anatomic therapeutic class category and only once in each preferred term category.

Subjects with major protocol deviations were listed with pertinent information by treatment group, site, and subject number. Major protocol deviations were summarized using subject counts by treatment group.

Subjects excluded from each analysis population were listed with the reason for exclusion.

The MITT Population was used for all efficacy analyses, and the Per-Protocol Population was used for selected efficacy analyses. For the purpose of analysis, the first, third, and fourth randomization cohorts were combined into a single randomization cohort and the second cohort was kept separate. All statistical testing for efficacy variables was 2-sided, employing a significance level of 0.05.

The primary efficacy endpoint was the average on-treatment score of the CeD GSRS. Baseline was the CeD GSRS score collected at Week 0 (defined as the CeD GSRS completion date for Week 0). A comparison was made between each of the 3 doses of Larazotide acetate versus placebo.

Analysis of covariance (ANCOVA) was performed, with treatment, baseline CeD GSRS (stratification factor for the randomization), gender, and randomization cohort as the independent factors, and baseline (as defined above) as a covariate. Contrasts were used to perform the 3 primary comparisons of each dose level versus placebo, each at the 5% level of significance. Secondary comparisons of pooled doses (eg, 0.5 mg + 1 mg + 2 mg) versus placebo were also performed.

Similar analysis was performed for the weekly average prior to each scheduled visit during the double-blind period as well as after the 4-week single-blind, run-out period without carrying forward data (ie, observed case analysis).

As a sensitivity analysis, a mixed model for repeated measures (MMRM) analysis was applied to the weekly on-treatment score of the CeD GSRS. This model included gender, the baseline stratification levels of CeD GSRS, randomization cohort as covariates, treatment, week, and treatment-by-week interaction as fixed factors, and subject identification as the unit for repeated measures. The contrast of the main effects was used for the between-group comparison when the assumption that data were missing at random held true.

Analyses of the primary endpoint were performed using the MITT and Per-Protocol Populations, with the MITT representing the primary analysis.

The secondary and exploratory efficacy endpoints are as described previously. The final Statistical Analysis Plan included 2 additional secondary endpoints, not previously specified in the final protocol (incorporating Amendment #5). The additional endpoints were changes from baseline to the end of treatment in the CeD PRO Abdominal Domain and CeD PRO Gastrointestinal Domain scores.

The ANCOVA described for the primary endpoint was used for analysis of the continuous secondary and exploratory endpoints.

Assessments 1 and 2 of CGA (CGA 1 and CGA 2) were analyzed using the Cochran-Mantel-Haenszel (CMH) test for the raw mean score difference controlling for the randomization strata. Modified ridit scores were used for these analyses. CGA 1 and CGA 2 were summarized with subject frequency distribution. Comparisons to placebo were performed including only data from the groups being compared.

Analyses of responder rates (ie, 10%, 20%, 30%, and 50% reduction for ≥ 6 of 12 weeks) for CeD GSRS, CeD PRO Abdominal Domain, CeD PRO Gastrointestinal Domain, and number of diarrhea and loose stools (BSFS scores of 5 - 7) were analyzed similarly using the CMH test.

Average on-treatment weekly numbers of CeD PRO Improved Symptom days and CeD PRO Symptomatic days were analyzed using the ANCOVA described for the primary endpoint.

The GSRS [Dimenäs 1993a, Dimenäs 1993b] is a questionnaire that contains 15 items, using a 7-point Likert scale with 1 representing the most positive option and 7 the most negative option. See Table 4 below. The scale is well validated for gastrointestinal diseases and accepted by the medical community and regulators, although it has not been validated for celiac disease. Five dimensions (Diarrhea syndrome, Indigestion syndrome, Constipation syndrome, Abdominal Pain syndrome, and Reflux syndrome) are defined for the GSRS below. A mean value for the items in the dimension was calculated and presented as the dimension score for that subject. In addition, the total GSRS score was calculated as the mean value for all 15 items. A celiac disease-related subtotal score, CeD GSRS, was calculated as the mean value for all 10 items in the dimensions of Diarrhea, Indigestion, and Abdominal Pain syndromes.

**Table 4.**

| **GSRS Dimensions** | **Items contained in dimension** |
|---|---|
| Diarrhea syndrome | 11. Increased passage of stools |
| | 12. Loose stools |
| | 14. Urgent need for defecation |
| Indigestion syndrome | 6. Rumbling |
| | 7. Abdominal distension |
| | 8. Eructation |
| | 9. Increased flatus |
| Constipation syndrome | 10. Decreased passage of stools |
| | 13. Hard stools |
| | 15. Feeling of incomplete evacuation |
| Abdominal Pain syndrome | 1. Abdominal pain |
| | 4. Hunger pain |
| | 5. Nausea and vomiting |
| Reflux syndrome | 2. Heartburn |
| | 3. Acid regurgitation |

If there were missing data for a subject in the GSRS questionnaire at a visit and the missing data were ≤ 50% of the item scores within a dimension, the dimension score was calculated using the non-missing values within that dimension. If more than 50% of the item scores were missing within a dimension, the dimension score was not calculated, and the dimension score for that subject was excluded from the analysis.

Anti-tTG (IgA and IgG) and anti-DGP (IgA and IgG), and change from baseline (Week 0) to Visit 4 (Week 4), End of Treatment Visit 6 (Week 12), and End of Study Visit 7 (Week 16) were summarized descriptively. Week 0 served as baseline.

The weekly average on-treatment individual scores for the CeD PRO (Non-Gastrointestinal Domain) were summarized descriptively and analyzed using the model specified for the primary analysis.

The SF12V2 is composed of 12 items, with each item scored on a Likert scale from 1 to 3 or 1 to 5. The results of the SF12V2 were summarized descriptively. The quality-adjusted life years were calculated based on the SF12V2 score.

The CeD-QoL data were descriptively summarized by treatment group and listed by individual subject.

Summary tables were provided for the CDAT and GFDCQ.

The safety population was used for all safety analyses. Safety was assessed by the type, incidence, severity, timing, seriousness, and relatedness to study drug of adverse events and by laboratory assessments.

Three categories of adverse events were defined based upon onset study day. Adverse events that had onset dates prior to the first dose of double-blind study drug were considered prior. Adverse events with onset dates on or after the first dose of double-blind study medication and within 7 days following the last dose of double-blind study drug were considered on-therapy or treatment-emergent. Events with onset dates more than 7 days after the last dose of double-blind study drug were considered post-therapy.

Adverse events were coded using MedDRA Version 14.1. Summaries were presented for all adverse events (overall and by severity), adverse events determined by the Investigator to be treatment-related (defined as possibly, probably, and definitely related) (overall and by severity), serious adverse events, and adverse events causing withdrawal from the study.

The incidence of adverse events was summarized using subject counts and percentages by system organ class, preferred term, and treatment group. Subjects were counted only once in each system organ class category and only once in each preferred term category. For the summary by severity, subjects were counted for the greatest severity.

All adverse events reported during the study were listed, documenting course, severity, treatment, and outcome. In addition, listings for serious adverse events and adverse events leading to withdrawal from the study were presented.

Other safety and tolerability parameters included vital sign measurements; physical examination results; ECG results; clinical laboratory test results of blood and urine, including standard safety panels for chemistry, hematology, and urinalysis.

For continuous variables, changes from baseline to each time point were summarized with the number of nonmissing values, mean, standard deviation, median, and minimum and maximum values. For categorical variables, subject counts and percentages were provided. Results of ECGs were classified as normal; abnormal, not clinically significant, and abnormal, clinically significant, as specified on the ECG page of the eCRF. Summaries were presented by treatment group.

Shifts (below, within, and above the normal range) from baseline to each time point were summarized for clinical laboratory test results using subject counts. The incidence of potentially clinically significant abnormal values was summarized for selected clinical laboratory data using subject counts and percentages. The criteria for identifying clinical laboratory data as potentially clinically significant were specified in Table 5 as follows:

**Table 5.**

| **Test** | **Criterion value** |
|---|---|
| **Serum chemistry** | |
| Aspartate aminotransferase | ≥ 3 × ULN |
| Alanine aminotransferase | ≥ 3 × ULN |
| Alkaline phosphatase | ≥ 3 × ULN |
| Glucose | ≤ 0.75 × LLN |
| Ferritin | ≤ 0.75 × LLN |

| **Hematology** | |
|---|---|
| Hemoglobin | ≤ 0.75 × LLN |
| Hematocrit | ≤ 0.75 × LLN |
| Neutrophil (absolute counts) | ≤ 0.75 × LLN or ≥ 1.5 × ULN |
| Neutrophil (%) | ≤ 0.75 × LLN or ≥ 1.5 × ULN |
| Lymphocytes (absolute counts) | ≤ 0.75 × LLN or ≥ 1.5 × ULN |
| Lymphocytes (%) | ≤ 0.75 × LLN or ≥ 1.5 × ULN |
| Leukocytes | ≤ 0.75 × LLN or ≥ 1.5 × ULN |

| | |
|---|---|
| Abbreviations: LLN = lower limit of normal range; ULN = upper limit of normal range. | |

Potentially clinically significant abnormal laboratory values were listed by individual subject.

The determination of sample size for the study was based on the effect and variability estimates for the change from baseline in the CeD GSRS score obtained from 2 previously conducted trials of Larazotide acetate (CLIN1001-006 and AT1001-011). Based on a standard deviation σ = 0.548 and a type I error rate α = 0.05, a sample size of 70 subjects per treatment group would provide over 80% power to detect a difference of 0.3 units in the change from baseline (in average CeD GSRS score) between any given active treatment group and placebo. A difference of 0.3 in CeD GSRS is regarded by the medical community as representing a clinically meaningful improvement in GSRS. Assuming a 14% dropout rate, 80 subjects per treatment group (total of 320) was required for randomization.

### Changes in the Conduct of the Study or Planned Analyses:

The original study protocol was amended 5 times. The main purposes of each amendment are described below:

### 1. Amendment #1:

Amendment 1, Version 2.0 clarified aspects of the protocol in order to make the document consistent with intended analysis and execution. Most clarifications were necessitated by a restructuring of the primary analysis. The general rationale for the changes was as follows:
- Primary Efficacy Endpoint - The primary efficacy endpoint was changed from the CeD PRO Gastrointestinal Domain to the Celiac Domains of the CeD GSRS. Both scales were similar in design; however, the GSRS had been previously validated whereas the CeD PRO had not. Validation of the CeD PRO was incorporated as a secondary endpoint.
- Removal of Interim Analysis - The initially planned interim analysis was not helpful as it would not provide any meaningful power upon which to base decisions.
- Revision of Statistical Analysis - The statistical section was revised to reflect the change in primary, secondary, and exploratory endpoints.
- Update to Safety and Efficacy Information - Safety information was updated to reflect increases in the total number of healthy volunteers/subjects exposed to the investigational product as well as to add safety and efficacy data from Study CLIN1001-006.
- Clarifications and Updates to Study Procedures - Clarifications were made to the inclusion/exclusion criteria in order to make them consistent with the intended subject population. Clinical laboratory evaluations (blood and urine) were added to Visit 7. The GFDCQ was revised to capture events occurring throughout the entire duration of the study.

This amendment was not released to clinical sites for use.

### 2. Amendment #2:

Amendment 2, Version 3.0 clarified aspects of the protocol in order to make the document consistent with intended analysis and execution. The general rationale for the changes was as follows:
- The randomization scheme was modified to account for the availability of the 2 mg active study drug arm.
- Clarifications were made to the inclusion/exclusion criteria and study procedures in order to make the criteria consistent with the intended subject population.

### 3. Amendment #3:

Amendment 3, Version 4.0 clarified aspects of the protocol in order to make the document consistent with intended analysis and execution. The general rationale for the changes was as follows:
- The randomization scheme was modified to account for the availability of the 0.5 mg active study drug arm.
- Corrections were made to the protocol for document consistency

### 4. Amendment #4:

Amendment 4, Version 5.0 clarified aspects of the protocol in order to make the document consistent with intended analysis and execution. The general rationale for the changes was as follows:
- Secondary Endpoints - Celiac disease serology anti-DGP and anti-tTG were changed from secondary to exploratory endpoints as it was realized that, in the absence of a gluten challenge, baseline values would not be high enough to demonstrate significant reduction.
- Revision of Statistical Analysis - The statistical section was revised to remove information about rebalancing of the study groups.
- Clarifications and Updates to Study Procedures - Clarifications were made to the inclusion/exclusion as well as the permitted and prohibited medications in order to make the criteria consistent with the intended subject population. Allowable windows for Visit 6 and Visit 7 were modified in order to assure availability of GSRS on the electronic diary at or in advance of the visits.

### 5. Amendment #5:

Amendment 5, Version 6.0 clarified aspects of the protocol in order to make the document consistent with intended analyses. The general rationale for the changes was as follows:
- Primary Endpoint - The primary endpoint was clarified from analysis of CeD GSRS between Visit 2 and Visit 6 to an average on-treatment score of the CeD GSRS.
- Secondary Endpoints - Secondary endpoints were added to the statistical portion of the protocol.
- Exploratory Endpoints - Exploratory endpoints were added to the statistical portion of the protocol.
- Revision of Statistical Analysis - The statistical section was revised to account for the clarification of the primary endpoint.

### 6. Other Changes in Study Conduct:

Subjects were prospectively stratified into 4 groups according to gender (85%:15%, female: male) and baseline CeD GSRS scores of <3 or ≥ 3 and equally distributed across treatment arms. Within each stratum, randomization was centralized using a permuted block randomization scheme determined by the randomization statistician. As described in Section 9.4.3, randomization was 1:1:1:1 to Larazotide acetate 0.5 mg, 1 mg, or 2 mg capsules, or matching placebo capsules. Subjects and all study personnel remained unaware of treatment allocation.

However, the 0.5 mg drug supplies were not available upon study initiation. As such, prior to availability of the 0.5 mg dose, a separate randomization cohort allowing for the remaining 3 groups and a 1:0:1:1 allocation within each stratum was used for the initial part of the study (with approximately 14 subjects enrolled in this randomization cohort). Subsequently, the randomization cohort was changed inadvertently to 1:2:1:1 (instead of 1:1:1:1) and approximately 202 additional subjects were enrolled. When the imbalance was noted (based on drug supply considerations), the over-enrolling arm was discontinued and the randomization allocation again became 1:0:1:1 (with approximately 113 subjects enrolled). After approximately 320 subjects were enrolled, the randomization reverted back to the original intended 1:1:1:1 allocation ratio (with approximately 13 subjects enrolled). The study remained double-blinded throughout these different allocations. Thus, a total of 4 randomization cohorts were used during the study. Details regarding how the different randomization cohorts were accounted for in the analyses (eg, blocking factor or stratification factor) are specified in the detailed description for each analysis.

### 7. Changes in the Planned Analyses:

There were no changes from the planned analyses specified in the Statistical Analysis Plan. Several additional data summaries were prepared after the study blind had been broken, including:
- Summaries of serum anti-tTG and anti-DGP antibodies were prepared for the Per-Protocol Population;
- Individual domains and total scores of GSRS, numbers of bowel movements, CeD PRO Symptomatic days, and CeD PRO Improved Symptom days were analyzed by study week.

### Study Subjects:

A total of 783 subjects were screened for study participation, 454 of whom were enrolled into the 4-week single-blind, placebo run-in phase. One hundred twelve (112) of the 454 subjects (∼25%) withdrew after enrollment but prior to randomization (during the placebo run-in phase) due to various reasons including failure to meet entrance criteria (59), no longer willing to participate (28), adverse events (14), lost to follow-up (7), protocol violation (2), and other (2). Thus, a total of 342 subjects were randomized to placebo (84 subjects), Larazotide acetate 0.5 mg TID (86 subjects), Larazotide acetate 1 mg TID (85 subjects), or Larazotide acetate 2 mg TID (87 subjects) in the 12-week double-blind treatment phase of the study.

Subject disposition for the double-blind treatment phase and the single-blind, run-out phase is summarized in Table 6. A total of 298 (87.1%) of the 342 subjects completed the double-blind treatment phase. Eleven subjects in each of the treatment groups withdrew from the double-blind treatment phase (13.1% placebo, 12.8% Larazotide acetate 0.5 mg, 12.9% Larazotide acetate 1 mg, and 12.6% Larazotide acetate 2 mg). Reasons for withdrawing from the double-blind treatment phase included adverse event (5.3%), no longer willing to participate (4.1%), lost to follow-up (2.3%), other (0.9%), and withdrawn due to pregnancy (0.3%). Specific reasons leading to withdrawal from the double-blind treatment phase were generally similar among the placebo and Larazotide acetate treatment groups with no clear trend noted for reasons leading to withdrawal. Treatment-emergent adverse events leading to withdrawal from the double-blind phase occurred in 3.6% of placebo subjects, 5.8% of Larazotide acetate 0.5 mg subjects, 5.9% of Larazotide acetate 1 mg subjects, and 5.7% of Larazotide acetate 2 mg subjects; additional information regarding subjects who withdrew due to treatment-emergent adverse events is presented in elsewhere.

Subjects who completed the double-blind treatment phase entered into the 4-week single-blind, run-out phase. Of the 298 subjects who entered, 293 subjects completed the single-blind, run-out phase. All 5 subjects who withdrew during the single-blind, run-out phase had received Larazotide acetate during double-blind treatment. Reasons for withdrawal included no longer willing to participate in the study (2 Larazotide acetate 0.5 mg subjects and 1 Larazotide acetate 1 mg subject), lost to follow-up (1 Larazotide acetate 2 mg subject), and other (1 Larazotide acetate 0.5 mg subject).

**Table 6 Subject Disposition - All Subjects**

| | **Placebo** | **Larazotide Acetate TID** | | | |
|---|---|---|---|---|---|
| | | **0.5 mg** | **1 mg** | **2 mg** | **Total** |
| Randomized, n | **84** | **86** | **85** | **87** | **342** |
| Double-Blind Treatment Period, n (%) | | | | | |
| Completed | 73 (86.9) | 75 (87.2) | 74 (87.1) | 76 (87.4) | 298 (87.1) |
| Withdrew (reason) | 11 (13.1) | 11 (12.8) | 11 (12.9) | 11 (12.6) | 44 (12.9) |
| Adverse event | 3 (3.6) | 5 (5.8) | 5 (5.9) | 5 (5.7) | 18 (5.3) |
| Lost to follow-up | 3 (3.6) | 2 (2.3) | 0 | 3 (3.4) | 8 (2.3) |
| Other | 1 (1.2) | 1 (1.2) | 0 | 1 (1.1) | 3 (0.9) |
| Subject no longer willing to participate in study | 4 (4.8) | 3 (3.5) | 6 (7.1) | 1 (1.1) | 14 (4.1) |
| Withdrawn due to pregnancy | 0 | 0 | 0 | 1 (1.1) | 1 (0.3) |
| Post-treatment Follow-up Period, n (%) | | | | | |
| Completed | 73 (86.9) | 72 (83.7) | 73 (85.9) | 75 (86.2) | 293 (85.7) |
| Withdrew (reason) | 0 | 3 (3.5) | 1 (1.2) | 1 (1.1) | 5 (1.5) |
| Lost to follow-up | 0 | 0 | 0 | 1 (1.1) | 1 (0.3) |
| Other | 0 | 1 (1.2) | 0 | 0 | 1 (0.3) |
| Subject no longer willing to participate in study | 0 | 2 (2.3) | 1 (1.2) | 0 | 3 (0.9) |

| | | | | | |
|---|---|---|---|---|---|
| Abbreviations: TID = three times daily. | | | | | |

Ten subjects (2 placebo subjects, 1 Larazotide acetate 0.5 mg subject, 4 Larazotide acetate 1 mg subjects, and 3 Larazotide acetate 2 mg subjects) had major protocol deviations identified during the study including failure to meet a specific inclusion/exclusion criterion, deviation from study medication administration, and receipt of an excluded concomitant medication/treatment. Three subjects had protocol deviations noted for which Sponsor approved the subject's continued participation in the study. These approvals included use of low-dose doxycycline for treatment of pre-existing dermatitis (Subject 402017 in the placebo group), diagnosis of primary biliary cirrhosis (Subject 424004 in the Larazotide acetate 1 mg group), and serologies that did not meet inclusion criteria, but subject was symptomatic and followed a GFD (Subject 407007 in the Larazotide acetate 2 mg group).

No subject treatment assignments were unblinded during this study.

### Efficacy Evaluation

### 1. Data Sets Analyzed

A summary of the data sets analyzed for safety and efficacy is presented in Table 7. A total of 342 subjects were randomized to placebo (84 subjects), Larazotide acetate 0.5 mg (86 subjects), Larazotide acetate 1 mg (85 subjects), or Larazotide acetate 2 mg (87 subjects) in the 12-week double-blind treatment phase of the study. Two of the randomized subjects (1 in the Larazotide acetate 0.5 mg group and 1 in the Larazotide acetate 1 mg group) were lost to follow-up prior to any postbaseline clinical visit, had no evidence of ever taking study drug, and were removed from the populations evaluating safety and efficacy. Therefore, 340 subjects (84 placebo; 85 Larazotide acetate 0.5 mg; 84 Larazotide acetate 1 mg and 87 Larazotide acetate 2 mg) were included in the Safety Population and in the MITT Population.

Prior to breaking the study blind, tables and listings were reviewed to determine those subjects who would be included in the Per-Protocol Population. The pre-specified criteria that had to be met for a subject to be included in the Per-Protocol Population were:
- No major protocol deviations or violations. A subject was considered to have major protocol deviations or violations if 1) the subject entered the study with significant deviations from the inclusion/exclusion criteria that affected the subject's safety and/or efficacy results or 2) the subject took any prohibited concomitant medication for more than 10 days or had any procedures that affected the subject's safety and/or efficacy results.
- An average study drug compliance rate of at least 80%.
- Completed Visit 2 and was on active/double-blind treatment for a minimum of 53 days.

A total of 272 randomized subjects (74 placebo, 62 Larazotide acetate 0.5 mg, 64 Larazotide acetate 1 mg, and 72 Larazotide acetate 2 mg) were determined to be eligible for inclusion in the Per-Protocol Population. The most common reason for exclusion from the Per-Protocol Population was < 80% compliance with study drug.

**Table 7. Data Sets Analyzed - All Subjects**

| | **Placebo** | **Larazotide Acetate TID** | | | |
|---|---|---|---|---|---|
| | | **0.5 mg** | **1 mg** | **2 mg** | **Total** |
| Randomized, n | 84 | 86 | 85 | 87 | 342 |
| Safety Population^{a}, n (%) | 84 (100.0) | 85 (98.8) | 84 (98.8) | 87 (100.0) | 340 (99.4) |
| MITT Population^{b}, n (%) | 84 (100.0) | 85 (98.8) | 84 (98.8) | 87 (100.0) | 340 (99.4) |
| Per-Protocol Population^{c}, n (%) | 74 (88.1) | 62 (72.1) | 64 (75.3) | 72 (82.8) | 272 (79.5) |

| | | | | | |
|---|---|---|---|---|---|
| Abbreviations: MITT = Modified Intent-to-Treat; TID = three times daily. a Safety Population: all subjects who received at least 1 dose of study drug with treatment assignments designated according to the actual study treatment initially received; primary population for evaluating safety. b MITT Population: all randomized and treated subjects included in the assigned treatment groups. Subjects were included in particular analyses if they had post-baseline assessments; primary population for evaluating efficacy. c Per-Protocol Population: all subjects in the MITT Population who had no major protocol deviations or violations thought to impact the ability to assess the effect of treatment, had an average study drug compliance rate of at least 80%, and completed Visit 2 and were on active/double-blind treatment for a minimum of 53 days. | | | | | |

### 2. Baseline Demographic

Baseline demographic characteristics of the MITT/Safety Population are summarized by treatment group in Table 8. Overall, baseline demographics were similar among the placebo and Larazotide acetate treatment groups. The subject population was primarily female (83.5%) and white (98.8%), with a mean age of 45.1 years. Overall, 19.7% of the subjects were ≥ 60 years of age. Mean BMI was 26.6 kg/m² and the majority of the subjects had never smoked (68.8%).

**Table 8. Baseline Demographics - MITT/Safety Population**

| **Variable** | **Placebo (N=84)** | **Larazotide Acetate TID** | | | **mg Total (N=340)** |
|---|---|---|---|---|---|
| | | **0.5 (N=85)** | **mg 1 (N=84)** | **mg 2 (N=87)** | |
| Sex, n (%) | | | | | |
| Female | 69 (82.1) | 73 (85.9) | 70 (83.3) | 72 (82.8) | 284 (83.5) |
| Male | 15 (17.9) | 12 (14.1) | 14 (16.7) | 15 (17.2) | 56 (16.5) |
| Age (years) | 45.5 | 44.1 | 46.1 (15.07) | 44.7 (15.96) | 45.1 (14.99) |
| Mean (SD) | (14.61) | (14.42) | | | |
| Median | 46 | 46 | 47 | 50 | 47 |
| Minimum, maximum | 19,71 | 18, 73 | 18, 77 | 18, 73 | 18, 77 |
| Age Group, n (%) | | | | | |
| < 60 years | 68 (81.0) | 72 (84.7) | 63 (75.0) | 70 (80.5) | 273 (80.3) |
| ≥ 60 years | 16 (19.0) | 13 (15.3) | 21 (25.0) | 17 (19.5) | 67 (19.7) |
| Race, n (%) | | | | | |
| White | 83 (98.8) | 83 (97.6) | 84 (100.0) | 86 (98.9) | 336 (98.8) |
| Asian | 0 | 1 (1.2) | 0 | 1 (1.1) | 2 (0.6) |
| Black | 0 | 1 (1.2) | 0 | 0 | 1 (0.3) |
| American Indian/Alaskan Native | 1 (1.2) | 0 | 0 | 0 | 1 (0.3) |
| Ethnicity, n (%) | | | | | |
| Hispanic/Latino | 4 (4.8) | 3 (3.5) | 2 (2.4) | 2 (2.3) | 11 (3.2) |
| Not Hispanic/Latino | 80 (95.2) | 82 (96.5) | 82 (97.6) | 85 (97.7) | 329 (96.8) |
| BMI (kg/m²) | | | | | |
| Mean (SD) | 26.6 (5.41) | 27.6 (5.36) | 26.0 (4.64) | 26.1 (4.57) | 26.6 (5.02) |
| Median | 25.8 | 27.3 | 24.7 | 25.9 | 25.9 |
| Minimum, maximum | 18.5, 42.3 | 18.2,44.5 | 18.5,42.3 | 18.7, 38.1 | 18.2, 44.5 |
| Smoking History, n (%) | | | | | |
| Ex-smoker | 27 (32.1) | 29 (34.1) | 30 (35.7) | 20 (23.0) | 106 (31.2) |
| Never smoked | 57 (67.9) | 56 (65.9) | 54 (64.3) | 67 (77.0) | 234 (68.8) |

| | | | | | |
|---|---|---|---|---|---|
| Abbreviations: BMI = body mass index; MITT = modified intent-to-treat; SD = standard deviation; TID = three times daily. | | | | | |

### 3. Baseline Disease Characteristics

Baseline disease characteristics of the MITT/Safety Population are summarized by treatment group in Table 9. Overall, the mean time between celiac disease symptom onset and diagnosis and entry into the study was 158.1 (± 153.32) and 64.2 (± 59.26) months, respectively. However, the mean time between celiac disease symptom onset and diagnosis and entry into the study was greater in the Larazotide acetate 1 mg (162.9 and 66.7 months, respectively) and 2 mg (174.6 and 71.5 months, respectively) groups compared with the placebo (145.5 and 60.3 months, respectively) and Larazotide acetate 0.5 mg (149.2 and 58.3 months, respectively) groups.

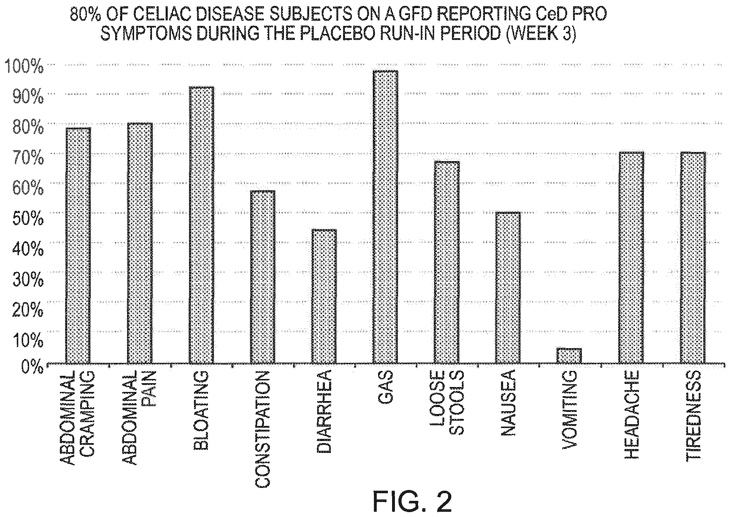

The majority of the subjects were diagnosed as having celiac disease by biopsy (97.4%). The disease presentation at initial diagnosis was generally similar among the placebo and Larazotide acetate treatment groups. Almost all subjects reported intestinal symptoms at the time of initial diagnosis, of which bloating (90.0%), recurrent abdominal pain (87.9%), diarrhea (78.2%), and abdominal distention/swelling in the stomach (76.5%) were the most common. Other extra-intestinal or associated diseases were reported for 76.5% of the population at the time of initial diagnosis, with no clinically important differences noted among the placebo and Larazotide acetate treatment groups for the incidences of specific diagnoses/diseases. As shown in Figure 2, more than 80% of celiac disease patients on a gluten-free diet reported CeD PRO symptoms during the placebo run-in period (week 3).

**Table 9 Baseline Disease Characteristics - MITT/Safety Population**

| **Variable** | **Placebo (N=84)** | **Larazotide Acetate TID** | | | |
|---|---|---|---|---|---|
| | | **0.5 mg (N=85)** | **1 mg (N=84)** | **2 mg (N=87)** | **Total (N=340)** |
| Time Since Celiac Disease Symptom Onset (months) | | | | | |
| Mean (SD) | 145.5 (141.13) | 149.2 (144.82) | 162.9 (150.61) | 174.6 (174.72) | 158.1 (153.32) |
| Median | 97.3 | 99.1 | 101.1 | 113.5 | 103.8 |
| Minimum, maximum | 17.6, 804.8 | 17.8, 728.3 | 14.2, 659.5 | 21.4, 868.4 | 14.2, 868.4 |

| Time Since Celiac Disease Diagnosis (months) | | | | | |
|---|---|---|---|---|---|
| N | 84 | 84 | 84 | 86 | 338 |
| Mean (SD) | 60.3 (51.27) | 58.3 (50.29) | 66.7 (68.20) | 71.5 (65.07) | 64.2 (59.26) |
| Median | 48.2 | 39.9 | 39.1 | 51.5 | 47 |
| Minimum, maximum | 13.8, 270.7 | 13.3,264.1 | 10.0, 439.1 | 8.1,321.0 | 8.1,439.1 |

| Method of Celiac Disease Diagnosis, n (%) | | | | | |
|---|---|---|---|---|---|
| Biopsy | 82 (97.6) | 84 (98.8) | 81 (96.4) | 84 (96.6) | 331 (97.4) |
| Capsule endoscopy with positive Celiac serology | 2 (2.4) | 0 | 2 (2.4) | 2 (2.3) | 6 (1.8) |
| Missing | 0 | 0 | 1 (1.2) | 0 | 0 |

| Time Since Start of Most Recent Gluten-Free Diet (months) | | | | | |
|---|---|---|---|---|---|
| Mean (SD) | 62.1 (58.73) | 60.7 (60.39) | 71.6 (86.69) | 70.9 (63.72) | 66.3 (68.14) |

**Table 9 Baseline Disease Characteristics - MITT/Safety Population**

| **Variable** | **Placebo (N=84)** | **Larazotide Acetate TID** | | | **mg Total (N=340)** |
|---|---|---|---|---|---|
| | | **0.5 (N=85)** | **mg 1 (N=84)** | **mg 2 (N=87)** | |
| Median Minimum, maximum | 49.1 13.8,367.6 | 39.1 13.0, 365.4 | 36.7 13.5, 546.7 | 50.2 13.6, 340.8 | 45 13.0, 546.7 |
| Celiac Disease Presentation at Initial Diagnosis, n (%) | | | | | |
| Intestinal | 84 (100.0) | 84 (98.8) | 84 (100.0) | 87 (100.0) | 339 (99.7) |
| Diarrhea | 67 (79.8) | 67 (78.8) | 68 (81.0) | 64 (73.6) | 266 (78.2) |
| Bloating | 75 (89.3) | 76 (89.4) | 77 (91.7) | 78 (89.7) | 306 (90.0) |
| Abdominal distention/swelling in the stomach | 64 (76.2) | 61 (71.8) | 67 (79.8) | 68 (78.2) | 260 (76.5) |
| Recurrent aphthous stomatitis/canker sores/lip, tongue or mouth ulcers | 25 (29.8) | 21 (24.7) | 23 (27.4) | 27 (31.0) | 96 (28.2) |
| Recurrent abdominal pain | 72 (85.7) | 77 (90.6) | 75 (89.3) | 75 (86.2) | 299 (87.9) |
| Steatorrhca/excessive fat in stool | 32 (38.1) | 24 (28.2) | 29 (34.5) | 29 (33.3) | 114 (33.5) |
| Other gastrointestinal features | 13 (15.5) | 22 (25.9) | 19 (22.6) | 23 (26.4) | 77 (22.6) |
| Extra-intestinal and associated disease | 62 (73.8) | 62 (72.9) | 71 (84.5) | 65 (74.7) | 260 (76.5) |
| Dermatitis herpetiformis | 17 (20.2) | 17 (20.0) | 21 (25.0) | 23 (26.4) | 78 (22.9) |
| Type 1 diabetes | 0 | 0 | 0 | 1 (1.1) | 1 (0.3) |
| Autoimmune thyroid disease | 13 (15.5) | 6 (7.1) | 13 (15.5) | 10 (11.5) | 42 (12.4) |
| Anemia | 33 (39.3) | 44 (51.8) | 42 (50.0) | 28 (32.2) | 147 (43.2) |
| Dental-enamel hypoplasia/tooth discoloration | 6 (7.1) | 9 (10.6) | 3 (3.6) | 12 (13.8) | 30 (8.8) |
| Osteopenia or osteoporosis | 22 (26.2) | 17 (20.0) | 23 (27.4) | 21 (24.1) | 83 (24.4) |
| Abnormal liver function tests | 5 (6.0) | 6 (7.1) | 5 (6.0) | 5 (5.7) | 21 (6.2) |
| Joint pain and/or joint disease | 35 (41.7) | 23 (27.1) | 31 (36.9) | 37 (42.5) | 126 (37.1) |
| Recurrent miscarriages/fertility problems | 11 (13.1) | 8 (9.4) | 7 (8.3) | 9 (10.3) | 35 (10.3) |
| Other extra-intestinal features and associated disease | 11 (13.1) | 13 (15.3) | 11 (13.1) | 13 (14.9) | 48 (14.1) |
| General | 11 (13.1) | 11 (12.9) | 11 (13.1) | 12 (13.8) | 45 (13.2) |
| Short stature | 3 (3.6) | 4 (4.7) | 1 (1.2) | 5 (5.7) | 13 (3.8) |
| Failure to thrive | 3 (3.6) | 2 (2.4) | 2 (2.4) | 5 (5.7) | 12 (3.5) |
| Other general features associated with Celiac | 7 (8.3) | 5 (5.9) | 8 (9.5) | 5 (5.7) | 25 (7.4) |

| | | | | | |
|---|---|---|---|---|---|
| Abbreviations: MITT = modified intent-to-treat; SD = standard deviation; TID = three times daily. | | | | | |

Significant ongoing medical histories reported by ≥ 10% of subjects in any treatment group are summarized for the MITT/Safety Population in Table 10. The majority of the subjects reported at least 1 significant ongoing medical history at baseline (97.6%). The most common ongoing medical histories included drug hypersensitivity (36.8%), seasonal allergy (25.0%), and gastro-oesophageal reflux disease (20.9%). No clinically important differences were observed among the placebo and Larazotide acetate treatment groups for the incidences of specific ongoing medical histories.

**Table 10. Significant Ongoing Medical History That Occurred in ≥10% of Any Treatment Group by Preferred Term - MITT/Safety Population**

| **Preferred Term, n (%)** | **Larazotide Acetate TID** | | | | |
|---|---|---|---|---|---|
| | **Placebo (N=84)** | **0.5 (N=85)** | **mg 1 (N=84)** | **mg 2 (N=87)** | **mg Total (N=340)** |
| Subjects With ≥ 1 Ongoing | 81 (96.4) | 84 (98.8) | 81 (96.4) | 86 (98.9) | 332 (97.6) |
| Medical History | | | | | |
| Drug hypersensitivity | 28 (33.3) | 31 (36.5) | 28 (33.3) | 38 (43.7) | 125 (36.8) |
| Seasonal allergy | 18 (21.4) | 22 (25.9) | 20 (23.8) | 25 (28.7) | 85 (25.0) |
| Gastro-oesophageal reflux disease | 15 (17.9) | 22 (25.9) | 17 (20.2) | 17 (19.5) | 71 (20.9) |
| Anxiety | 17 (20.2) | 20 (23.5) | 15 (17.9) | 15 (17.2) | 67 (19.7) |
| Depression | 14 (16.7) | 22 (25.9) | 12 (14.3) | 16 (18.4) | 64 (18.8) |
| Hypothyroidism | 16 (19.0) | 11 (12.9) | 18 (21.4) | 15 (17.2) | 60 (17.6) |
| Insomnia | 8 (9.5) | 14 (16.5) | 21 (25.0) | 17 (19.5) | 60 (17.6) |
| Anaemia | 9 (10.7) | 23 (27.1) | 18 (21.4) | 9 (10.3) | 59 (17.4) |
| Arthralgia | 19 (22.6) | 9 (10.6) | 16 (19.0) | 14 (16.1) | 58 (17.1) |
| Hypertension | 14 (16.7) | 11 (12.9) | 13 (15.5) | 13 (14.9) | 51 (15.0) |
| Asthma | 11 (13.1) | 11 (12.9) | 13 (15.5) | 14 (16.1) | 49 (14.4) |
| Osteopenia | 10 (11.9) | 9 (10.6) | 16 (19.0) | 13 (14.9) | 48 (14.1) |
| Migraine | 14 (16.7) | 9 (10.6) | 14 (16.7) | 9 (10.3) | 46 (13.5) |
| Constipation | 8 (9.5) | 10 (11.8) | 12 (14.3) | 9 (10.3) | 39(11.5) |
| Diarrhoea | 10 (11.9) | 9 (10.6) | 10 (11.9) | 9 (10.3) | 38 (11.2) |
| Headache | 5 (6.0) | 14 (16.5) | 10 (11.9) | 9 (10.3) | 38 (11.2) |
| Dermatitis herpetiformis | 9 (10.7) | 10 (11.8) | 7 (8.3) | 8 (9.2) | 34 (10.0) |
| Osteoporosis | 11 (13.1) | 7 (8.2) | 6 (7.1) | 8 (9.2) | 32 (9.4) |
| Back pain | 4 (4.8) | 3 (3.5) | 9 (10.7) | 8 (9.2) | 24(7.1) |
| Fatigue | 3 (3.6) | 9 (10.6) | 2 (2.4) | 4 (4.6) | 18 (5.3) |

| | | | | | |
|---|---|---|---|---|---|
| Abbreviations: MedDRA = Medical Dictionary for Regulatory Activities; TID = three times daily. Note: A subject with multiple histories is counted only once in each category; medical histories are coded using MedDRA v14.1. | | | | | |

### 4. Prior and Concomitant Medications

Prior medication use was similar among the treatment groups (placebo 96.4%; Larazotide acetate 0.5 mg 91.8%; Larazotide acetate 1 mg 95.2%; Larazotide acetate 2 mg 95.4%). Overall, the most common types (≥ 10% of all subjects) of prior medications used were multivitamins (29.7%), vitamin D and analogues (22.9%), selective serotonin reuptake inhibitors (21.2%), thyroid hormones (18.5%), propionic acid derivatives (17.6%), H₂-receptor antagonists (17.4%), progestogens and estrogens, fixed combinations (16.5%), anilides (15.9%), calcium (15.9%), other lipid modifying agents (14.1%), proton pump inhibitors (12.4%), benzodiazepine derivatives (12.1%), other antihistamines for systemic use (11.2%), HMG-CoA reductase inhibitors (10.0%), and platelet aggregation inhibitors excluding heparin (10.0%).

Concomitant medication use was similar among the treatment groups (placebo 95.2%; Larazotide acetate 0.5 mg 92.9%; Larazotide acetate 1 mg 96.4%; Larazotide acetate 2 mg 95.4%). Among subjects treated with Larazotide acetate, the most commonly used (≥ 20% of subjects) concomitant medications by ATC class were multivitamins, other combinations (30.9%), anilides (25.4%), and Vitamin D and analogues (23.0%). Concomitant medications by preferred term used by at least 10% of subjects in either the pooled Larazotide acetate group or the placebo group included multivitamins (30.1% and 23.8%, respectively); Vitamin D NOS (18.4% and 14.3%, respectively); paracetamol (15.6% and 4.8%, respectively); calcium (13.3% and 13.1%, respectively); ibuprofen (12.9% and 16.7%, respectively); fish oil (9.8% and 15.5%, respectively); acetylsalicyclic acid (9.4% and 10.7%, respectively); and levothyroxine sodium (8.2% and 11.9%, i

Concomitant medications used by ≥ 5.0% of the pooled Larazotide acetate group are summarized by anatomical therapeutic chemical class and individual treatment group for the MITT/Safety Population in Table 11. Concomitant corticosteroids were predominantly topical (eg, hydrocortisone, triamcinolone) or inhalation (*e.g*., fluticasone, budesonide) agents. One Larazotide acetate (0.5 mg) subject and 2 placebo subjects reported use of prednisolone/prednisolone acetate; 1 Larazotide acetate (2 mg) subject reported use of corticosteroid NOS for systemic use.

**Table 11 Concomitant Medications by Anatomical Therapeutic Chemical Class Used by ≥ 5.0% of Total Larazotide Acetate Group - Safety Population**

| **Anatomical Therapeutic Chemical** Class, n (%) | **Placebo (N=84)** | Larazotide **Acetate TID** | | | |
|---|---|---|---|---|---|
| | | **0.5 (N=85)** | **mg 1 (N=84)** | **mg 2 (N=87)** | **mg Pooled Doses (N=256)** |
| Subjects with at least 1 concomitant medication | 80 (95.2) | 79 (92.9) | 81 (96.4) | 83 (95.4) | 242 (94.9) |
| Multivitamins, Other Combinations | 20 (23.8) | 27 (31.8) | 25 (29.8) | 27 (31.0) | 79 (30.9) |
| Anilides | 12 (14.3) | 18 (21.2) | 22 (26.2) | 25 (28.7) | 65 (25.4) |
| Vitamin D and Analogues | 19 (22.6) | 20 (23.5) | 20 (23.8) | 19 (21.8) | 59 (23.0) |
| H₂-Receptor Antagonists | 11 (13.1) | 19 (22.4) | 15 (17.9) | 16 (18.4) | 50 (19.5) |
| Selective Serotonin Reuptake Inhibitors | 20 (23.8) | 21 (24.7) | 15 (17.9) | 14 (16.1) | 50 (19.5) |
| Propionic Acid Derivatives | 20 (23.8) | 20 (23.5) | 13 (15.5) | 12 (13.8) | 45 (17.6) |
| Thyroid Hormones | 18 (21.4) | 10 (11.8) | 18 (21.4) | 17 (19.5) | 45 (17.6) |
| Progestogens and Estrogens, Fixed Combinations | 12 (14.3) | 16 (18.8) | 14(16.7) | 11 (12.6) | 41 (16.0) |
| Calcium | 12 (14.3) | 13 (15.3) | 13 (15.5) | 14. (16.1) | 40 (15.6) |
| Benzodiazepine Derivatives | 9 (10.7) | 10(11.8) | 14 (16.7) | 11 (12.6) | 35 (13.7) |
| Other Lipid Modifying Agents | 13 (15.5) | 14 (16.5) | 10 (11.9) | 7 (8.0) | 31 (12.1) |
| Other Antihistamines for Systemic Use | 7 (8.3) | 10(11.8) | 11 (13.1) | 9 (10.3) | 30 (11.7) |
| HMG COA Reductase Inhibitors | 10 (11.9) | 4 (4.7) | 13 (15.5) | 7 (8.0) | 24 (9.4) |
| Other Antidepressants | 6 (7.1) | 11 (12.9) | 4 (4.8) | 9 (10.3) | 24 (9.4) |
| Platelet Aggregation Inhibitors excl. Heparin | 9 (10.7) | 7 (8.2) | 11 (13.1) | 6 (6.9) | 24 (9.4) |
| Benzodiazepine Related Drugs | 3 (3.6) | 5 (5.9) | 7 (8.3) | 11 (12.6) | 23 (9.0) |
| Selective Beta-2-Adrenoreceptor Agonists | 6 (7.1) | 6 (7.1) | 9 (10.7) | 6 (6.9) | 21 (8.2) |
| Corticosteroids | 11 (3.1) | 7 (8.2) | 4 (4.8) | 9 (10.3) | 20 (7.8) |
| Calcium, Combinations with Other Drugs | 6 (7.1) | 7 (8.2) | 5 (6.0) | 6 (6.9) | 18. (7.0) |
| Natural Opium Alkaloids | 6 (7.1) | 9 (10.6) | 3 (3.6) | 6 (6.9) | 18 (7.0) |
| Ascorbic Acid (Vitamin C), Plain | 3 (3.6) | 5 (5.9) | 4 (4.8) | 8 (9.2) | 17 (6.6) |
| Glucocorticoids | 2 (2.4) | 7 (8.2) | 4 (4.8) | 6 (6.9) | 17 (6.6) |
| Natural and Semisynthetic Estrogens, Plain. | 5 (6.0) | 4 (4.7) | 4 (4.8) | 8 (9.2) | 16 (6.3) |
| Piperazine Derivatives | 10 (11.9) | 5 (5.9) | 3 (3.6) | 8 (9.2) | 16 (6.3) |
| Antianemic Preparations | 6 (7.1) | 3 (3.5) | 8 (9.5) | 4 (4.6) | 15 (5.9) |
| Vitamin B-Complex, Plain | 1 (1.2) | 6 (7.1) | 3 (3.6) | 6 (6.9) | 15 (5.9) |
| ACE Inhibitors, Plain | 5 (6.0) | 4 (4.7) | 6 (7.1) | 4 (4.6) | 14 (5.5) |
| Unspecified Herbal and Traditional Medicine | 8 (9.5) | 3 (3.5) | 7 (8.3) | 4 (4.6) | 14 (5.5) |

| | | | | | |
|---|---|---|---|---|---|
| Abbreviations: TID = three times daily. | | | | | |

### 6. Measurement of Treatment Compliance

In the MITT/Safety Population, overall compliance with study drug was > 90% in each of the treatment groups (92.3% placebo; 90.9% Larazotide acetate 0.5 mg; 90.9% Larazotide acetate 1 mg; and 93.6% Larazotide acetate 2 mg). In each treatment group, some subjects had calculated compliance rates > 100%. Subjects who failed to return study drug at any of their visits would have appeared to have higher compliance rates.

Results of the Gluten-Free Diet Compliance Questionnaire (GFDCQ) showed that the majority of the subjects in each treatment group were following a GFD (≥ 92.0%) and most were very confident that they understood the GFD (≥ 85.1%). Based on the results of GFDCQ, a higher proportion of subjects in the Larazotide acetate 0.5 mg dose group reported that they had knowingly ingested food or beverages containing gluten (35%) during the study compared with the other treatment groups (27% in the placebo and 25% in the 1 mg group and 22% in the 2 mg dose group). Similarly, 59% of the 0.5 mg Larazotide acetate group reported that they had ingested something they thought was gluten-free but later learned it was not, compared with 52%, 39% and 38% of the placebo and Larazotide acetate 1 mg and 2 mg dose groups, respectively.

Results of the Celiac Disease Adherence Test (CDAT), which was administered at the end of the placebo run-out, showed that the majority of the subjects in each treatment group considered accidental exposures to gluten very or somewhat important to their health (≥ 83.3%) and that most were able to follow the GFD when dining outside the home (≥ 72.6%); few subjects reported eating foods containing gluten more than 6 times over the past 4 weeks (≤ 9.5%), which was the placebo run-out period. No important differences were observed among the placebo and Larazotide acetate treatment groups for responses to the CDAT.

### 7. Efficacy Results and Tabulations of Individual Subject Data

The Larazotide acetate 0.5 mg dose met the primary endpoint, average on-treatment CeD GSRS score, with benefit over placebo also shown in secondary and exploratory endpoints. The higher doses of Larazotide acetate (1 mg and 2 mg) did not demonstrate any improvement over placebo. Thus, the presentation of efficacy results focuses on the Larazotide acetate 0.5 mg group compared to placebo.

### A. Primary Efficacy Endpoint:

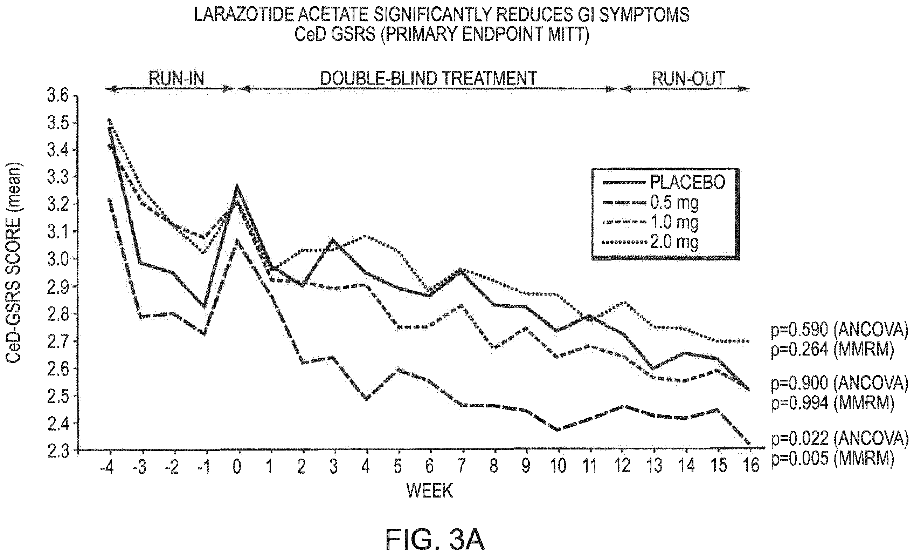

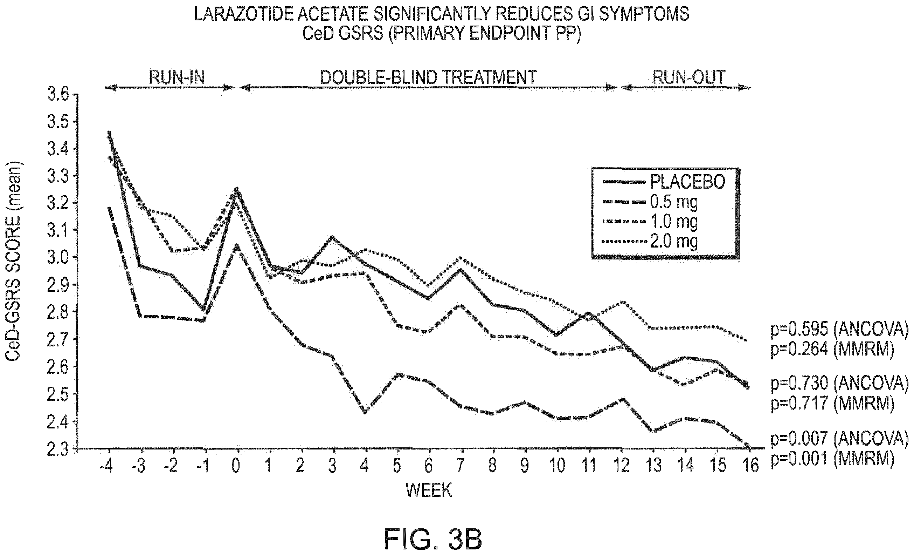

The primary efficacy endpoint, average on-treatment CeD GSRS score, is summarized for the MITT Population in Table 12. Gastrointestinal symptoms, as measured by average on-treatment CeD GSRS score, were reduced in both the Larazotide acetate 0.5 mg (3.05 to 2.59) and placebo (3.26 to 2.88) groups, with a statistically significantly lower mean difference of -0.23 in favor of the Larazotide acetate 0.5 mg group. Results were similar for the primary ANCOVA (difference from placebo -0.23) and supportive MMRM (difference from placebo -0.22) statistical models in the MITT Population (Table 12). Similar results were observed in the Per-Protocol Population. Figures 3A and 3B show that Larazotide acetate significantly reduced gastrointestinal symptoms as measured by the average on-treatment CeD GSRS score.

**Table 12. Average On-Treatment CeD GSRS Score - MITT Population**

| | | **Larazotide Acetate TID** | | | |
|---|---|---|---|---|---|
| | **Placebo (N=84)** | **0.5 (N=85)** | **mg 1 (N=84)** | **mg 2 (N=87)** | **mg Pooled Doses (N=256)** |
| Baseline score | | | | | |
| N | 84 | 85 | 84 | 87 | 256 |
| Mean (SD) | 3.26 (0.92) | 3.05 (0.84) | 3.21 (0.77) | 3.25 (0.96) | 3.17 (0.86) |
| Average on-treatment score | | | | | |
| N | 80 | 81 | 81 | 86 | 248 |
| Mean (SD) | 2.88 (0.72) | 2.59 (0.68) | 2.84 (0.78) | 2.92 (0.84) | 2.79 (0.78) |

| | | **Difference fr om Placebo** | | | |
|---|---|---|---|---|---|
| ANCOVA results^{a} | | | | | |
| Least squares mean | | -0.23 | -0.01 | 0.05 | -0.06 |
| 95% confidence interval | | -0.43, -0.03 | -0.20, 0.17 | -0.13,0.23 | -0.22, 0.09 |
| p-value | | 0.022 | 0.900 | 0.590 | 0.409 |
| MMRM results^{b} | | | | | |
| Least squares mean | | -0.22 | -0.00 | 0.08 | -0.05 |
| 95% confidence interval | | -0.38, -0.07 | -0.15, 0.14 | -0.06, 0.22 | -0.17, 0.07 |
| p-value | | 0.005 | 0.994 | 0.264 | 0.437 |

| | | | | | |
|---|---|---|---|---|---|
| Abbreviations: ANCOVA = analysis of covariance; CeD GSRS = celiac disease domain of the Gastrointestinal Symptom Rating Scale; MITT = modified intent-to-treat; MMRM = mixed model for repeated measures; SD = standard deviation; TID = three times daily. a Model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

### B. Change from Baseline to End of Treatment in the CeD GSRS Score:

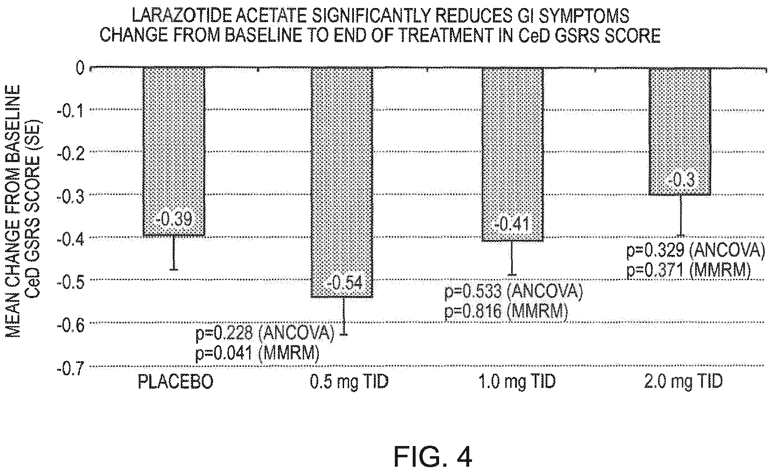

Change from baseline to end of treatment in the CeD GSRS score is summarized for the MITT Population in Table 13. Gastrointestinal symptoms, as measured by change from baseline to end of treatment in CeD GSRS score, were reduced in the Larazotide acetate 0.5 mg group compared to placebo; the difference (-0.17) was not statistically significant for the ANCOVA model in the MITT or Per-Protocol Populations. For the MMRM statistical model, the difference from placebo (-0.26) was statistically significant in the MITT Population, but not in the Per-Protocol Population, although trends in favor of Larazotide acetate were observed in all parameters at the 0.5 mg dose. Figure 4 shows that Larazotide acetate significantly reduced gastrointestinal syndromes as measured by change from baseline to the end of treatment in CeD GSRS score.

**Table 13. Change From Baseline to End of Treatment in the CeD GSRS Score - MITT Population**

| | | **Larazotide Acetate TID** | | | |
|---|---|---|---|---|---|
| | **Placebo (N=84)** | **0.5 (N=85)** | **mg 1 (N=84)** | **mg 2 (N=87)** | **mg Pooled Doses (N=256)** |
| Baseline score | | | | | |
| N | 84 | 85 | 84 | 87 | 256 |
| Mean (SD) | 3.26 (0.92) | 3.05 (0.84) | 3.21 (0.77) | 3.25 (0.96) | 3.17 (0.86) |
| End of treatment | | | | | |
| N | 80 | 81 | 81 | 86 | 248 |
| Mean (SD) of actual | 2.74 (0.93) | 2.54 (0.81) | 2.80 (0.97) | 2.87 (1.05) | 2.74 (0.96) |
| Mean (SD) of change | -0.50 (0.99) | -0.54 (0.88) | -0.40 (0.95) | -0.37 (1.03) | -0.43 (0.96) |

| | | **Difference from Placebo in Actual Score** | | | |
|---|---|---|---|---|---|
| ANCOVA results^{a} | | | | | |
| Least squares mean | | -0.17 | 0.08 | 0.13 | 0.01 |
| 95% confidence interval | | -0.5,0.1 | -0.2, 0.3 | -0.1,0.4 | -0.2, 0.2 |
| p-value | | 0.228 | 0.533 | 0.329 | 0.903 |
| MMRM results^{b} | | | | | |
| Least squares mean | | -0.26 | 0.03 | 0.11 | -0.04 |
| 95% confidence interval | | -0.51,0.0 | -0.22, 0.3 | -0.13, 0.4 | -0.24, 0.2 |
| p-value | | 0.041 | 0.816 | 0.371 | 0.700 |

| | | | | | |
|---|---|---|---|---|---|
| Abbreviations: ANCOVA = analysis of covariance; CeD GSRS = celiac disease domain of the Gastrointestinal Symptom Rating Scale; MITT = modified intent-to-treat; MMRM = mixed model for repeated measures; SD = standard deviation; TID = three times daily. a Model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

Based on the ANCOVA and MMRM models for the MITT Population, statistically significantly lower mean CeD GSRS scores (ie, fewer symptoms) were observed in the Larazotide acetate 0.5 mg group compared to the placebo group at Week 3 (-0.33 and -0.37, respectively), Week 4 (-0.41 and -0.32, respectively), Week 7 (-0.38 and -0.30, respectively), Week 8 (-0.33 and -0.28, respectively), Week 9 (-0.31 and - 0.27, respectively), and Week 11 (-0.33 and -0.31, respectively); the results were also statistically significant at Week 10 (-0.30) for the MMRM model.

### C. Average On-Treatment Score of the CeD PRO Abdominal Domain:

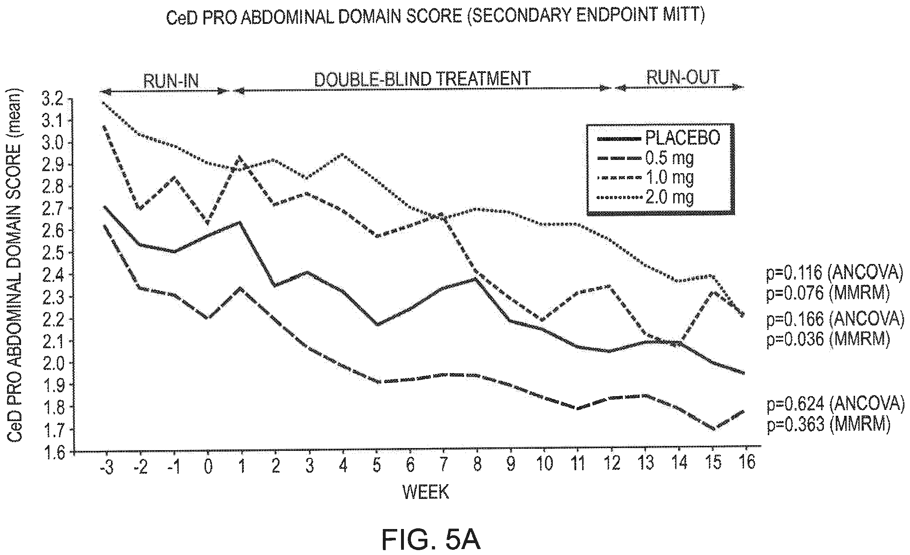

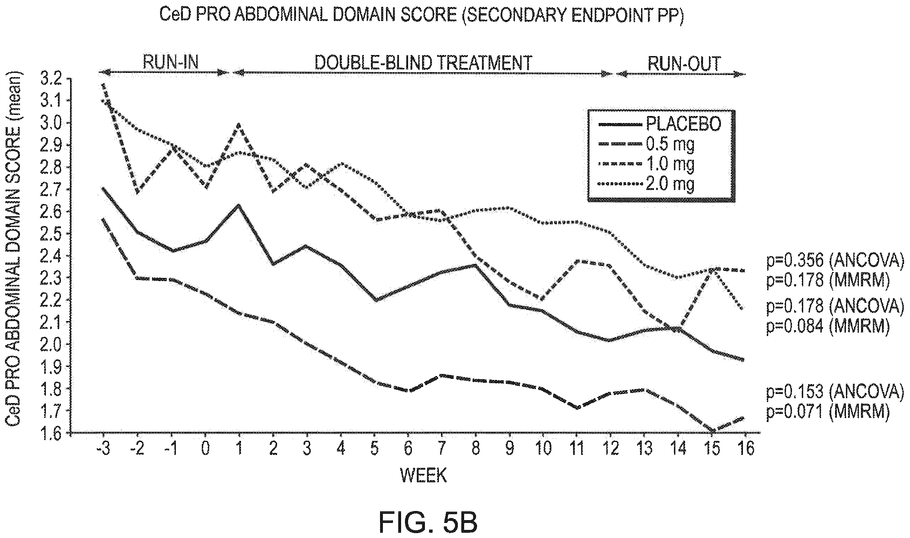

The average on-treatment score of the CeD PRO Abdominal Domain is summarized for the MITT Population in Table 14. For the average on-treatment CeD PRO Abdominal Domain score, the Larazotide acetate 0.5 mg group was trending but not statistically significantly different from placebo for the ANCOVA model (-0.08) or the MMRM model (-0.11) in the MITT Population. Similar results were observed for the Per-Protocol Population. Mean weekly CeD PRO Abdominal Domain scores are summarized in Figures 5A and 5B.

**Table 14. Average On-Treatment CeD PRO Abdominal Domain Score - MITT Population**

| | **Placebo (N=84)** | **Larazotide Acetate TID** | | | |
|---|---|---|---|---|---|
| | | **0.5 (N=85)** | **mg 1 (N=84)** | **mg 2 (N=87)** | **mg Pooled Doses (N=256)** |
| Baseline score | | | | | |
| N | 84 | 84 | 84 | 87 | 255 |
| Mean (SD) | 2.56 (1.71) | 2.19 (1.34) | 2.63 (1.59) | 2.90 (1.58) | 2.58 (1.53) |
| Average on-treatment score | | | | | |
| N | 81 | 84 | 84 | 87 | 255 |
| Mean (SD) | 2.27 (1.30) | 2.04 (1.28) | 2.59 (1.55) | 2.77 (1.50) | 2.47 (1.48) |

| | | **Difference from Placebo** | | | |
|---|---|---|---|---|---|
| ANCOVA results^{a} | | | | | |
| Least squares mean | | -0.08 | 0.22 | 0.25 | 0.13 |
| 95% confidence interval | | -0.4, 0.2 | -0.1,0.5 | -0.1,0.6 | -0.1,0.4 |
| p-value | | 0.624 | 0.166 | 0.116 | 0.327 |
| MMRM results^{b} | | | | | |
| Least squares mean | | -0.11 | 0.24 | 0.20 | 0.11 |
| 95% confidence interval | | -0.4, 0.1 | 0.0, 0.5 | -0.0, 0.4 | -0.1, 0.3 |
| p-value | | 0.363 | 0.036 | 0.076 | 0.243 |

| | | | | | |
|---|---|---|---|---|---|
| Abbreviations: ANCOVA = analysis of covariance; CeD = celiac disease; GSRS = Gastrointestinal Symptom Rating Scale; MITT = modified intent-to-treat; MMRM = mixed model for repeated measures; PRO = patient reported outcome; SD = standard deviation; TID = three times daily. a Model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

### D. Change from Baseline to End of Treatment in the CeD PRO Abdominal Domain:

Change from baseline to end of treatment in the CeD PRO Abdominal Domain score is summarized for the MITT Population in Table 15. For the change from baseline to the end of treatment in the CeD PRO Abdominal Domain score, the Larazotide acetate 0.5 mg group was not statistically significantly different from placebo for the ANCOVA model (-0.02) or the MMRM model (-0.04) in the MITT Population. Similar results were observed for the Per-Protocol Population.

**Table 15. Change From Baseline to End of Treatment in the CeD PRO Abdominal Domain - MITT Population**

| | **Placebo (N=84)** | **Larazotide Acetate** TID | | | |
|---|---|---|---|---|---|
| | | **0.5 (N=85)** | **mg 1 (N=84)** | **mg 2 (N=87)** | **mg Pooled Doses (N=256)** |
| Baseline score | | | | | |
| N | 84 | 84 | 84 | 87 | 255 |
| Mean (SD) | 2.56 (1.71) | 2.19 (1.34) | 2.63 (1.59) | 2.90 (1.58) | 2.58 (1.53) |
| End of treatment | | | | | |
| N | 81 | 84 | 84 | 87 | 255 |
| Mean (SD) of actual | 2.04 (1.49) | 1.93 (1.47) | 2.45 (1.72) | 2.74 (1.75) | 2.38 (1.68) |
| Mean (SD) of change | -0.43 (1.73) | -0.26 (1.47) | -0.18 (1.27) | -0.16 (1.32) | -0.20 (1.35) |

| | | **Difference** | **from Placebo** | **in Actual Score** | |
|---|---|---|---|---|---|
| ANCOVA results^{a} | | | | | |
| Least squares mean | | -0.02 | 0.32 | 0.47 | 0.25 |
| 95% confidence interval | | -0.4, 0.4 | -0.1,0.7 | 0.1, 0.9 | -0.1,0.6 |
| p-value | | 0.932 | 0.125 | 0.023 | 0.135 |
| MMRM results^{b} | | | | | |
| Least squares mean | | -0.04 | 0.33 | 0.35 | 0.21 |
| 95% confidence interval | | -0.4, 0.4 | -0.1, 0.7 | -0.0, 0.7 | -0.1, 0.5 |
| p-value | | 0.860 | 0.092 | 0.072 | 0.180 |

| | | | | | |
|---|---|---|---|---|---|
| Abbreviations: ANCOVA = analysis of covariance; CeD = celiac disease; GSRS = Gastrointestinal Symptom Rating Scale; MITT = modified intent-to-treat; MMRM = mixed model for repeated measures; PRO = patient reported outcome; SD = standard deviation; TID = three times daily. a Model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

No statistically significant differences from placebo were observed for the Larazotide acetate 0.5 mg group in the change from baseline to each study week in the CeD PRO Abdominal Domain for the MITT Population. In the Per-Protocol Population, a statistically significant difference from placebo was observed in the Larazotide acetate 0.5 mg group (-0.43) at Week 8 for the MMRM model.

### E. Average On-Treatment Score of the CeD PRO Gastrointestinal Domain:

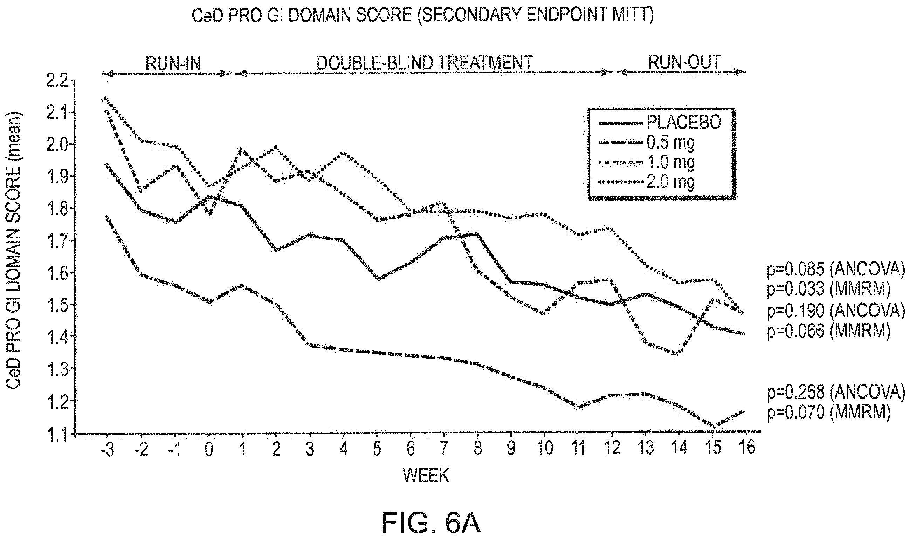

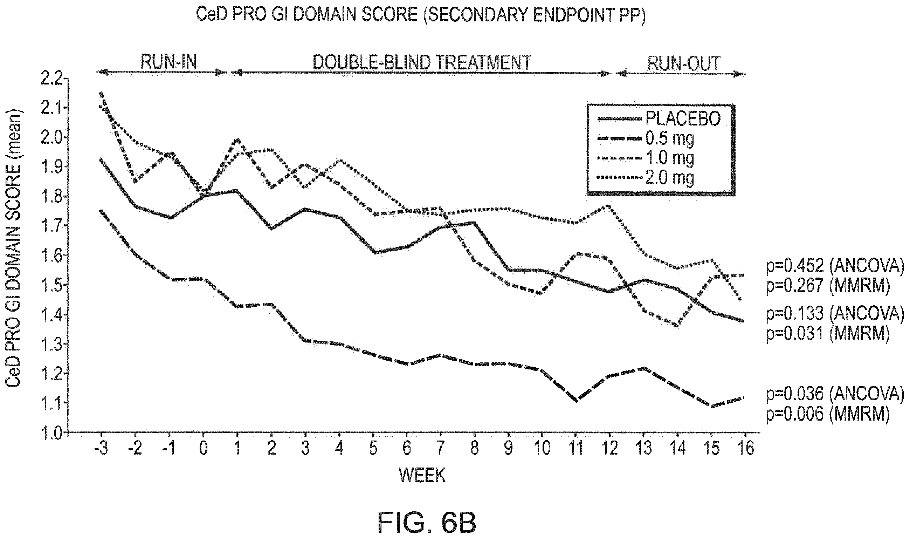

The average on-treatment score of the CeD PRO Gastrointestinal Domain is summarized for the MITT Population in Table 16. The average on-treatment CeD PRO Gastrointestinal Domain score was reduced in both the Larazotide acetate 0.5 mg (1.86 to 1.69) and placebo (2.27 to 2.054) groups; the difference was not statistically significant for the ANCOVA model (-0.16) or the MMRM model (-0.19) in the MITT Population. In the Per-Protocol Population, a statistically significant difference from placebo was observed in the Larazotide acetate 0.5 mg group (-0.33; p=0.036) based on the ANOVA model as well as the MMRM model (-0.32; p=0.006). Mean CeD PRO Gastrointestinal Domain scores are summarized in Figures 6A and 6B.

**Table 16. Average On-Treatment CeD PRO Gastrointestinal Domain Score - MITT Population**

| | **Placebo (N=84)** | **Larazotide Acetate TID** | | | |
|---|---|---|---|---|---|
| | | **0.5 (N=85)** | **mg 1 (N=84)** | **mg 2 mg (N=87)** | **Pooled Doses (N=256)** |
| Baseline score | | | | | |
| N | 84 | 84 | 84 | 87 | 255 |
| Mean (SD) | 2.27 (1.46) | 1.86 (1.10) | 2.23 (1.29) | 2.35 (1.29) | 2.15 (1.24) |
| Average on-treatment score | | | | | |
| N | 81 | 84 | 84 | 87 | 255 |
| Mean (SD) | 2.05 (1.19) | 1.69 (1.01) | 2.21 (1.33) | 2.31 (1.26) | 2.07 (1.23) |

| | | **Difference from Placebo** | | | |
|---|---|---|---|---|---|
| ANCOVA results^{a} | | | | | |
| Least squares mean | | -0.16 | 0.15 | 0.19 | 0.06 |
| 95% confidence interval | | -0.4,0.1 | -0.1,0.4 | -0.1,0.4 | -0.2,0.3 |
| p-value | | 0.268 | 0.263 | 0.152 | 0.584 |
| MMRM results^{b} | | | | | |
| Least squares mean | | -0.19 | 0.16 | 0.16 | 0.05 |
| 95% confidence interval | | -0.4, 0.0 | -0.0, 0.4 | -0.0, 0.4 | -0.1, 0.2 |
| p-value | 0.070 | 0.092 | 0.093 | 0.570 | |

| | | | | | |
|---|---|---|---|---|---|
| Abbreviations: ANCOVA = analysis of covariance; CeD = celiac disease; GSRS = Gastrointestinal Symptom Rating Scale; MITT = modified intent-to-treat; MMRM = mixed model for repeated measures; PRO = patient reported outcome; SD = standard deviation; TID = three times daily. a Model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

### F. Change from Baseline to End of Treatment in the CeD PRO Gastrointestinal Domain

Change from baseline to end of treatment in the CeD PRO Gastrointestinal Domain score is summarized for the MITT Population in Table 17. For the change from baseline to the end of treatment in the CeD PRO Gastrointestinal Domain score, the Larazotide acetate 0.5 mg group was not statistically significant different from placebo for the ANCOVA model (-0.11) or the MMRM model (-0.12) in the MITT Population. Similar results were observed for the Per-Protocol Population.

**Table 17. Change From Baseline to End of Treatment in the CeD PRO Gastrointestinal Domain - MITT Population**

| | | **Larazotide Acetate TID** | | | |
|---|---|---|---|---|---|
| | **Placebo (N=84)** | **0.5 (N=85)** | **mg 1 (N=84)** | **mg 2 mg (N=87)** | **Pooled Doses (N=256)** |
| Baseline score | | | | | |
| N | 84 | 84 | 84 | 87 | 255 |
| Mean (SD) | 2.27 (1.46) | 1.86 (1.10) | 2.23 (1.29) | 2.35 (1.29) | 2.15 (1.24) |
| End of treatment | | | | | |
| N | 81 | 84 | 84 | 87 | 255 |
| Mean (SD) of actual | 1.84 (1.35) | 1.59 (1.19) | 2.10 (1.44) | 2.35 (1.50) | 2.02 (1.41) |
| Mean (SD) of change | -0.38 (1.45) | -0.28 (1.24) | -0.13 (1.00) | -0.00 (1.23) | -0.13 (1.16) |

| | | **Difference from Placebo in Actual Score** | | | |
|---|---|---|---|---|---|
| ANCOVA results^{a} | | | | | |
| Least squares mean | | -0.11 | 0.25 | 0.43 | 0.19 |
| 95% confidence interval | | -0.5, 0.3 | -0.1, 0.6 | 0.1, 0.8 | -0.1, 0.5 |
| p-value | | 0.548 | 0.165 | 0.013 | 0.196 |
| MMRM results^{b} | | | | | |
| Least squares mean | | -0.12 | 0.24 | 0.36 | 0.16 |
| 95% confidence interval | | -0.5, 0.2 | -0.1,0.6 | 0.0, 0.7 | -0.1,0.4 |
| p-value | | 0.485 | 0.150 | 0.028 | 0.238 |

| | | | | | |
|---|---|---|---|---|---|
| Abbreviations: ANCOVA = analysis of covariance; CeD = celiac disease; GSRS = Gastrointestinal Symptom Rating Scale; MITT = modified intent-to-treat; MMRM = mixed model for repeated measures; PRO = patient reported outcome; SD = standard deviation; TID = three times daily. a Model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

The mean change from baseline to each study week in the CeD PRO Gastrointestinal Domain is summarized by study week for the MITT Population and for the Per-Protocol Population. Based on the MMRM model for the MITT Population, the mean decrease from baseline in the CeD PRO Gastrointestinal Domain was statistically significantly larger in the Larazotide acetate 0.5 mg group compared to the placebo group at Week 7 and Week 8. Based on the ANCOVA and MMRM models for the Per-Protocol Population, the mean decrease from baseline in the CeD PRO Gastrointestinal Domain was statistically significantly larger in the Larazotide acetate 0.5 mg group compared to the placebo group at Week 1, Week 3, Week 8, and Week 11; the difference from placebo was also statistically significant at Week 4 and Week 14 in the ANCOVA model. The difference from placebo was also statistically significant at Week 6 and Week 7 in the MMRM model.

### G. Average On-Treatment Total GSRS Score:

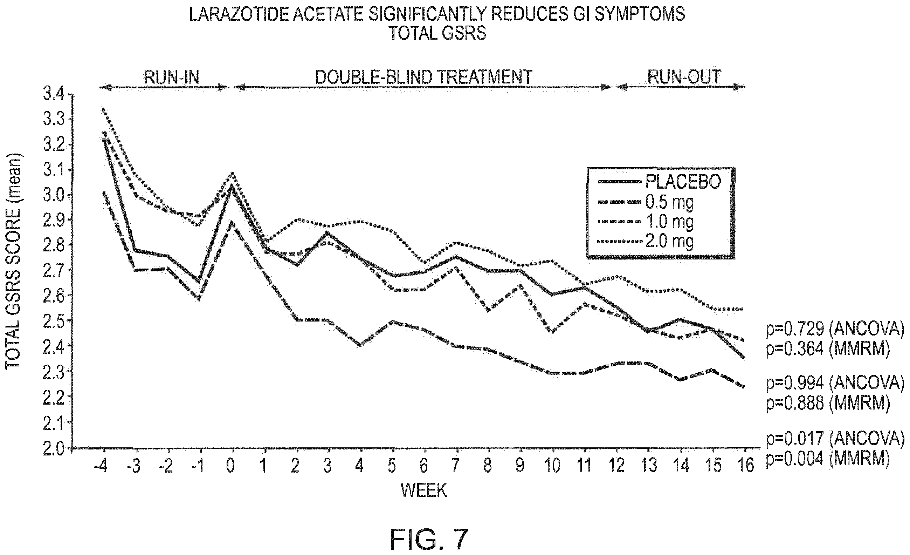

The average on-treatment total GSRS score is summarized for the MITT Population in Table 18. The average on-treatment total GSRS score was reduced in both the Larazotide acetate 0.5 mg (2.89 to 2.47) and placebo (3.03 to 2.70) groups, with a statistically significant mean difference of -0.22 in favor of the Larazotide acetate 0.5 mg group. Results were similar for the ANCOVA (difference from placebo -0.22) and MMRM (difference from placebo -0.21) statistical models in the MITT Population (Table 18). Similar results were observed in the Per-Protocol Population. Figure 7 shows that Larazotide acetate significantly reduced gastrointestinal symptoms as measured by total GSRS score.

**Table 18. Average On-Treatment Total GSRS Score - MITT Population**

| | | **Larazotide Acetate TID** | | | |
|---|---|---|---|---|---|
| | **Placebo (N=84)** | **0.5 (N=85)** | **mg 1 (N=84)** | **mg 2 mg (N=87)** | **Pooled Doses (N=256)** |
| Baseline score | | | | | |
| N | 84 | 85 | 84 | 87 | 256 |
| Mean (SD) | 3.03 (0.82) | 2.89 (0.80) | 3.03 (0.75) | 3.09 (0.89) | 3.00 (0.82) |
| Average on-treatment score | | | | | |
| N | 80 | 81 | 81 | 86 | 248 |
| Mean (SD) | 2.70 (0.67) | 2.47 (0.65) | 2.70 (0.75) | 2.76 (0.79) | 2.64 (0.74) |

| | | **Difference from Placebo** | | | |
|---|---|---|---|---|---|
| ANCOVA results^{a} | | | | | |
| Least squares mean | | -0.22 | 0.00 | 0.03 | -0.06 |
| 95% confidence interval | | -0.4, -0.0 | -0.2, 0.2 | -0.1,0.2 | -0.2, 0.1 |
| p-value | | 0.017 | 0.994 | 0.729 | 0.373 |
| MMRM results^{b} | | | | | |
| Least squares mean | | -0.21 | 0.01 | 0.06 | -0.05 |
| 95% confidence interval | | -0.35, -0.1 | -0.12,0.1 | -0.07, 0.2 | -0.16, 0.1 |
| p-value | | 0.004 | 0.888 | 0.364 | 0.413 |

| | | | | | |
|---|---|---|---|---|---|
| Abbreviations: ANCOVA = analysis of covariance; GSRS = Gastrointestinal Symptom Rating Scale; MITT = modified intent-to-treat; MMRM = mixed model for repeated measures; SD = standard deviation; TID = three times daily. a Model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

The average total GSRS is summarized by study week for the MITT Population and for the Per-Protocol Population. Based on the ANCOVA and the MMRM models for the MITT Population, the average total GSRS was statistically significantly smaller in the Larazotide acetate 0.5 mg group compared to the placebo group at Week 3, Week 4, Week 7, Week 8, Week 9, Week 10, and Week 11. Similar results were observed in the Per-Protocol Population.

### H. Average On-Treatment Score for Individual Domains of the Total GSRS:

The average on-treatment score is summarized for individual domains of the total GSRS in the MITT Population in Table 19. A statistically significantly lower mean score (ie, fewer symptoms) was observed in the Larazotide acetate 0.5 mg group compared to the placebo group for Indigestion syndrome (2.87 versus 3.18; difference from placebo -0.25), Constipation syndrome (2.45 versus 2.58; difference from placebo - 0.25), and Abdominal Pain syndrome (2.42 versus 2.58; difference from placebo -0.25).

**Table 19. Average On-Treatment Individual Domain Scores of Total GSRS - MITT Population**

| | | | | |
|---|---|---|---|---|
| | | **Larazotide Acetate TID** | | |

| **Domain Statistic** | **Placebo (N=84)** | **0.5 (N=85)** | **mg 1 (N=84)** | **mg 2 mg (N=87)** |
|---|---|---|---|---|
| **Diarrhea syndrome** | | | | |
| Baseline score, N | 84 | 85 | 84 | 87 |
| Mean (SD) | 3.20 (1.55) | 2.74 (1.27) | 3.12 (1.41) | 2.96 (1.52) |
| Average on-treatment, N | 80 | 81 | 81 | 86 |
| Mean (SD) | 2.77 (1.15) | 2.40 (1.05) | 2.61 (1.13) | 2.74 (1.21) |
| Difference from placebo | | | | |
| LS mean (95% CI)^{a} | | -0.19 (-0.48, 0.09) | -0.09 (-0.36, 0.18) | 0.11 (-0.15, 0.38) |
| p-value^{a} | | 0.185 | 0.504 | 0.393 |

| **Indigestion syndrome** | | | | |
|---|---|---|---|---|
| Baseline score, N | 84 | 85 | 84 | 87 |
| Mean (SD) | 3.57 (1.08) | 3.36 (1.08) | 3.57 (1.01) | 3.61 (0.99) |
| Average on-treatment, N | 80 | 81 | 81 | 86 |
| Mean (SD) | 3.18 (0.80) | 2.87 (0.81) | 3.18 (0.95) | 3.23 (0.88) |
| Difference from placebo | | | | |
| LS mean (95% CI)^{a} | | -0.25 (-0.47, -0.02) | -0.01 (-0.21, 0.20) | 0.02 (-0.18, 0.23) |
| p-value^{a} | | 0.029 | 0.957 | 0.813 |

| **Constipation syndrome** | | | | |
|---|---|---|---|---|
| Baseline score, N | 84 | 85 | 84 | 87 |
| Mean (SD) | 2.75 (1.37) | 2.80 (1.29) | 2.92 (1.32) | 3.02 (1.37) |
| Average on-treatment, N | 80 | 81 | 81 | 86 |
| Mean (SD) | 2.58 (1.09) | 2.45 (1.04) | 2.64 (1.14) | 2.61 (1.14) |
| Difference from placebo | | | | |
| LS mean (95% CI)^{a} | | -0.25 (-0.51, 0.00) | -0.02 (-0.26, 0.22) | -0.10 (-0.34, 0.13) |
| p-value^{a} | | 0.050 | 0.843 | 0.389 |

| **Abdominal Pain syndrome** | | | | |
|---|---|---|---|---|
| Baseline score, N | 84 | 85 | 84 | 87 |
| Mean (SD) | 2.91 (1.08) | 2.95 (1.04) | 2.83 (0.91) | 3.04 (1.01) |
| Average on-treatment, N | 80 | 81 | 81 | 86 |
| Mean (SD) | 2.58 (0.89) | 2.42 (0.77) | 2.62 (0.84) | 2.68 (0.94) |
| Difference from placebo | | | | |
| LS mean (95% CI)^{a} | | -0.25 (-0.47, 0.03) | - 0.07 (-0.14, 0.27) | 0.03 (-0.18, 0.23) |
| p-value" | | 0.024 | 0.519 | 0.796 |

| **Reflux syndrome** | | | | |
|---|---|---|---|---|
| Baseline score, N | 84 | 85 | 84 | 87 |
| Mean (SD) | 2.27 (1.44) | 2.22 (1.39) | 2.26 (1.37) | 2.40 (1.20) |
| Average on-treatment, N | 80 | 81 | 81 | 86 |
| Mean (SD) Difference from placebo | 2.00 (1.07) | 1.87 (0.92) | 2.08 (1.08) | 2.19 (1.07) |
| LS mean (95% CI)^{a} | | -0.14 (-0.40, 0.11) | 0.08 (-0.16, 0.32) | 0.12 (-0.11, 0.36) |
| p-value^{a} | | 0.273 | 0.513 | 0.307 |

| | | | | |
|---|---|---|---|---|
| Abbreviations: ANCOVA = analysis of covariance; CI = confidence interval; GSRS = Gastrointestinal Symptom Rating Scale; LS = least squares; MITT = modified intent-to-treat; SD = standard deviation; TID = three times daily. a ANCOVA model includes treatment, gender, baseline GSRS randomization strata, and randomization cohort as fixed effects and the baseline score as a covariate. | | | | |

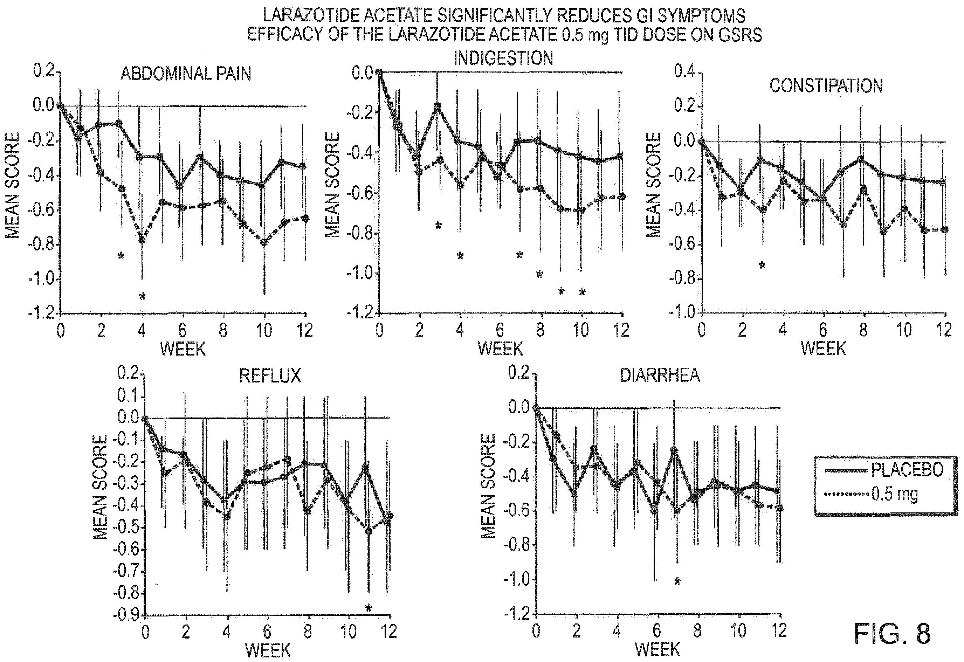

Change from baseline to each study week for the MITT and Per-Protocol Populations is summarized for Diarrhea syndrome, Indigestion syndrome, Constipation syndrome, Abdominal Pain syndrome, and Reflux Pain syndrome (Figure 8). Based on the ANCOVA and the MMRM statistical models for the MITT Population, the change from baseline to each study week for Indigestion syndrome was statistically significantly different in the Larazotide acetate 0.5 mg group compared to the placebo group at Week 3, Week 4, Week 7, Week 8, and Week 9 in favor of the Larazotide acetate 0.5 mg group; the difference from placebo was also statistically significant at Week 10 in the MMRM model in favor of the Larazotide acetate 0.5 mg group. Generally similar results were observed for Indigestion syndrome in the Per-Protocol Population. Isolated statistically significant differences from placebo were observed for the Larazotide acetate 0.5 mg group in the analyses of the other syndromes.

### I. Average On-Treatment Number of Weekly Bowel Movements

### (All BSFS Scores):

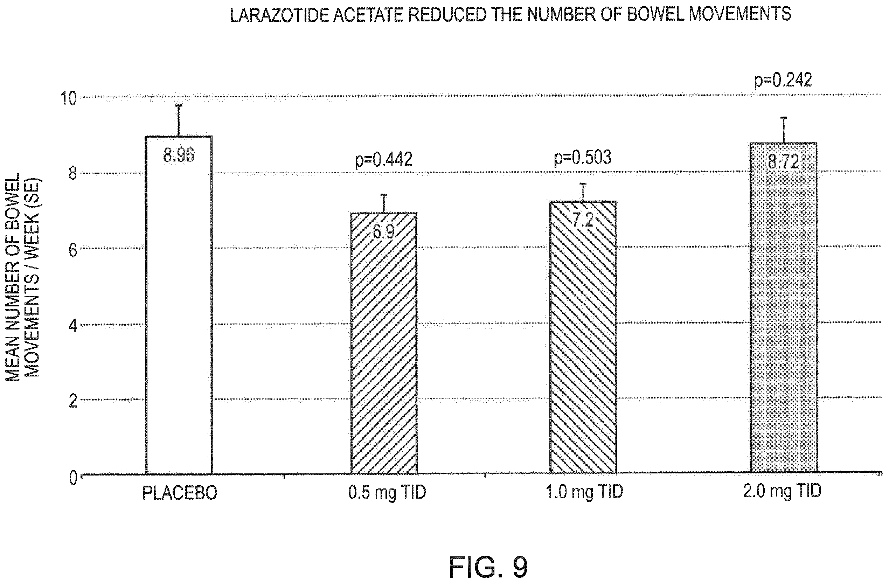

The average on-treatment number of weekly bowel movements as recorded in the BSFS daily diary is summarized for the MITT Population in Table 20 and Figure 9. The average number of weekly bowel movements was reduced in the Larazotide acetate 0.5 group (6.90) compared to the placebo group (8.96); however, this difference was not statistically significant. Results were similar in the Per-Protocol Population.

**Table 20. Average On-Treatment Weekly Number of Bowel Movements as Recorded in BSFS Daily Diary - MITT Population**

| | | **Larazotide Acetate TID** | | | |
|---|---|---|---|---|---|
| | **Placebo (N=84)** | **0.5 (N=85)** | **mg 1 (N=84)** | **mg 2 mg (N=87)** | **Pooled Doses (N=256)** |
| Baseline score | | | | | |
| N | 79 | 82 | 84 | 83 | 249 |
| Mean (SD) | 9.00 (7.11) | 7.59 (4.62) | 7.25 (4.78) | 7.95 (5.45) | 7.59 (4.95) |
| Average on-treatment score | | | | | |
| N | 78 | 81 | 84 | 82 | 247 |
| Mean (SD) | 8.96 (7.02) | 6.90 (4.19) | 7.20 (4.15) | 8.72 (5.94) | 7.61 (4.88) |

| | | **Difference from Placebo** | | | |
|---|---|---|---|---|---|
| ANCOVA results" | | | | | |
| Least squares mean | | -0.39 | -0.32 | 0.55 | -0.05 |
| 95% confidence interval | | -1.38, 0.60 | -1.25,0.61 | -0.38, 1.48 | -0.82, 0.72 |
| p-value | | 0.442 | 0.503 | 0.242 | 0.898 |

| | | | | | |
|---|---|---|---|---|---|
| Abbreviations: ANCOVA = analysis of covariance; BSFS = Bristol Stool Form Scale; GSRS = Gastrointestinal Symptom Rating Scale; MITT = modified intent-to-treat; SD = standard deviation; TID = three times daily. a Model includes treatment, gender, baseline GSRS randomization strata, and randomization cohort as fixed effects and the baseline score as a covariate. | | | | | |

Change in the average number of weekly bowel movements from baseline to each study week is summarized for the MITT Population and for the Per-Protocol Population. No statistically significant differences were observed between the Larazotide acetate 0.5 mg and placebo groups for the change from baseline in average number of weekly bowel movements to each study visit, although a trend in favor of the Larazotide acetate 0.5 mg dose was observed. The average number of weekly bowel movements is summarized over time for the MITT Population and for the Per-Protocol Population.

### J. Average On-Treatment Number of Weekly Bowel Movements With BSFS Scores of 5 to 7 (Diarrhea and Loose Stools):

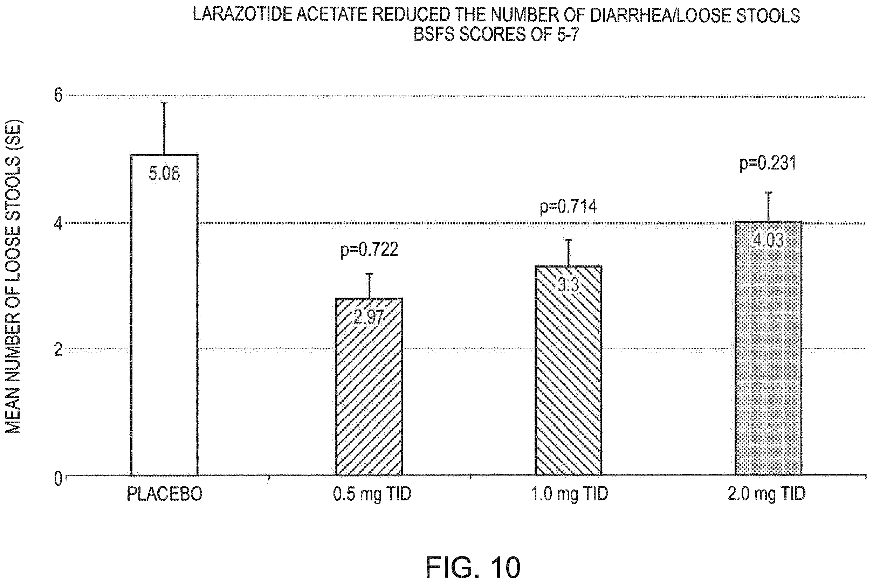

The average on-treatment number of weekly bowel movements with BSFS scores of 5 to 7 (diarrhea and loose stools) is summarized for the MITT Population in Table 21 and Figure 10. The average number of weekly bowel movements with BSFS scores of 5 to 7 was reduced in both the Larazotide acetate 0.5 mg (3.44 to 2.97) and placebo (5.46 to 5.06) groups; this difference was not statistically significant, although a trend in favor of the Larazotide acetate 0.5 mg dose was observed. Results were similar in the Per-Protocol Population.

**Table 21. Average On-Treatment Weekly Number of Bowel Movements With BSFS Scores of 5 to 7 (Diarrhea and Loose Stools) - MITT Population**

| | | **Larazotide Acetate TID** | | | |
|---|---|---|---|---|---|
| | **Placebo (N=84)** | **0.5 (N=85)** | **mg 1 (N=84)** | **mg 2 (N=87)** | **mg Pooled Doses (N=256)** |
| Baseline score | | | | | |
| N | 79 | 82 | 84 | 83 | 249 |
| Mean (SD) | 5.46 (7.43) | 3.44 (4.35) | 3.50 (4.38) | 3.53 (4.22) | 3.49 (4.30) |
| Average on-treatment score | | | | | |
| N | 78 | 81 | 84 | 82 | 247 |
| Mean (SD) | 5.06 (7.51) | 2.97 (3.50) | 3.30 (3.93) | 4.03 (4.08) | 3.43 (3.85) |

| | | **Difference from Placebo** | | | |
|---|---|---|---|---|---|
| ANCOVA results^{a} | | | | | |
| Least squares mean | | -0.16 | -0.15 | 0.51 | 0.06 |
| 95% confidence interval | | -1.0, 0.7 | -1.0, 0.7 | -0.3,1.3 | -0.6, 0.8 |
| p-value | | 0.722 | 0.714 | 0.231 | 0.855 |

| | | | | | |
|---|---|---|---|---|---|
| Abbreviations: ANCOVA = analysis of covariance; BSFS = Bristol Stool Form Scale; GSRS = Gastrointestinal Symptom Rating Scale; MITT = modified intent-to-treat; SD = standard deviation; TID = three times daily. a Model includes treatment, gender, baseline GSRS randomization strata, and randomization cohort as fixed effects and the baseline score as a covariate. | | | | | |

### K. Percentage of Subjects With Pre-Specified Reductions From Baseline in Weekly Average CeD PRO Abdominal Domain Scores:

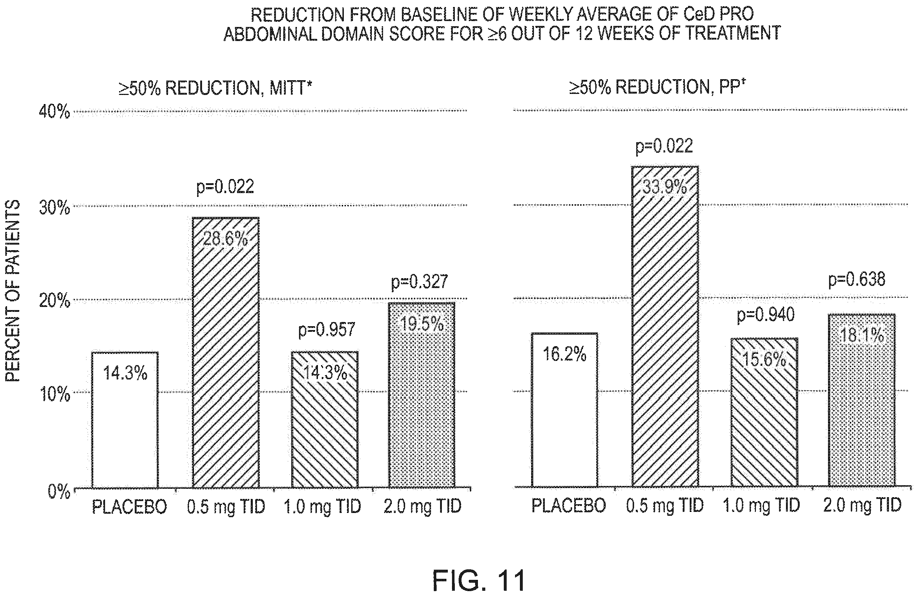

The percentage of subjects with pre-specified reductions from baseline in on-treatment weekly average of CeD PRO Abdominal Domain score for ≥ 6 of 12 weeks of treatment is summarized for the MITT Population in Table 22 and Figure 11. A statistically significantly larger percentage of subjects in the Larazotide acetate 0.5 mg group (28.6%) than the placebo group (14.3%) experienced reductions of ≥ 50%. Similar results were observed in the Per-Protocol Population (Larazotide acetate 0.5 mg 33.9%, placebo 16.2%, p=0.022; Figure 11).

**Table 22. Subjects With Pre-Specified Reductions From Baseline in On-Treatment Weekly Average of CeD PRO Abdominal Domain for ≥ 6 of 12 Weeks of Treatment - MITT Population**

| **Percent Reduction from Baseline** | **Placebo (N=84)** | **Larazotide Acetate TID** | | |
|---|---|---|---|---|
| | | **0.5 (N=85)** | **mg 1 (N=84)** | **mg 2 mg (N=87)** |
| Number of subjects | 84 | 84 | 84 | 87 |
| ≥ 10% | | | | |
| n (%) of subjects | 48 (57.1) | 46 (54.8) | 47 (56.0) | 51 (58.6) |
| p-value versus placebo^{a} | | 0.566 | 0.887 | 0.884 |
| ≥ 20% | | | | |
| n (%) of subjects | 38 (45.2) | 39 (46.4) | 37 (44.0) | 41 (47.1) |
| p-value versus placebo^{a} | | 0.536 | 0.898 | 0.843 |
| ≥ 30% | | | | |
| n (%) of subjects | 28 (33.3) | 35 (41.7) | 30 (35.7) | 32 (36.8) |
| p-value versus placebo^{a} | | 0.106 | 0.702 | 0.654 |
| ≥ 50% | | | | |
| n (%) of subjects | 12 (14.3) | 24 (28.6) | 12 (14.3) | 17 (19.5) |
| p-value versus placebo^{a} | | 0.022 | 0.957 | 0.327 |

| | | | | |
|---|---|---|---|---|
| Abbreviations: CeD = celiac disease; GSRS = Gastrointestinal Symptom Rating Scale; MITT = modified intent-to-treat; PRO = patient reported outcomes; TID = three times daily. a Cochran-Mantel-Haenszel test stratified by gender, baseline GSRS randomization strata, and randomization cohort. | | | | |

### L. Percentage of Subjects With Pre-Specified Reductions From Baseline in Weekly Average CeD PRO Gastrointestinal Domain Scores:

The percentage of subjects with pre-specified reductions from baseline in on-treatment weekly average of CeD PRO Gastrointestinal Domain score for ≥ 6 of 12 weeks of treatment is summarized for the MITT Population in Table 23. A statistically significantly larger percentage of subjects in the Larazotide acetate 0.5 mg group than the placebo group experienced reductions of ≥ 50% (34.5% versus 16.7%). Similar results were observed in the Per-Protocol Population.

**Table 23. Subjects With Pre-Specified Reductions From Baseline in On-Treatment Weekly Average of CeD PRO Gastrointestinal Domain for ≥ 6 of 12 Weeks of Treatment - MITT Population**

| | | **Larazotide Acetate TID** | | |
|---|---|---|---|---|
| **Percent Reduction from Baseline** | **Placebo (N=84)** | **0.5 (N=85)** | **mg 1 (N=84)** | **mg 2 mg (N=87)** |
| Number of subjects | 84 | 84 | 84 | 87 |
| ≥ 10% | | | | |
| n (%) of subjects | 45 (53.6) | 45 (53.6) | 47 (56.0) | 51 (58.6) |
| p-value versus placebo^{a} | | 0.703 | 0.764 | 0.506 |
| ≥ 20% | | | | |
| n (%) of subjects | 36 (42.9) | 41 (48.8) | 38 (45.2) | 43 (49.4) |
| p-value versus placebo^{a} | | 0.354 | 0.739 | 0.390 |
| ≥ 30% | | | | |
| n (%) of subjects | 31 (36.9) | 37 (44.0) | 30 (35.7) | 26 (29.9) |
| p-value versus placebo^{a} | | 0.133 | 0.920 | 0.354 |
| ≥ 50% | | | | |
| n (%) of subjects | 14 (16.7) | 29(34.5) | 15 (17.9) | 15 (17.2) |
| p-value versus placebo^{a} | | 0.002 | 0.828 | 0.865 |

| | | | | |
|---|---|---|---|---|
| Abbreviations: CeD = celiac disease; GSRS = Gastrointestinal Symptom Rating Scale; MITT = modified intent-to-treat; PRO = patient reported outcome; TID = three times daily. a Cochran-Mantel-Haenszel test stratified by gender, baseline GSRS randomization strata, and randomization cohort. | | | | |

### M. Percentage of Subjects With Pre-Specified Reductions From Baseline in Weekly Average CeD GSRS Scores:

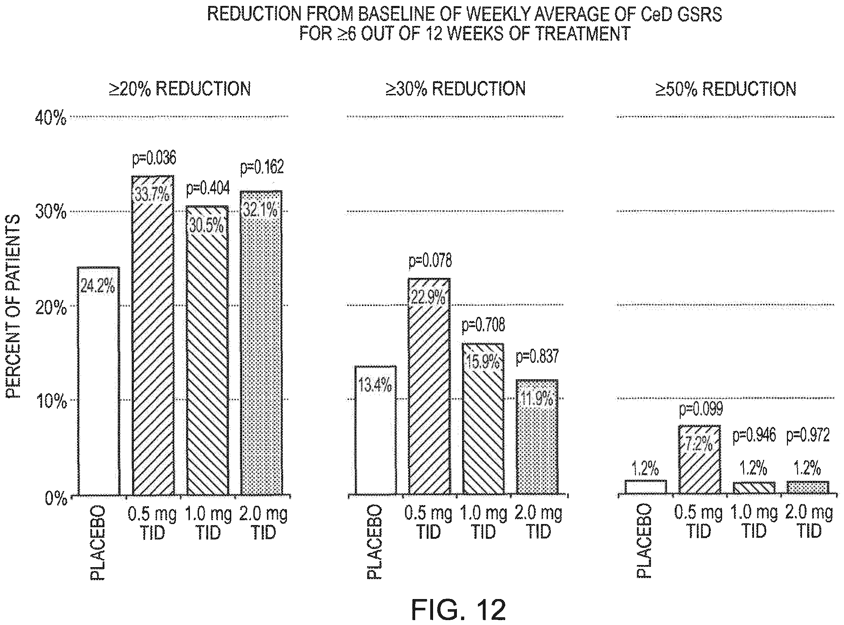

The percentage of subjects with pre-specified reductions from baseline in on-treatment weekly average of CeD GSRS Score for ≥ 6 of 12 weeks of treatment is summarized for the MITT Population in Table 24 and Figure 12. A statistically significantly larger percentage of subjects in the Larazotide acetate 0.5 mg group (33.7%) than the placebo group (24.4%) experienced reductions of ≥ 20%. Results were similar in the Per-Protocol Population.

**Table 24. Subjects With Pre-Specified Reductions From Baseline in On-Treatment Weekly Average of CeD GSRS for ≥ 6 of 12 Weeks of Treatment - MITT Population**

| **Percent Reduction from Baseline** | **Placebo** | **Larazotide Acetate TID** | | | |
|---|---|---|---|---|---|
| | | **0.5 (N=85)** | **mg 1 (N=84)** | **mg 2 (N=87)** | **mg (N=84)** |
| Number of subjects | 82 | 83 | 82 | 84 | |
| ≥ 10% | | | | | |
| n (%) of subjects | 38 (46.3) | 41 (49.4) | 30 (36.6) | 36 (42.9) | |
| p-value versus placebo^{a} | | 0.219 | 0.186 | 0.716 | |
| ≥ 20% | | | | | |
| n (%) of subjects | 20 (24.4) | 28 (33.7) | 25 (30.5) | 27 (32.1) | |
| p-value versus placebo^{a} | | 0.036 | 0.404 | 0.162 | |
| ≥ 30% | | | | | |
| n (%) of subjects | 11 (13.4) | 19 (22.9) | 13 (15.9) | 10 (11.9) | |
| p-value versus placebo^{a} | | 0.078 | 0.708 | 0.837 | |
| ≥ 50% | | | | | |
| n (%) of subjects | 1 (1.2) | 6 (7.2) | 1 (1.2) | 1 (1.2) | |
| p-value versus placebo^{a} | | 0.099 | 0.946 | 0.972 | |

| | | | | | |
|---|---|---|---|---|---|
| Abbreviations: CeD GSRS = celiac disease domain of the Gastrointestinal Symptom Rating Scale; MITT = modified intent-to-treat; TID = three times daily. a Cochran-Mantel-Haenszel test stratified by gender, baseline GSRS randomization strata, and randomization cohort. | | | | | |

### N. Percentage of Subjects With Pre-Specified Reductions From Baseline in Weekly Number of Bowel Movements With Diarrhea or Loose Stools:

The percentage of subjects with pre-specified reductions from baseline in weekly number of bowel movements with BFSF score of 5 to 7 (diarrhea and loose stools) for ≥ 6 of 12 weeks of treatment is summarized for the MITT Population in Table 25. No statistically significant differences from placebo were observed between the Larazotide acetate 0.5 mg and placebo groups. Similar results were observed in the Per-Protocol Population.

**Table 25. Subjects With Pre-Specified Reductions From Baseline in Weekly Number of Bowel Movements With BSFS Scores of 5 to 7 (Diarrhea and Loose Stools) - MITT Population**

| | | **Larazotide Acetate TID** | | |
|---|---|---|---|---|
| **Percent from Baseline** | **Reduction Placebo (N=84)** | **0.5 (N=85)** | **mg 1 (N=84)** | **mg 2 (N=87) mg** |
| Number of subjects | 79 | 81 | 84 | 82 |
| ≥ 10% | | | | |
| n (%) of subjects | 33 (41.8) | 27 (33.3) | 31 (36.9) | 30 (36.6) |
| p-value versus placebo^{a} | | 0.209 | 0.582 | 0.670 |
| ≥ 20% | | | | |
| n (%) of subjects | 30 (38.0) | 25 (30.9) | 29 (34.5) | 25 (30.5) |
| p-value versus placebo^{a} | | 0.215 | 0.708 | 0.411 |
| ≥ 30% | | | | |
| n (%) of subjects | 29 (36.7) | 21 (25.9) | 27 (32.1) | 19 (23.2) |
| p-value versus placebo^{a} | | 0.078 | 0.619 | 0.094 |
| ≥ 50% | | | | |
| n (%) of subjects | 26 (32.9) | 18 (22.2) | 18 (21.4) | 17 (20.7) |
| p-value versus placebo^{a} | | 0.162 | 0.107 | 0.113 |

| | | | | |
|---|---|---|---|---|
| Abbreviations: BSFS = Bristol Stool Form Scale; GSRS = Gastrointestinal Symptom Rating Scale; MITT = modified intent-to-treat; TID = three times daily. a Cochran-Mantel-Haenszel test stratified by gender, baseline GSRS randomization strata, and randomization cohort. | | | | |

### O. Average On-Treatment Weekly Number of CeD PRO Improved Symptom Days:

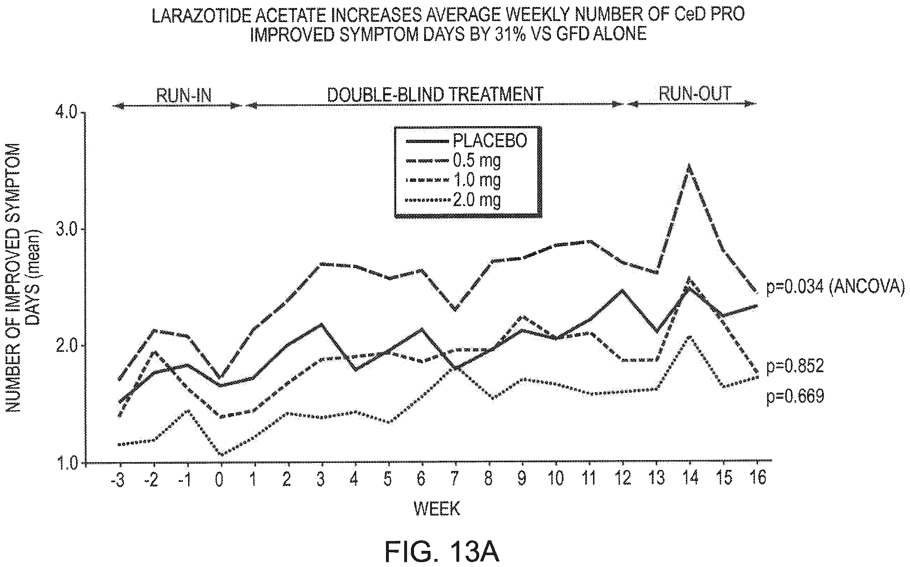

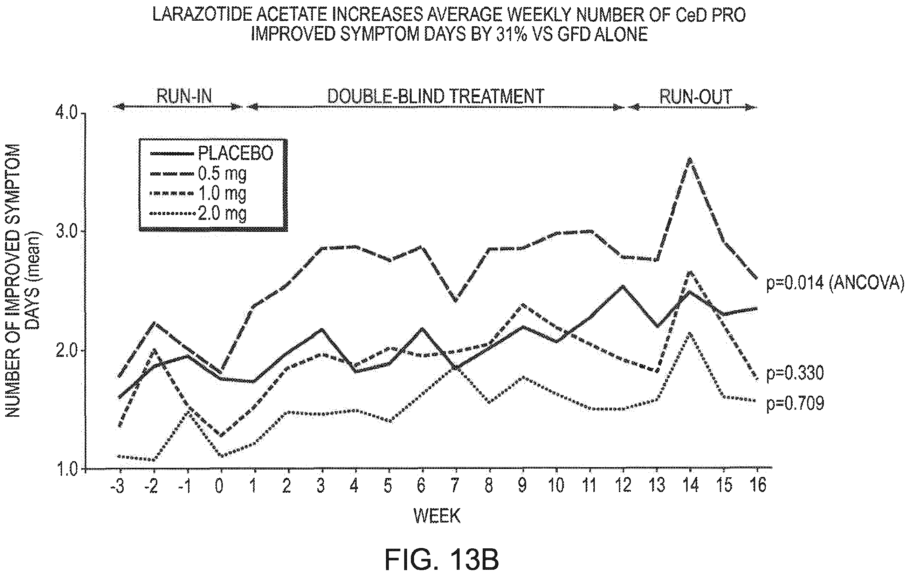

The average on-treatment number of CeD PRO Improved Symptom days is summarized for the MITT Population in Table 26. A statistically significant increase in the average on-treatment number of CeD PRO Improved Symptom days was observed in the Larazotide acetate 0.5 mg group (1.70 to 2.51 days) compared to the placebo group (1.65 to 1.99 days). A 31% increase in CeD PRO Improved Symptom days was observed with the Larazotide acetate 0.5 mg dose versus GFD alone (there were 0.49 days/week increased CeD PRO Improved Symptom days or 5.88 more CeD PRO Improved Symptom days during the 12-week treatment period. Figures 13A and 13B show that Larazotide acetate increased average weekly number of CeD PRO Improved Symptom days compared to gluten-free diet alone.

**Table 26. Average On-Treatment Weekly Number of CeD PRO Improved Symptom Days - MITT Population**

| | | **Larazotide Acetate TID** | | | |
|---|---|---|---|---|---|
| | **Placebo (N=84)** | **0.5 (N=85)** | **mg 1** (N=84) | **mg 2 (N=87)** | **mg Pooled Doses (N=256)** |
| Baseline score | | | | | |
| N | 84 | 84 | 84 | 87 | 255 |
| Mean (SD) | 1.65 (1.81) | 1.70 (1.66) | 1.38 (1.73) | 1.05 (1.44) | 1.37 (1.63) |
| Average on-treatment score | | | | | |
| N | 81 | 84 | 84 | 87 | 255 |
| Mean (SD) | 1.99 (1.98) | 2.51 (1.88) | 1.80 (1.90) | 1.46 (1.59) | 1.92 (1.84) |

| | | **Difference from Placebo** | | | |
|---|---|---|---|---|---|
| ANCOVA results^{a} | | | | | |
| Least squares mean | | 0.49 | 0.04 | -0.09 | 0.15 |
| 95% confidence interval | | 0.04, 0.94 | -0.38, 0.46 | -0.52, 0.33 | -0.21, 0.50 |
| p-value | | 0.034 | 0.852 | 0.669 | 0.416 |

| | | | | | |
|---|---|---|---|---|---|
| Abbreviations: ANCOVA = analysis of covariance; GSRS = Gastrointestinal Symptom Rating Scale; MITT = modified intent-to-treat; SD = standard deviation; TID = three times daily. a Model includes treatment, gender, baseline GSRS randomization strata, and randomization cohort as fixed effects and the baseline score as a covariate. | | | | | |

The number of CeD PRO Improved Symptom days is summarized by study week for the MITT Population and for the Per-Protocol Population. Based on the ANCOVA and MMRM models for the MITT Population, statistically significant increases in the number of CeD PRO Improved Symptom days were observed in the Larazotide acetate 0.5 mg group compared to the placebo group at Week 4, Week 10, and Week 14; statistically significant increases were also observed for the MMRM model at Week 8 and Week 11. In the Per-Protocol Population, statistically significant increases were observed based on the ANCOVA and MMRM models at Week 4, Week 5, Week 8, Week 10, Week 11, and Week 14; statistically significant increases were also observed for the ANCOVA model at Week 3 and for the MMRM model at Week 6.

### P. Average On-Treatment Weekly Number of CeD PRO Symptomatic Days:

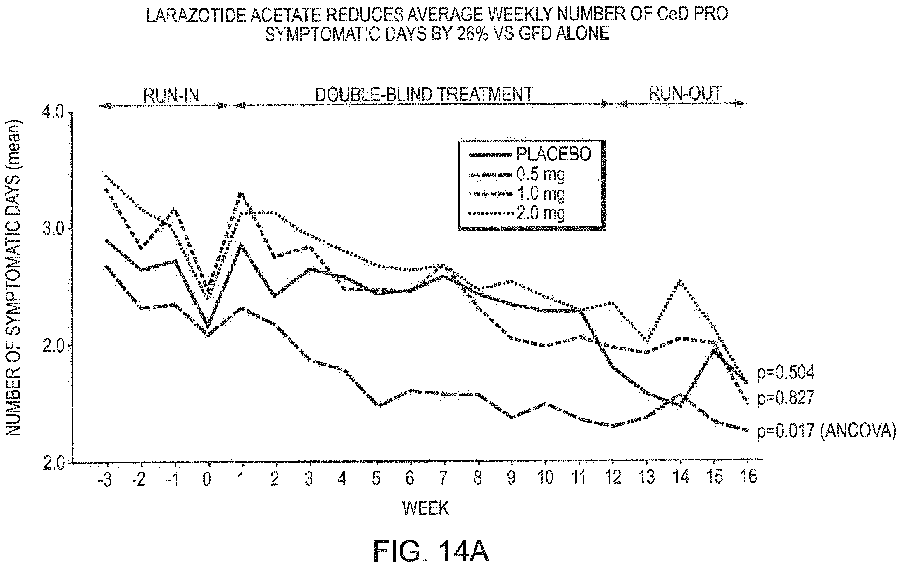

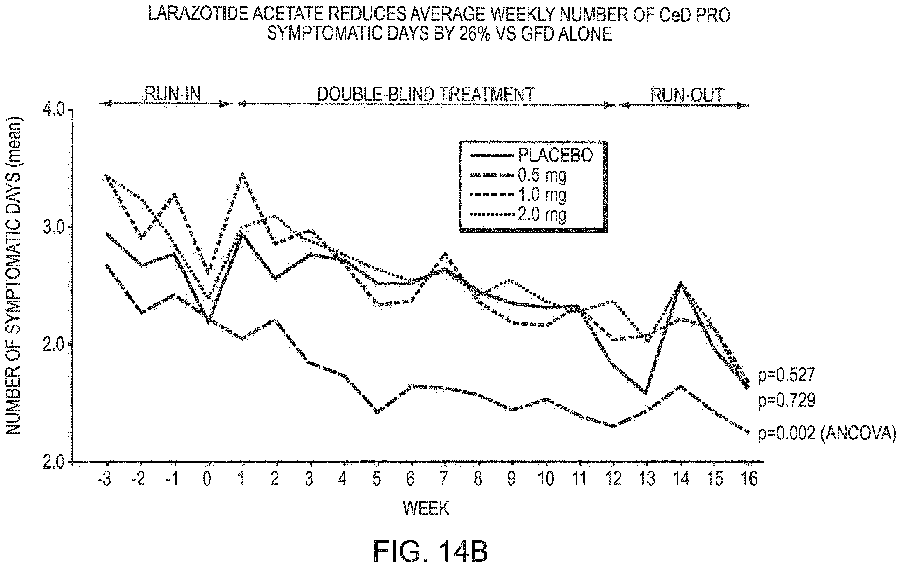

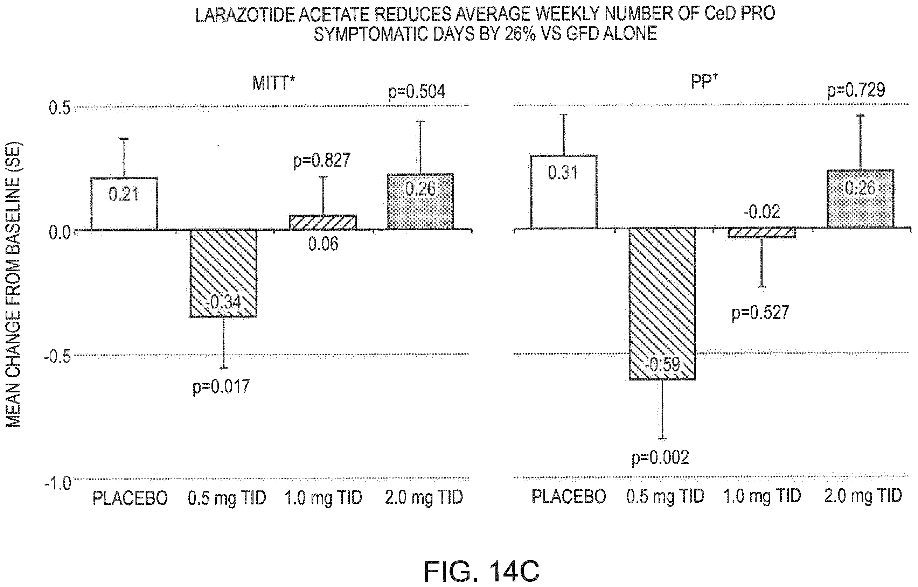

The average on-treatment weekly number of CeD PRO Symptomatic days is summarized for the MITT Population in Table 27. The average on-treatment weekly number of CeD PRO Symptomatic days in the Larazotide acetate 0.5 mg group was reduced (2.07 to 1.73 days), but was increased in the placebo group (2.14 to 2.38 days); the difference between the groups was statistically significant. Similar results were observed in the Per-Protocol Population. Figures 14A, 14B, and 14C show that Larazotide acetate reduced average weekly number of CeD PRO Symptomatic days compared to gluten-free diet alone.

**Table 27. Average On-Treatment Weekly Number of CeD PRO Symptomatic Days - MITT Population**

| | | **Larazotide Acetate TID** | | | |
|---|---|---|---|---|---|
| | **Placebo** | **0.5** | **mg 1** | **mg 2** | **mg Pooled Doses** |
| | **(N=84)** | **(N=85)** | **(N=84)** | **(N=87)** | **(N=256)** |
| Baseline score | | | | | |
| N | 84 | 84 | 84 | 87 | 255 |
| Mean (SD) Average on-treatment score | 2.14 (2.07) | 2.07 (2.09) | 2.43 (2.05) | 2.37 (2.02) | 2.29 (2.05) |
| N | 81 | 84 | 84 | 87 | 255 |
| Mean (SD) | 2.38 (2.01) | 1.73 (1.69) | 2.48 (2.00) | 2.63 (1.82) | 2.29 (1.88) |

| | | **Difference from Placebo** | | | |
|---|---|---|---|---|---|
| ANCOVA results^{a} | | | | | |
| Least squares mean | | -0.56 | -0.05 | 0.15 | -0.15 |
| 95% confidence interval | | -1.02, -0.10 | -0.48, 0.38 | -0.28, 0.57 | -0.51, 0.20 |
| p-value | | 0.017 | 0.827 | 0.504 | 0.396 |

| | | | | | |
|---|---|---|---|---|---|
| Abbreviations: ANCOVA = analysis of covariance; GSRS = Gastrointestinal Symptom Rating Scale; MITT = modified intent-to-treat; SD = standard deviation; TID = three times daily. a Model includes treatment, gender, baseline GSRS randomization strata, and randomization cohort as fixed effects and the baseline score as a covariate. | | | | | |

The number of CeD PRO Symptomatic days is summarized by study week for the MITT Population and for the Per-Protocol Population. A 26% reduction in the CeD PRO Symptomatic days was observed with the Larazotide acetate 0.5 mg dose versus the GFD alone (there were 0.56 days/week or 6.72 fewer CeD PRO Symptomatic Days during the 12-week treatment period. Based on the ANCOVA and MMRM models for the MITT Population, statistically significant reductions in the number of CeD PRO Symptomatic days were observed in the Larazotide acetate 0.5 mg group compared to the placebo group at Week 3 through Week 9 and Week 11; statistically significant reductions were also noted for Week 10 and Week 14 in the MMRM model. Similar results were observed in the Per-Protocol Population.

### Q. Average On-Treatment Score of CeD PRO Non-Gastrointestinal Domain (Headache and Tiredness):

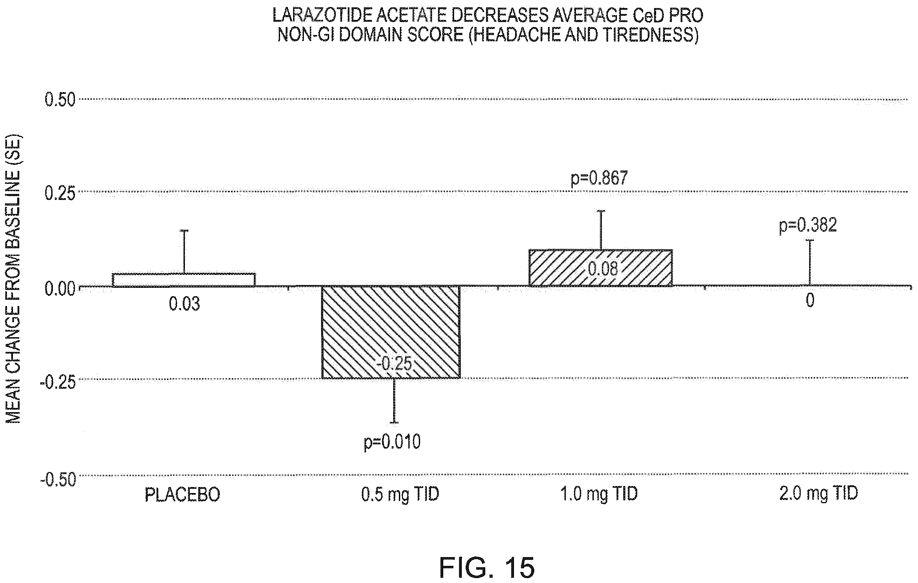

The average on-treatment score of the CeD PRO Non-Gastrointestinal Domain is summarized for the MITT Population in Table 28. Based on the ANCOVA for the MITT Population, no difference was observed between the Larazotide acetate 0.5 mg and placebo groups for the average on-treatment score of the CeD PRO Non-Gastrointestinal Domain. A statistically significantly greater mean change from baseline in the average on-treatment score of the CeD PRO Non-Gastrointestinal Domain was observed in the Larazotide acetate 0.5 mg group (2.69 to 2.44) compared to the placebo group (2.73 to 2.75) based on the MMRM model (-0.31). Figure 15 shows that Larazotide acetate decreased average CeD PRO Non-Gastrointestinal Domain score.

**Table 28. Average On-Treatment CeD PRO Non-Gastrointestinal Domain Score - MITT Population**

| | | **Larazotide Acetate TID** | | | |
|---|---|---|---|---|---|
| | **Placebo** | **0.5** | **mg 1** | **mg 2** | **mg Pooled Doses** |
| | **(N=84)** | **(N=85)** | **(N=84)** | **(N=87)** | **(N=256)** |
| Baseline score | | | | | |
| N | 84 | 84 | 84 | 87 | 255 |
| Mean (SD) | 2.73 (1.76) | 2.69 (1.62) | 2.64 (1.60) | 3.11 (1.82) | 2.82 (1.69) |

| Average on-treatment score | | | | | |
|---|---|---|---|---|---|
| N | 81 | 84 | 84 | 87 | 255 |
| Mean (SD) | 2.75 (1.55) | 2.44 (1.43) | 2.73 (1.36) | 3.11 (1.81) | 2.76 (1.57) |

| | | **Difference from Placebo** | | | |
|---|---|---|---|---|---|
| ANCOVA results^{a} | | | | | |
| Least squares mean | | -0.25 | 0.02 | 0.09 | -0.05 |
| 95% confidence interval | | -0.6, 0.1 | -0.3, 0.3 | -0.2, 0.4 | -0.3, 0.2 |
| p-value | | 0.123 | 0.879 | 0.555 | 0.714 |
| MMRM results^{b} | | | | | |
| Least squares mean | | -0.31 | 0.02 | 0.10 | -0.06 |
| 95% confidence interval | | -0.5, -0.1 | -0.2, 0.2 | -0.1,0.3 | -0.2, 0.1 |
| p-value | | 0.010 | 0.867 | 0.382 | 0.481 |

| | | | | | |
|---|---|---|---|---|---|
| Abbreviations: ANCOVA = analysis of covariance; CeD = celiac disease; GSRS = Gastrointestinal Symptom Rating Scale; MITT = modified intent-to-treat; MMRM = mixed model for repeated measures; PRO = patient reported outcome; SD = standard deviation; TID = three times daily. a Model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

### R. Average On-Treatment Score of CeD PRO Nausea, Constipation, and Vomiting Domains:

The average on-treatment score is summarized for individual domains of the CeD PRO in the MITT Population in Table 29. No statistically significant difference was observed between the Larazotide acetate 0.5 mg and placebo groups for the average on-treatment score of the CeD PRO Nausea, Constipation, or Vomiting Domains.

**Table 29. Average On-Treatment CeD PRO Nausea, Constipation, and Vomiting Domain Scores - MITT Population**

| | | **Larazotide Acetate TID** | | | |
|---|---|---|---|---|---|
| **Domain** Statistic | **Placebo (N=84)** | **0.5 (N-85)** | **mg 1 (N=84)** | **mg 2 (N=87)** | **mg Pooled Doses (N=256)** |
| **Nausea** | | | | | |
| Baseline score, N | 84 | 84 | 84 | 87 | 255 |
| Mean (SD) | 1.26 (2.07) | 0.91 (1.28) | 0.95 (1.55) | 1.01 (1.75) | 0.96 (1.54) |
| Avg on-treatment, N | 81 | 84 | 84 | 87 | 255 |
| Mean (SD) Difference vs. placebo ANCOVA^{a} | 0.97 (1.19) | 0.83 (0.99) | 1.14 (1.44) | 1.20 (1.83) | 1.06 (1.47) |
| | | -0.05 | (-0.37, 0.29 (-0.01, 0.59) | 0.32 (0.03, 0.62) | 0.19 (-0.06, 0.43) |
| LS mean (95% CI) | | 0.27) | | | |
| p-value MMRM^{b} | | 0.743 | 0.059 | 0.032 | 0.139 |
| | | -0.05 | (-0.28, 0.25 (0.04, 0.47) | 0.31 (0.10, 0.52) | 0.17 (-0.00, 0.35) |
| LS mean (95% CI) | | 0.18) | | | |
| p-value | | 0.664 | 0.019 | 0.004 | 0.055 |

| Constipation | | | | | |
|---|---|---|---|---|---|
| Baseline score, N | 84 | 84 | 84 | 87 | 255 |
| Mean (SD) | 1.60 (2.23) | 1.47 (1.87) | 1.69 (2.18) | 1.66 (2.09) | 1.61 (2.05) |
| Avg on-treatment, N | 81 | 84 | 84 | 87 | 255 |
| Mean (SD) Difference vs. placebo ANCOVA^{a} | 1.39 (1.76) | 1.49 (1.66) | 1.60 (1.79) | 1.62 (1.66) | 1.57 (1.70) |
| LS mean (95% CI) | | 0.02 (-0.4,0.4) | 0.13 (-0.3, 0.5) | 0.19 (-0.2, 0.6) | 0.11 (-0.2, 0.4) |
| p-value MMRM^{b} | | 0.929 | 0.501 | 0.331 | 0.485 |
| LS mean (95% CI) | | -0.01 (-0.3, 0.3) | 0.10 (-0.2, 0.4) | 0.17 (-0.1, 0.4) | 0.09 (-0.1, 0.3) |
| p-value | | 0.971 | 0.499 | 0.229 | 0.460 |

| Vomiting | | | | | |
|---|---|---|---|---|---|
| Baseline score, N | 84 | 84 | 84 | 87 | 255 |
| Mean (SD) | 0.09 (0.37) | 0.00 (0.03) | 0.03 (0.12) | 0.05 (0.27) | 0.03 (0.18) |
| Avg on-treatment, N | 81 | 84 | 84 | 87 | 255 |
| Mean (SD) Difference vs. placebo ANCOVA^{a} | 0.10 (0.35) | 0.02 (0.05) | 0.06 (0.14) | 0.12 (0.40) | 0.07 (0.25) |
| LS mean (95% CI) | | -0.05 (-0.1, 0.0) | -0.04 (-0.1, 0.0) | 0.03 (-0.1, 0.1) | -0.02 (-0.1, 0.1) |
| p-value MMRM^{b} | | 0.265 | 0.409 | 0.512 | 0.581 |
| LS mean (95% CI) | | -0.05 (-0.1, 0.0) | -0.05 (-0.1, 0.0) | 0.03 (-0.0, 0.1) | -0.02 (-0.1, 0.0) |
| p-value | | 0.106 | 0.110 | 0.329 | 0.338 |

| | | | | | |
|---|---|---|---|---|---|
| Abbreviations: ANCOVA = analysis of covariance; Avg = average; CI = confidence interval; GSRS = Gastrointestinal Symptom Rating Scale; LS = least squares; MITT = modified intent-to-treat; MMRM = mixed model for repeated measures; SD = standard deviation; TID = three times daily. a ANCOVA model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

### S. Clinician Global Assessment of Disease Activity:

The CGA of disease activity at end of treatment is summarized for the MITT Population in Table 30. No statistically significant difference was observed between the Larazotide acetate 0.5 mg and placebo groups.

**Table 30. Clinician Global Assessment of Disease Activity at End of Treatment - MITT Population**

| | | **Larazotide Acetate TID** | | | |
|---|---|---|---|---|---|
| **Percent Reduction** | **Placebo** | **0.5** | **mg 1** | **mg 2** | **mg** |
| **from Baseline** | **(N=84)** | **(N=85)** | **(N=84)** | **(N=87)** | |
| Assessment 1 | | | | | |
| Number of subjects | 72 | 73 | 74 | 76 | |
| 1) Complete remission, n (%) | 5 (6.9) | 4 (5.5) | 4 (5.4) | 2 (2.6) | |
| 2) Remission/mild disease n (%) | 14 (19.4) | 17 (23.3) | 11 (14.9) | 19 (25.0) | |
| 3) Mild disease n (%) | 27 (37.5) | 37 (50.7) | 37 (50.0) | 27 (35.5) | |
| 4) Mild/moderate disease n (%) | 13 (18.1) | 8 (11.0) | 7 (9.5) | 12 (15.8) | |
| 5) Moderate disease n (%) | 11 (15.3) | 5 (6.8) | 12 (16.2) | 13 (17.1) | |
| 6) Moderate/severe disease n (%) | 2 (2.8) | 2 (2.7) | 2 (2.7) | 2 (2.6) | |
| 7) Severe disease n (%) | 0 | 0 | 1 (1.4) | 1 (1.3) | |
| Mean (SD) | 3.24 (1.22) | 2.99 (1.05) | 3.30 (1.22) | 3.33 (1.25) | |
| p-value versus placebo^{a} | | 0.742 | 0.805 | 0.141 | |
| Assessment 2 | | | | | |
| Number of subjects | 70 | 72 | 73 | 74 | |
| 1) Much improved, n (%) | 20 (28.6) | 14 (19.4) | 18 (24.7) | 19 (25.7) | |
| 2) Minimally improved, n (%) | 28 (40.0) | 36 (50.0) | 31 (42.5) | 27 (36.5) | |
| 3) No change, n (%) | 19 (27.1) | 19 (26.4) | 19 (26.0) | 24 (32.4) | |
| 4) Minimally worse, n (%) | 2 (2.9) | 3 (4.2) | 4 (5.5) | 4 (5.4) | |
| 5) Much worse, n (%) | 1 (1.4) | 0 | 1 (1.4) | 0 | |
| Mean (SD) | 2.09 (0.90) | 2.15 (0.78) | 2.16 (0.91) | 2.18 (0.88) | |
| p-value versus placebo^{a} | | 0.498 | 0.776 | 0.530 | |

| | | | | | |
|---|---|---|---|---|---|
| Abbreviations: GSRS = Gastrointestinal Symptom Rating Scale; MITT = modified intent-to-treat; SD = standard deviation; TID = three times daily. a Cochran-Mantel-Haenszel test stratified by gender, baseline GSRS randomization strata, and randomization cohort. | | | | | |

### T. Average On-Treatment General Well-Being Question:

The average on-treatment General Well-Being Question score is summarized for the MITT Population in Table 31. No statistically significant difference was observed between the Larazotide acetate 0.5 mg and placebo groups for the average on-treatment General Well-Being Question score in the MITT Population. However, based on the MMRM in the Per-Protocol Population, a statistically significantly higher mean score (*i.e.,* better overall well-being) was observed in the Larazotide acetate 0.5 mg group compared to the placebo group (6.63 versus 6.22; difference from placebo 0.32, p=0.011).

**Table 31. Average On-Treatment General Well-Being Question Score - MITT Population**

| | | **Larazotide Acetate TID** | | | |
|---|---|---|---|---|---|
| | **Placebo (N=84)** | **0.5 (N=85)** | **mg 1 (N=84)** | **mg 2 (N=87)** | **mg Pooled Doses (N=256)** |
| Baseline score | | | | | |
| N | 84 | 85 | 84 | 86 | 255 |
| Mean (SD) | 5.95 (1.76) | 6.24 (1.49) | 6.09 (1.55) | 5.91 (1.67) | 6.08 (1.57) |

| Average on-treatment score | | | | | |
|---|---|---|---|---|---|
| N | 81 | 84 | 84 | 87 | 255 |
| Mean (SD) | 6.24 (1.26) | 6.49 (1.48) | 6.02 (1.40) | 5.93 (1.60) | 6.15 (1.51) |

| | | **Difference from Placebo** | | | |
|---|---|---|---|---|---|
| ANCOVA results^{a} | | | | | |
| Least squares mean | | 0.11 | -0.28 | -0.25 | -0.14 |
| 95% confidence interval | | -0.2, 0.4 | -0.6, 0.0 | -0.5, 0.0 | -0.4, 0.1 |
| p-value | | 0.492 | 0.064 | 0.096 | 0.263 |

| MMRM results^{b} | | | | | |
|---|---|---|---|---|---|
| Least squares mean | | 0.17 | -0.27 | -0.23 | -0.11 |
| 95% confidence interval | -0.0, 0.4 | -0.5, -0.1 | -0.4, -0.0 | -0.3, 0.1 | |
| p-value | 0.125 | 0.011 | 0.027 | 0.210 | |

| | | | | | |
|---|---|---|---|---|---|
| Abbreviations: ANCOVA = analysis of covariance; GSRS = Gastrointestinal Symptom Rating Scale; MITT = modified intent-to-treat; MMRM = mixed model for repeated measures; SD = standard deviation; TID = three times daily. a Model includes treatment, gender, baseline GSRS randomization strata, and randomization cohort as fixed effects and the baseline score as a covariate. b Model includes treatment and study week as main effects, gender, baseline GSRS randomization strata, and randomization cohort, and the baseline score as a covariates, and patient reported outcome over study weeks as repeated measures. | | | | | |

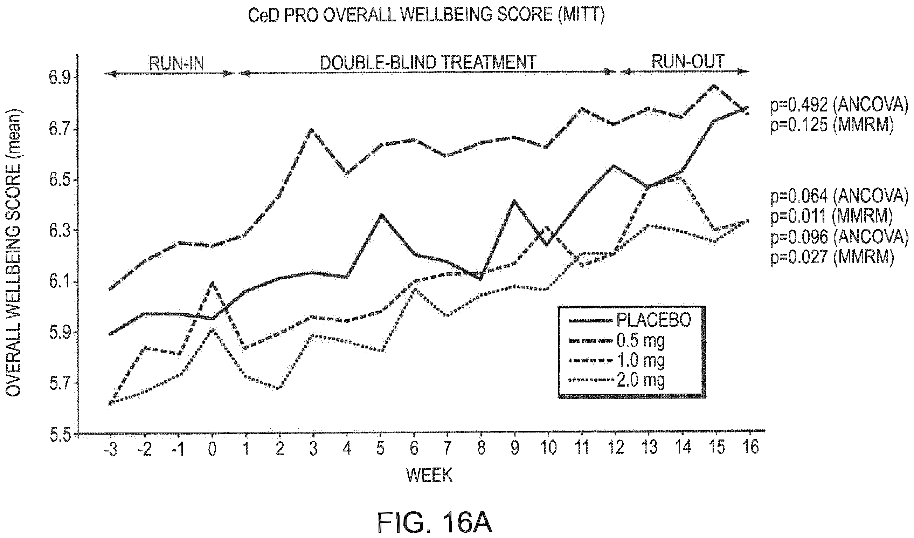

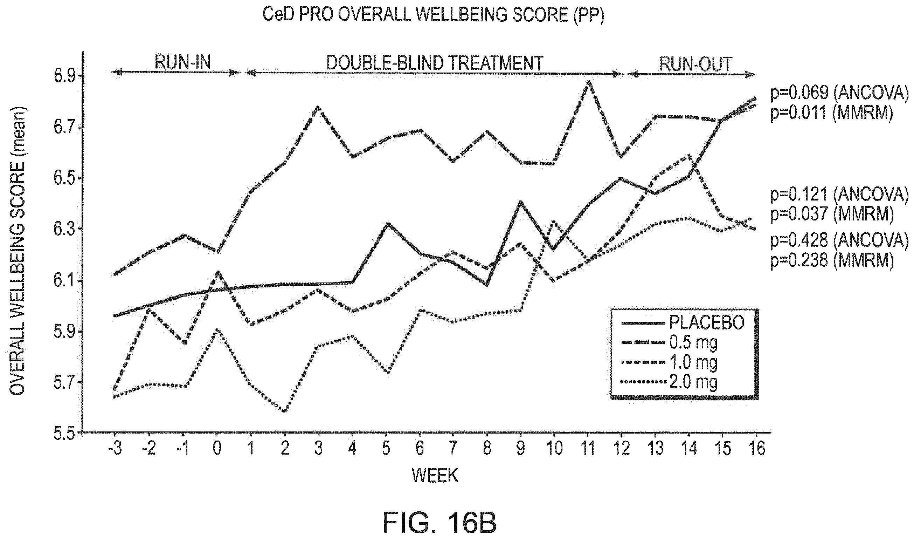

Change in the General Well-Being Question score from baseline to each study week is summarized for the MITT Population and for the Per-Protocol Population. Mean CeD GSRS scores are summarized graphically over time in Figure 16A for the MITT Population and Figure 16B for the Per-Protocol Population. Based on the ANCOVA and MMRM models, the General Well-Being Question score was higher in the Larazotide acetate 0.5 mg group compared to the placebo group at Week 8 in the MITT Population and at Week 3, Week 4, Week 8, and Week 11 in the Per-Protocol Population. In addition, statistically significant differences were noted at Week 2 and Week 6 in the Per-Protocol Population based on the MMRM model.

### U. Anti-tTG (IgA and IgG) Antibody Titers

Within each of the treatment groups in the MITT population, average anti-tTG antibody titers (IgA) were < 10 units and remained relatively unchanged throughout the study. There were no clinically meaningful post baseline differences in the anti-tTG antibody titers (IgA and IgG) between any of the Larazotide acetate and placebo groups in the MITT Population. Results were similar for the Per-Protocol Population.

### V. Anti-DGP (IgA and IgG) Antibody Titers:

Within each of the treatment groups in the MITT population, average anti-DGP antibody titers (IgA) remained relatively unchanged throughout the study. There were no clinically meaningful post baseline differences in the anti-DGP (IgA and IgG) antibody titers between any of the Larazotide acetate and placebo groups in the MITT Population. Results were similar for the Per-Protocol Population.

### W. SF12V2 Questionnaire:

Change from baseline to end of treatment in the total score and subscale total scores of the SF12V2 questionnaire is summarized for the MITT Population in Table 32.

**Table 32 Change From Baseline to End of Treatment for the SF12V2 Questionnaire - MITT Population**

| | | **Larazotide Acetate TID** | | |
|---|---|---|---|---|
| **Total and Subscale Totals** | **Placebo (N=84)** | **0.5 (N=85)** | **mg 1 (N=84)** | **mg 2 mg (N=87)** |
| **Total** | | | | |
| Baseline | | | | |
| N | 84 | 85 | 84 | 87 |
| Mean (SD) | 42.51 (8.22) | 41.93 (7.53) | 42.36 (6.32) | 40.91 (7.88) |
| Change to end of treatment | | | | |
| N | 72 | 74 | 74 | 76 |
| Mean (SD) | 0.17 (6.74) | 1.46 (5.32) | 0.50 (5.81) | 2.38 (6.97) |

| **Physical Component** | | | | |
|---|---|---|---|---|
| Baseline | | | | |
| N | 84 | 85 | 84 | 87 |
| Mean (SD) | 43.12 (6.03) | 43.29 (4.75) | 42.06 (5.13) | 42.68 (4.97) |
| Change to end of treatment | | | | |
| N | 72 | 74 | 74 | 76 |
| Mean (SD) | -1.87 (6.02) | -1.75 (4.48) | -0.31 (5.70) | -1.49 (5.97) |

| **Mental Component** | | | | |
|---|---|---|---|---|
| Baseline | | | | |
| N | 84 | 85 | 84 | 87 |
| Mean (SD) | 46.86 (6.92) | 46.33 (6.94) | 46.67 (7.28) | 45.78 (6.91) |
| Change to end of treatment | | | | |
| N | 72 | 74 | 74 | 76 |
| Mean (SD) | 0.53 (6.55) | 1.13 (6.61) | 0.23 (6.54) | 1.07 (7.75) |

| | | | | |
|---|---|---|---|---|
| Abbreviations: MITT = modified intent-to-treat; SD = standard deviation; SF12V2 = Short Form 12 health survey, Version 2; TID = three times daily. | | | | |

### X. Celiac Disease - Quality of Life Questionnaire:

Change from baseline to end of treatment in the total score and subscale total scores of the CeD-QoL questionnaire is summarized for the MITT Population in Table 33. Generally, the Larazotide acetate group had a slightly greater change from baseline compared to placebo.

**Table 33 Change From Baseline to End of Treatment in Celiac Disease Quality of Life - MITT Population**

| **Total and Subscale Totals** | **Placebo (N=84)** | **Larazotide Acetate TID** | | |
|---|---|---|---|---|
| | | **0.5 (N=85)** | **mg 1 (N=84)** | **mg 2 mg (N=87)** |
| **Standardized Total** | | | | |
| Baseline | | | | |
| N | 84 | 85 | 83 | 87 |
| Mean (SD) | 62.96 (16.53) | 62.79 (16.56) | 62.59 (14.48) | 65.61 (16.59) |
| Change to end of treatment | | | | |
| N | 71 | 74 | 73 | 76 |
| Mean (SD) | 5.23 (12.14) | 5.97 (12.73) | 6.59 (12.25) | 5.93 (12.15) |

| **Dysphoria Subscale** | | | | |
|---|---|---|---|---|
| Baseline | | | | |
| N | 84 | 85 | 83 | 87 |
| Mean (SD) | 15.83 (3.72) | 15.54 (3.86) | 15.83 (3.03) | 16.09 (3.46) |
| Change to end of treatment | | | | |
| N | 71 | 74 | 73 | 76 |
| Mean (SD) | 0.99 (2.57) | 1.23 (3.34) | 1.08 (2.36) | 1.05 (2.98) |

| **Limitation Subscale** | | | | |
|---|---|---|---|---|
| Baseline | | | | |
| N | 84 | 85 | 83 | 87 |
| Mean (SD) | 27.52 (8.06) | 27.31 (7.92) | 26.55 (7.74) | 28.87 (7.99) |
| Change to end of treatment | | | | |
| N | 71 | 74 | 73 | 76 |
| Mean (SD) | 2.34 (6.27) | 2.82 (6.27) | 3.25 (6.51) | 2.67 (5.90) |

| **Health Concerns Subscale** | | | | |
|---|---|---|---|---|
| Baseline | | | | |
| N | 84 | 85 | 83 | 87 |
| Mean (SD) | 14.33 (4.91) | 14.56 (5.02) | 14.81 (4.12) | 15.01 (5.08) |
| Change to end of treatment | | | | |
| N | 71 | 74 | 73 | 76 |
| Mean (SD) | 1.44 (3.78) | 1.27(4.11) | 1.49 (4.01) | 1.59 (3.89) |

| **Inadequate Treatment Subscale** | | | | |
|---|---|---|---|---|
| Baseline | | | | |
| N | 84 | 85 | 83 | 87 |
| Mean (SD) | 5.27 (2.39) | 5.38 (2.28) | 5.40 (2.18) | 5.63 (2.45) |
| Change to end of treatment | | | | |
| N | 71 | 74 | 73 | 76 |
| Mean (SD) | 0.46 (2.20) | 0.65 (2.24) | 0.77 (2.54) | 0.62 (2.03) |

| | | | | |
|---|---|---|---|---|
| Abbreviations: MITT = modified intent-to-treat; SD = standard deviation; TID = three times daily. | | | | |

For the primary efficacy analysis, the CeD GSRS score at Week 0 was included as a covariate in the model with treatment, gender, and the baseline stratification levels as fixed effects. Randomization cohort was also included as a fixed effect.

Missing efficacy data were not imputed. However, a sensitivity analysis using MMRM analysis was included for the important efficacy endpoints. This analysis is robust against data missing at random.

No interim analysis was planned or conducted.

Analyses were not stratified by center, and no summaries were provided for individual centers.

No multiplicity adjustments were made as this was a Phase IIb, hypothesis-generating study.

The Per-Protocol Population excluded subjects with major protocol deviations thought to impact the ability to assess the effect of treatment. Exclusion of subjects from the Per-Protocol Population was reviewed, documented, and approved before the study was unblinded.

There was no active control group in this study.

Subgroup analysis was not pre-specified and the study was not powered for subgroup analysis; therefore, this section is not applicable.

Efficacy results were summarized for 3 dose groups of Larazotide acetate (0.5 mg TID, 1 mg TID, and 2 mg TID). The relationship between Larazotide acetate concentration and efficacy was not analyzed.

Drug-drug and drug-disease interactions were not examined in this Phase IIb study.

No by-subject displays of individual response to study drug are presented, except as provided in the subject data listings.

### Efficacy Conclusions:

The primary efficacy endpoint of average on-treatment CeD GSRS score was met in this dose-ranging Phase IIb study as a statistically significantly lower mean score (*i.e.,* fewer symptoms) was observed in the Larazotide acetate 0.5 mg TID group (2.59) compared to the placebo group (2.88). Results for the Larazotide acetate 0.5 mg group were consistent across analysis methods and analysis population (MITT ANCOVA p=0.022 and MMRM p=0.005; Per-Protocol ANCOVA p=0.007 and MMRM p=0.001). No statistically significant differences from placebo were observed for the other Larazotide acetate groups (1 mg and 2 mg TID).

In the analyses of secondary endpoints, statistically significant differences from placebo favoring the Larazotide acetate 0.5 mg group were observed for the change from baseline to the end of treatment in CeD GSRS score in the MITT Population (p=0.041) and for average on-treatment score of CeD PRO Gastrointestinal Domain in the Per-Protocol Population (p=0.013) using the MMRM analysis method.

Other statistically significant differences from placebo in key exploratory endpoints, consistent with the efficacy of Larazotide acetate 0.5 mg include:
- Lower mean total GSRS score in the Larazotide acetate 0.5 mg group compared to the placebo group
- A larger percentage of subjects in the Larazotide acetate 0.5 mg group than the placebo group experienced reductions of ≥ 50% in the CeD PRO Abdominal Domain score
- The number of CeD PRO Improved Symptom days was increased in the Larazotide acetate 0.5 mg group compared to the placebo group
- The number of CeD PRO Symptomatic days was reduced in the Larazotide acetate 0.5 mg group, whereas an increase was observed in the placebo group
- Lower mean CeD PRO Non-Gastrointestinal Domain score in the Larazotide acetate 0.5 mg group compared to the placebo group
- No clinically meaningful increase in anti-tTG or anti-DGP antibodies was observed in any of the treatment groups.

In summary, this study demonstrates efficacy for Larazotide acetate 0.5 mg in the treatment of celiac disease, by meeting the primary endpoint as well as a range of secondary and exploratory endpoints. In addition, the daily CeD PRO was validated in this study and clinically meaningful endpoints for Phase 3 were identified.

### Safety Evaluation

### Extent of Exposure:

Overall exposure to study drug during the double-blind treatment phase is presented in Table 34. Exposure to study drug was similar among the placebo and Larazotide acetate treatment groups. Mean treatment duration ranged from 79.1 to 80.9 days across the treatment groups and the mean total number of capsules received ranged from 214.5 to 225.3.

**Table 34. Exposure to Study Drug During the Double-Blind Treatment Phase - Safety Population**

| | **Placebo (N=84)** | **Larazotide Acetate TID** | | |
|---|---|---|---|---|
| | | **0.5 (N=85)** | **mg 1 (N=84)** | **mg 2 mg (N=87)** |
| **Treatment Duration (days)^{a}** | N=81 | N=84 | N=84 | N=87 |
| Mean (SD) | 80.9(13.86) | 79.1 (17.20) | 79.7 (14.98) | 80.6 (15.77) |
| Median | 84 | 84 | 84 | 84 |
| Minimum, Maximum | 11,95 | 6, 97 | 5,92 | 11,95 |
| **Total Dose Received (capsules)^{b}** | N=81 | N=84 | N=84 | N=87 |
| Mean (SD) | 225.1 (44.65) | 214.5 (52.66) | 218.3 (55.05) | 225.3 (49.26) |
| Median | 236 | 229.5 | 232 | 241 |
| Minimum, Maximum | 22,281 | 20,310 | 18,346 | 32, 306 |

| | | | | |
|---|---|---|---|---|
| Abbreviations: SD = standard deviation; TID = three times daily. a. Treatment duration was calculated as the difference between the end of the double-blind treatment period (Week 12 or early withdrawal) and the day of randomization. b. Total dose received was calculated as the sum of the number of capsules dispensed - number of capsules returned during the double-blind treatment period. | | | | |

### Adverse Events:

An overview of treatment-emergent adverse events is presented in Table 35. The overall incidence of treatment-emergent adverse events was similar for subjects who received placebo (63.1%) and those who received Larazotide acetate (pooled doses 62.5%). In addition, no dose-response was observed for the overall incidence of treatment-emergent adverse events, with similar frequencies among the Larazotide acetate 0.5 mg (63.5%), 1 mg (61.9%), and 2 mg (62.1%) groups. The incidences of treatment-emergent adverse events that were severe, serious, or led to withdrawal from the study were also comparable among the placebo and Larazotide acetate treatment groups.

**Table 35. Overview of Treatment-Emergent Adverse Events - Safety Population**

| **Variable, n (%)** | **Placebo (N=84)** | **Larazotide Acetate TID** | | | |
|---|---|---|---|---|---|
| | | **0.5 (N=85)** | **mg 1 (N=84)** | **mg 2 mg (N=87)** | **Pooled Doses (N=256)** |
| Subjects with any treatment-emergent adverse event | 53 (63.1) | 54 (63.5) | 52 (61.9) | 54 (62.1) | 160 (62.5) |
| Subjects with severe treatment-emergent adverse events | 5 (6.0) | 4 (4.7) | 4 (4.8) | 3 (3.4) | 11 (4.3) |
| Subjects with serious treatment-emergent adverse events | 1 (1.2) | 2 (2.4) | 0 | 0 | 2 (0.8) |
| Subjects who withdrew from study due to treatment-emergent adverse events | 3 (3.6) | 4 (4.7) | 5 (6.0) | 3 (3.4) | 12 (4.7) |

| | | | | | |
|---|---|---|---|---|---|
| Abbreviations: TID = three times daily. | | | | | |

Treatment-emergent adverse events that occurred in ≥ 2.0% of the pooled Larazotide acetate group are presented by system organ class and treatment group in Table 36.

**Table 36. Treatment-Emergent Adverse Events That Occurred in ≥ 2.0% of the Pooled Larazotide Acetate Doses - Safety Population**

| **System Organ Preferred Term Class** | **Placebo (N=84)** | **Larazotide Acetate TID** | | | |
|---|---|---|---|---|---|
| | | **0.5 (N=85)** | **mg 1 (N=84)** | **mg 2 mg (N=87)** | **Pooled Doses (N=256)** |
| Subjects with at least 1 event | 53 (63.1) | 54 (63.5) | 52 (61.9) | 54 (62.1) | 160 (62.5) |
| Gastrointestinal Disorders | 21 (25.0) | 20 (23.5) | 28 (33.3) | 27 (31.0) | 75 (29.3) |
| Diarrhoea | 7 (8.3) | 7 (8.2) | 5 (6.0) | 8 (9.2) | 20 (7.8) |
| Nausea | 9 (10.7) | 4 (4.7) | 7 (8.3) | 7 (8.0) | 18 (7.0) |
| Constipation | 2 (2.4) | 3 (3.5) | 7 (8.3) | 5 (5.7) | 15 (5.9) |
| Abdominal pain | 4 (4.8) | 1 (1.2) | 5 (6.0) | 6 (6.9) | 12 (4.7) |
| Abdominal pain upper | 2 (2.4) | 3 (3.5) | 4 (4.8) | 2 (2.3) | 9 (3.5) |
| Flatulence | 0 | 2 (2.4) | 4 (4.8) | 3 (3.4) | 9 (3.5) |
| Gastroocsophageal reflux disease | 0 | 2 (2.4) | 1 (1.2) | 6 (6.9) | 9(3.5) |
| Abdominal distension | 1 (1.2) | 2 (2.4) | 5 (6.0) | 1 (1.1) | 8(3.1) |
| Vomiting | 6(7.1) | 2 (2.4) | 2 (2.4) | 3 (3.4) | 7 (2.7) |
| Dyspepsia | 3 (3.6) | 1 (1.2) | 2 (2.4) | 2 (2.3) | 5 (2.0) |
| Infections and Infestations | 25 (29.8) | 23 (27.1) | 23 (27.4) | 18 (20.7) | 64 (25.0) |
| Sinusitis | 5 (6.0) | 5 (5.9) | 5 (6.0) | 4 (4.6) | 14 (5.5) |
| Nasopharyngitis | 4 (4.8) | 4 (4.7) | 5 (6.0) | 3 (3.4) | 12 (4.7) |
| Influenza | 4 (4.8) | 3 (3.5) | 4 (4.8) | 2 (2.3) | 9 (3.5) |
| Urinary tract infection | 2 (2.4) | 1 (1.2) | 1 (1.2) | 5 (5.7) | 7 (2.7) |
| Gastroenteritis viral | 2 (2.4) | 1 (1.2) | 3 (3.6) | 2 (2.3) | 6 (2.3) |
| Upper respiratory tract infection | 0 | 2 (2.4) | 1 (1.2) | 3 (3.4) | 6 (2.3) |
| Nervous System Disorders | 10 (11.9) | 8 (9.4) | 6 (7.1) | 12 (13.8) | 26 (10.2) |
| Headache | 8 (9.5) | 2 (2.4) | 5 (6.0) | 6 (6.9) | 13 (5.1) |
| General Disorders and Administration Site Conditions | 2 (2.4) | 4 (4.7) | 5 (6.0) | 8 (9.2) | 17 (6.6) |
| Fatigue | 0 | 1 (1.2) | 3 (3.6) | 6 (6.9) | 10 (3.9) |

| | | | | | |
|---|---|---|---|---|---|
| Abbreviations: TID = three times daily. | | | | | |

The overall incidence of treatment-emergent adverse events was similar between the placebo group (63.1%) and the pooled Larazotide acetate group (62.5%). The most common types of events reported in placebo- and larazotide-treated subjects were gastrointestinal disorders (25.0% and 29.3%, respectively) and infections and infestations (29.8% and 25.0%, respectively).

Among the specific events reported, constipation (5.9% versus 2.4%), fatigue (3.9% versus 0), flatulence (3.5% versus 0), gastro-oesophageal reflux disease (3.5% versus 0), and upper respiratory tract infection (2.3% versus 0) occurred more frequently in the pooled Larazotide group compared to the placebo group. Treatment-emergent adverse events of nausea (10.7% versus 7.0%), headache (9.5% versus 5.1%), and vomiting (7.1% versus 2.7%) were more common in the placebo group compared to the pooled Larazotide acetate group.

The incidence of fatigue (placebo 0; Larazotide acetate 0.5 mg 1.2%; 1 mg 3.6%; and 2 mg 6.9%) and flatulence (placebo 0; Larazotide acetate 0.5 mg 2.4%; 1 mg 4.8%; and 2 mg 3.4%) tended to increase with increasing dose; no other potential dose-response was observed among the treatment groups for specific adverse events. In each of the groups, the majority of the treatment-emergent adverse events reported were considered by the Investigator to be mild or moderate in severity, with few subjects reporting severe events (placebo 6.0%; Larazotide acetate 0.5 mg 4.7%; 1 mg 4.8%; and 2 mg 3.4%). In addition, the majority of the treatment-emergent adverse events reported were considered by the Investigator to be unrelated to study drug; the frequency of subjects with related events was comparable among the treatment groups (placebo 16.7%; Larazotide acetate 0.5 mg 11.8%; 1 mg 19.0%; and 2 mg 17.2%).

The frequencies of adverse events reported during the single-blind, placebo run-in period were comparable among the treatment groups (placebo 27.4%; Larazotide acetate 0.5 mg 27.1%; 1 mg 25.0%; and 2 mg 23.0%), as were the frequencies of events during the single-blind placebo run-out period (placebo 17.9%; Larazotide acetate 0.5 mg 16.5%; 1 mg 10.7%; and 2 mg 19.5%). Similar to the profile noted during the double-blind treatment period, the most common events in the run-in and run-out periods were infections and infestations and gastrointestinal disorders.

No subjects died during the study.

One subject in the placebo group (appendicitis) and 2 subjects in the Larazotide acetate 0.5 mg group (depression and cholecystitis acute) had treatment-emergent serious adverse events during the study. Study drug was interrupted for these subjects and the events resolved within 2 to 6 days. Each of these events was considered severe; however, none was considered by the Investigator to be related to study drug. One additional subject (Subject 444010) in the Larazotide acetate 0.5 mg group had a serious adverse event of appendicitis that began > 7 days after the last dose of study drug and, therefore, was considered not treatment-emergent.

Subjects who experienced serious adverse events are presented in Table 37.

**Table 37. Listing of Subjects With Serious Adverse Events**

| **Serious AE** | | | | | | | | |
|---|---|---|---|---|---|---|---|---|
| **Subject** | **Age (years)/(Preferred** | | **AE** | **StartAE** | **StopTreatment** | | | |
| **Identifier** | **Gender** | **Term)** | **Study Day** | **Study Day** | **Emergent** | **Severity** | **Relationship** | **Action Taken** |
| **Placebo** | | | | | | | | |
| 477006 | 54/F | Appendicitis | 8 | 10 | Yes | Severe | Unlikely | Interrupted |

| **Larazotide Acetate 0.5 mg TID** | | | | | | | | |
|---|---|---|---|---|---|---|---|---|
| 444010 | 21/F | Appendicitis | 66 | 67 | No | Moderate | Definitely not | Interrupted/Other treatment given |
| 457006 | 61/F | Depression | 87 | 92 | Yes | Severe | Unlikely | Interrupted/Other treatment given |
| 457007 | 21/F | Cholecystitis acute | 14 | 15 | Yes | Severe | Definitely not | Interrupted/Other treatment given |

| | | | | | | | | |
|---|---|---|---|---|---|---|---|---|
| Abbreviations: AE = Adverse Event; F = female; TID = three times daily. | | | | | | | | |

Fifteen subjects, 3 (3.6%) in the placebo group, 4 (4.7%) in the Larazotide acetate 0.5 mg group, 5 (6.0%) in the Larazotide acetate 1 mg group, and 3 (3.4%) in the Larazotide acetate 2 mg group, withdrew from the study due to treatment-emergent adverse events. The majority of the treatment-emergent adverse events leading to withdrawal were gastrointestinal disorders, with no dose-related trends observed for the types of events that led to premature discontinuation from study. Two subjects in the Larazotide acetate 1 mg group were withdrawn due to diarrhea; no other event that led to withdrawal was reported by more than 1 Larazotide acetate subject.

No subject withdrew due to a treatment-emergent serious adverse event. All of the treatment-emergent adverse events leading to withdrawal were of mild or moderate severity, except for 1 severe event of headache reported by a subject in the Larazotide acetate 2 mg group.

Three additional subjects in the Larazotide acetate treatment groups discontinued due to non-treatment-emergent adverse events. Subject 436002 (0.5 mg group) withdrew due to myalgia, musculoskeletal stiffness, asthenia, and arthritis that began on Day -7. Subject 419010 (2 mg group) withdrew due to arthralgia, myalgia, headache, asthenia, and nausea that began on Day -12. Subject 424013 (2 mg group) withdrew due to headache that began on Day -23.

Subjects with treatment-emergent adverse events that led to study withdrawal are presented in Table 38.

**Table 38. Listing of Subjects With Treatment-Emergent Adverse Events That Led to Withdrawal From the Study**

| **Subject** | **Age (years)/** | | **TEAE** | **StartTEAE** | **Stop** | | |
|---|---|---|---|---|---|---|---|
| **Identifier** | **Gender** | **TEAE Preferred Term** | **Study Day** | **Study Day** | **Severity** | **Relationship** | **Serious** |
| **Placebo** | | | | | | | |
| 433014 | 48/F | Diarrhoea | 61 | 79 | Mild | Probably | No |
| | | Nausea | 61 | 79 | Mild | Probably | No |
| | | Abdominal pain upper | 61 | 79 | Mild | Probably | No |
| 474002 | 25/F | Colitis microscopic | 55 | Ongoing | Mild | Definitely not | No |
| 475002 | 28/F | Headache | 11 | 11 | Moderate | Probably | No |

| **Larazotide 0.5 mg TID** | | | | | | | |
|---|---|---|---|---|---|---|---|
| 409010 | 32/F | Basedow's disease | 21 | Ongoing | Moderate | Unlikely | No |
| 418006 | 57/F | Migraine | 22 | 32 | Moderate | Possibly | No |
| 461010 | 43/M | Eructation | 1 | 12 | Moderate | Probably | No |
| | | Flatulence | 1 | 12 | Moderate | Probably | No |
| | | Abdominal pain upper | 1 | 12 | Moderate | Probably | No |
| 468002 | 61/M | Gastrooesophageal reflux disease | 1 | Ongoing | Moderate | Definitely not | No |

| **Larazotide 1 mg TID** | | | | | | | |
|---|---|---|---|---|---|---|---|
| 402007 | 77/M | Diarrhoea | 2 | 9 | Moderate | Definitely not | No |
| 416019 | 28/F | Vertigo | 7 | 17 | Moderate | Probably | No |
| 422013 | 38/F | Abdominal distension | 0 | Ongoing | Moderate | Unlikely | No |
| | | Fatigue | 28 | Ongoing | Moderate | Unlikely | No |
| | | Faecal incontinence | 28 | Ongoing | Moderate | Unlikely | No |
| 436010 | 30/M | Coeliac disease | 58 | 70 | Moderate | Unlikely | No |
| | | Gastroenteritis viral | 58 | 70 | Moderate | Definitely not | No |
| 477013 | 69/F | Diarrhoea | 46 | 53 | Moderate | Possibly | No |

| **Larazotide 2 mg TID** | | | | | | | |
|---|---|---|---|---|---|---|---|
| 402013 | 41/F | Anxicty | 79 | 92 | Moderate | Definitely not | No |
| 415004 | 24/F | Headache | 11 | Ongoing | Severe | Definitely | No |
| 474004 | 23/F | Memory impairment | 0 | Ongoing | Mild | Unlikely | No |
| | | Disturbance in attention | 0 | Ongoing | Mild | Unlikely | No |
| | | Constipation | 0 | 59 | Mild | Possibly | No |
| | | Nausea | 0 | 59 | Mild | Possibly | No |

| | | | | | | | |
|---|---|---|---|---|---|---|---|
| Abbreviations: F = female; M = male; TEAE = treatment-emergent adverse event; TID = three times daily | | | | | | | |

No subjects died in the study. Similar proportions of subjects in the placebo and pooled Larazotide acetate group experienced serious adverse events (1.2% and 0.8%, respectively), and treatment-emergent adverse events leading to withdrawal from the study (3.6% and 4.7%, respectively). No dose-related trends were apparent for the incidence of serious adverse events or for treatment-emergent adverse events leading to withdrawal.

### Laboratory Values Over Time

### Hematology:

Mean values for hematology parameters were generally similar among the placebo and Larazotide acetate treatment groups at Week 4, end of treatment, and end of follow-up. At the end of treatment, no change or small mean increases or decreases from baseline were observed for each parameter (Table 39). None of the mean changes in hematology parameters were considered to be clinically significant.

**Table 39. Mean Values and Mean Changes From Baseline to End of Treatment in Hematology Values - Safety Population**

| **Mean Value (SD)**/ **Mean Change (SD)** | **Placebo (N=84)** | **Larazotide Acetate TID** | | | |
|---|---|---|---|---|---|
| | | **0.5 (N=85)** | **mg 1 (N=84)** | **mg 2 (N=87)** | **mg** |
| | N=68 | N=71 | N=71 | N=71 | |
| Hemoglobin (g/dL) | 13.6 (1.39)/ -0.2 (0.54) | /13.4 (1.16)/ 0 (0.57) | 13.5 (1.28)/ 0.1 (0.64) | 13.6 0.1 (0.59) | (1.15)/ |
| Hematocrit (%) | 40.5 (4.18)/ -0.3 (1.64) | / 40 (3.24)/ 0.1 (1.89) (N = 70) | 40.3 (3.77)/ 0.5 (2.11) | 40.5 0.4 (1.84) (N = 70) | (3.31)/ |
| Platelet count (K/µL) | 249.6 (63.12)/ -7.8 (38.4) | /244 (55.89)/ 2.2 (24.1) | 250 (57.67)/ -2 (27.95) | 253.3 3.1 (34.9) | (63.89)/ |
| White blood cells (K/µL) | 6 (1.75)/ 0(1.2) | / 5.9 (1.55)/ 0.1(1.17) | 6.2 (1.72)/ -0.2 (1.44) | 5.8 0.2 (1.26) | (1.45)/ |
| Basophils (%) | 0.3 (0.47)/ -0.1 (0.6) | / 0.4 (0.49)/ 0.1 (0.66) | 0.3 (0.47)/ -0.1 (0.62) | 0.4 0(0.64) | (0.52)/ |
| Eosinophils (%) | 3.5 (3.02)/ 0.2 (1.98) | / 2.7 (2.23)/ -0.1 (1.95) | 2.4 (1.67)/ -0.1 (1.37) | 3.2 0(1.93) | (2.36) |
| Lymphocytes (%) | 29.3 (7.97)/ 0.1 (6.7) | / 29.2 (6.88)/ -0.2 (5.37) | 29.2 (9.21)/ 0.3 (7.24) | 30.5 0.9 (9.23) | (10.23)/ |
| Monocytes (%) | 7.6 (2.26)/ 0.1 (2.38) | /6.9 (2.27)/ -0.1 (1.73) | 7.4 (2.88)/ -0.1 (2.55) | 7.5 -0.4 (2.39) | (2.39)/ |
| Neutrophils (%) | 59.4 (9.09)/ -0.3 (8.61) | / 61 (7.35)/ 0.3 (6.67) | 60.8 (10.18)/ -0.2 (8.81) | 58.5 -0.6 (10.28) | (11.48)/ |

| | | | | | |
|---|---|---|---|---|---|
| Abbreviations: SD = standard deviation; TID = three times daily. | | | | | |

### Chemistry:

Mean values for chemistry parameters were generally similar across the placebo and Larazotide acetate treatment groups at Week 4, end of treatment, and end of follow-up. At the end of treatment, no change or small mean increases or decreases from baseline were observed for each parameter (Table 40). None of the mean changes in chemistry parameters were considered to be clinically significant.

**Table 40. Mean Values and Mean Changes From Baseline to End of Treatment in Chemistry Values - Safety Population**

| | | **Larazotide Acetate TID** | | |
|---|---|---|---|---|
| **Mean Value (SD)/ Mean Change (SD)** | **Placebo (N=84)** | **0.5 (N=85)** | **mg 1 (N=84)** | **mg 2 mg (N=87)** |
| | N=69 | N=74 | N=72 | N=73 |
| AST (IU/L) | 22.4 (7.45)/ -1.2 (5.36) | 23.7 (9.15)/ -1.5 (6.95) | 20.9 (6.48)/ -0.5 (4.95) | 22.9 (11.29)/ -0.9 (10.40) |
| ALT (IU/L) | 20.9 (9.69)/ -2.4 (8.09) | 22.7 (10.83)/ -0.8 (8.74) | 21.9 (16.39)/ 0.8 (9.97) | 22.2 (13.96)/ -0.6 (16.86) |
| Alkaline phosphatase (IU/L) | 59.9 (19.05)/ -2.3 (13.50) | 63.1 (35.71)/ -3.0 (7.91) | 63.3 (29.25)/ 2.5 (19.49) | 64.2 (24.66)/ -0.4 (11.19) |
| Total bilirubin (mg/dL) | 0.5 (0.22)/ -0.0 (0.20) | 0.6 (0.37)/ -0.0 (0.33) | 0.5 (0.23)/ -0.0 (0.22) | 0.5 (0.29)/ -0.0 (0.25) |
| BUN (mg/dL) | 13.0 (3.59)/ 0.2 (3.04) | 12.1 (3.96)/ 0.2 (3.83) | 12.9 (4.67)/ 0.3 (3.32) | 13.0 (4.57)/ 1.0 (3.94) |
| Creatinine (mg/dL) | 0.8 (0.15)/ -0.0 (0.11) | 0.8 (0.15)/ -0.0 (0.10) | 0.8 (0.16)/ 0.0 (0.09) | 0.8 (0.15)/ 0.0 (0.10) |
| Calcium (mg/dL) | 9.5 (0.35)/ 0.0 (0.34) | 9.4 (0.30)/ -0.1 (0.33) | 9.4 (0.36)/ 0.0 (0.36) | 9.4 (0.36)/ -0.0 (0.30) |
| Chloride (mmol/L) | 103.2 (2.58)/ -0.4 (2.55) | 104.7 (2.48)/ -0.1 (2.84) (N = 73) | 103.8 (2.66)/ -0.3 (2.40) (N = 71) | 103.6 (2.59)/ -0.7 (2.40) |
| Glucose (random) (mg/dL) | 86.2 (12.99)/ -1.6 (13.96) | 93.2 (14.35)/ 2.4 (15.72) | 93.1 (17.61)/ 2.2 (17.94) | 90.9 (21.61)/ 0.2 (21.60) |
| Potassium (mmol/L) | 4.2 (0.34)/ -0.0 (0.34) | 4.1 (0.33)/ -0.1 (0.38) | 4.1 (0.25)/ -0.1 (0.35) | 4.2 (0.34)/ -0.1 (0.43) |
| Sodium (mmol/L) | 138.2 (2.23)/ -0.1 (1.80) | 138.3 (1.49)/ -0.1 (2.39) | 138.1 (2.10)/ 0.2 (2.24) | 138.1 (2.17)/ -1.0 (2.06) |
| Albumin (g/dL) | 4.3 (0.27)/ 0.0 (0.26) | 4.1 (0.34)/ 0.0 (0.28) | 4.2 (0.31)/ 0.0 (0.24) | 4.2 (0.37)/ 0.0 (0.26) |

| | | | | |
|---|---|---|---|---|
| Abbreviations: ALT = alanine aminotransferase; AST = aspartate aminotransferase; BUN = blood urea nitrogen; SD = standard deviation; TID = three times daily. | | | | |

### Individual Subject Changes:

Almost all subjects had normal values for hematology and chemistry parameters at baseline and remained normal at Week 4, end of treatment, and end of follow-up. Shifts were generally similar in the placebo and Larazotide acetate groups. No dose-related trends in laboratory shifts were apparent. None of the shifts in hematology or chemistry parameters were of clinical concern.

### Individual Clinically Significant Abnormalities:

The proportions of subjects who developed potentially clinically significant abnormal laboratory values during the treatment period were similar among the placebo (20.2%) and the Larazotide acetate 0.5 mg (17.6%), 1 mg (22.6%), and 2 mg (19.5%) treatment groups. A decrease in serum ferritin was the most common potentially clinically significant abnormal value observed, with no important differences noted among the placebo and Larazotide treatment groups. A summary of potentially clinically significant laboratory values during the treatment period are presented by treatment group in Table 41.

**Table 41. Potentially Clinically Significant Abnormal Laboratory Values During the Treatment Period - Safety Population**

| | | **Placebo (N=84)** | **Larazotide Acetate TID** | | |
|---|---|---|---|---|---|
| | | | **0.5 mg (N=85)** | **1 mg (N=84)** | **2 mg (N=87)** |
| Subjects with at least 1 PCS abnormal test | | 17 (20.2) | 15 (17.6) | 19 (22.6) | 17 (19.5) |

| **Hematology** | **PCS Criterion** | | | | |
|---|---|---|---|---|---|
| Hemoglobin | ≤ 0.75 × LLN | 1 (1.2) | 0 | 0 | 0 |
| Hematocrit | 0.75 × LLN | 0 | 0 | 0 | 0 |
| White blood cells | ≤ 0.75 × LLN or ≥ 1.5 × ULN | 1 (1.2) | 0 | 0 | 0 |
| Neutrophils | ≤ 0.75 × LLN or ≥ 1.5 × ULN | 3 (3.6) | 2 (2.4) | 2 (2.4) | 2 (2.3) |
| Lymphocytes | ≤ 0.75 × LLN or ≥ 1.5 × ULN | 4 (4.8) | 1 (1.2) | 5 (6.0) | 5 (5.7) |
| Ferritin serum | ≤ 0.75 × LLN | 9 (10.7) | 13 (15.3) | 12 (14.3) | 9 (10.3) |

| **Chemistry** | | | | | |
|---|---|---|---|---|---|
| Glucose random | ≤ 0.75 × LLN | 1 (1.2) | 0 | 0 | 2 (2.3) |
| AST | ≥ 3 × ULN | 0 | 1 (1.2) | 0 | 0 |
| ALT | ≥ 3 × ULN | 0 | 0 | 0 | 0 |
| Alkaline phosphatase | ≥ 3 × ULN | 0 | 1 (1.2) | 0 | 0 |

| | | | | | |
|---|---|---|---|---|---|
| Abbreviations: ALT = alanine aminotransferase; AST = aspartate aminotransferase; LLN = lower limit of normal; PCS = potentially clinically significant; TID = three times daily; ULN = upper limit of normal. | | | | | |

### Vital Signs, Physical Findings, and Other Observations Related to Safety

### Vital Signs and Physical Examination:

No clinically meaningful differences were observed among the placebo and Larazotide acetate treatment groups for mean changes from baseline to Week 4, Week 8, end of treatment, or end of follow-up in vital sign variables including systolic and diastolic blood pressure, pulse rate, and body temperature.

Small mean decreases (range: -0.1 to -0.3 kg/m²) from baseline to Week 4, Week 8, end of treatment, and end of follow-up in BMI were observed in the placebo group, whereas small mean increases or no changes (range: 0 to 0.2 kg/m²) were observed in the Larazotide 0.5, 1, and 2 mg treatment groups. Generally similar results were also observed for mean changes from baseline in body weight (placebo range: -0.2 to -0.8 kg, Larazotide range: -0.1 to 0.5 kg).

### Electrocardiograms:

None of the ECGs performed at screening and the end of treatment were considered clinically significant. One subject (Subject 402013) in the Larazotide acetate 2 mg group had a clinically significant abnormal finding on an ECG performed on Study Day 93 in the placebo run-out phase. The abnormality was indicated by the Investigator as possibly due to hypertension and the subject's systolic and diastolic blood pressure at that time was 155/97 mmHg. The subject was prematurely withdrawn from the study due to an adverse event of moderate anxiety; an event of moderate blood pressure increased was also reported. The subject had no prior history of hypertension.

### Pregnancy Testing:

One female (Subject 458014) of childbearing potential in the Larazotide acetate 2 mg group had a positive serum pregnancy test on Study Day 27 and was discontinued from the study. All pregnancy testing performed on this subject prior to the start of dosing had been negative. She had an uncomplicated delivery of a healthy baby in October 2013.

### Safety Conclusions:

Larazotide acetate was well tolerated and an acceptable safety profile was demonstrated across all 3 doses of Larazotide acetate. The overall incidence of treatment-emergent adverse events was similar between the placebo group (63.1%) and the pooled Larazotide acetate group (62.5%). The most common types of events reported in subjects who were treated with placebo or Larazotide acetate were gastrointestinal disorders (25.0% and 29.3%, respectively) and infections and infestations (29.8% and 25.0%, respectively). The incidences of treatment-emergent adverse events that were severe (6.0% and 4.3%, respectively), serious (1.2% versus 0.8%, respectively), or led to withdrawal from the study (3.6% versus 4.7%, respectively) were comparable among the placebo and Larazotide acetate treatment groups.

There were no clinically significant differences in hematology or chemistry values, ECG results, or vital signs between the placebo and Larazotide acetate treatment groups.

### Discussion and Overall Conclusion

The clinical study as described herein was a multicenter, randomized, double-blind, placebo-controlled trial designed to establish the efficacy and safety of Larazotide acetate in subjects with celiac disease following a GFD. Specifically, a total of 342 subjects with celiac disease (98.8% white, 0.6% Asian, 0.3% black, and 0.3% American Indian/Alaska Native) with a mean age of 45.2 years (range: 18 to 77 years of age) were randomized into the study.

The primary efficacy endpoint was based on results of the CeD GSRS, which is a subscale of the validated GSRS scale used for evaluating subjects with celiac disease. The average on-treatment score of the CeD GSRS was selected for the primary endpoint as it captures the daily treatment effect in a chronic, variable condition like celiac disease. The doses of Larazotide acetate selected for this study (0.5 mg, 1 mg, and 2 mg TID) were based on results of previous clinical studies that had studied doses ranging from 0.25 mg to 8 mg TID.

The primary efficacy endpoint of the study was met at the dose of 0.5 mg TID of Larazotide acetate. Larazotide acetate 0.5 mg TID significantly reduced gastrointestinal symptoms associated with celiac disease as measured by the CeD GSRS score, whereas the higher dose groups (1 mg and 2 mg TID) showed no benefit over placebo. These results are consistent with previous studies of Larazotide acetate where lower doses (0.25 mg and 1 mg) have proven to be more effective compared to placebo than higher doses of 4 mg or 8 mg. In contrast with systemically absorbed small molecule drugs, a typical dose-response relationship was not observed as this is a non-absorbed oral peptide where lower doses appear to be more effective than higher doses. The reason for this is unknown, however; this phenomenon may be explained by the mechanism of action of Larazotide acetate, as it is thought to work via the ZO-1 pathway through regulation of the tight junctions in the small intestine. It is conceivable that doses above a certain level may act as a competitive inhibitor. This is not unprecedented for non-absorbed oral peptides, and a similar reverse dose-response relationship has been observed with linaclotide acetate, another small non-absorbed oral peptide. Linaclotide acetate was evaluated for treatment of irritable bowel syndrome with constipation and chronic constipation in dose-finding, Phase II studies at doses up to 1000 µg/day; however, lower doses (145 µg/day and 290 µg/day) were proven more efficacious than the higher doses and were subsequently approved for Irritable Bowel Syndrome-Constipation and Chronic Idiopathic Constipation by the FDA and the European Medicines Agency.

The study met the primary endpoint, with a statistically significantly lower average on-treatment score for the CeD GSRS in the Larazotide acetate 0.5 mg group compared to the placebo group. Statistically significant differences from placebo favoring the Larazotide acetate 0.5 mg group were observed for the secondary endpoints of change from baseline to the end of treatment in CeD GSRS score in the MITT Population and for average on-treatment score of CeD PRO Gastrointestinal Domain in the Per-Protocol Population using the MMRM analysis method. Differences from placebo in favor of the Larazotide acetate 0.5 mg group were consistently observed for all of the secondary endpoints, although not all achieved statistical significance. Analyses of exploratory endpoints demonstrated consistent differences from placebo favoring Larazotide acetate 0.5 mg for the reduction of the number of CeD PRO Symptomatic days, increase of CeD PRO Improved Symptom days, weekly average CeD PRO Abdominal Domain for 6 of 12 weeks, CeD PRO Non-Gastrointestinal Domain (headache and tiredness), total GSRS score, and the number of weekly bowel movements. A 26% reduction in CeD PRO Symptomatic days (there were 0.56 days/week or 6.72 fewer CeD PRO Symptomatic Days during the 12-week treatment period) and a 31% increase in CeD PRO Improved Symptom days (there were 0.49 days/week increased CeD PRO Improved Symptom days or 5.88 more CeD PRO Improved Symptom days during the 12-week treatment period) were observed with the 0.5 mg dose of Larazotide acetate versus GFD alone. In addition, no clinically meaningful increase in anti-tTG (IgA and IgG) or in anti-DGP (IgA and IgG) antibodies was seen during the study.

The least squares mean differences from placebo for the primary and secondary endpoints are presented below in Table 42 for the Larazotide acetate 0.5 mg group using the MITT and Per-Protocol Populations.

**Table 42.**

| | **Larazotide Acetate 0.5 mg LSM difference from placebo/p-value** | | | |
|---|---|---|---|---|
| | **MITT Population** | | **Per-Protocol Population** | |
| **Endpoints** | **ANCOVA** | **MMRM** | **ANCOVA** | **MMRM** |
| **Primary:** Average on-treatment CeD GSRS | -0.23/ p=0.022 | -0.22/ p=0.005 | -0.30/ p=0.007 | -0.27/ 0.001 |
| **Secondary:** | | | | |
| Change from baseline to end of treatment in | -0.17/ | -0.26/ | -0.21/ | -0.22/ |
| CeD GSRS | p=0.228 | p=0.041 | p=0.177 | p=0.108 |
| Average on-treatment CeD PRO Abdominal Domain | -0.08/ p=0.624 | -0.11/ p=0.363 | -0.27/ p=0.153 | -0.25/ p=0.071 |
| Change from baseline to end of treatment in CeD PRO Abdominal Domain | -0.02/ p=0.932 | -0.04/ p=0.860 | -0.12/ p=0.608 | -0.09/ p=0.670 |
| Average on-treatment CeD PRO Gastrointestinal Domain | -0.16/ p=0.268 | -0.19/ p=0.070 | -0.33/ p=0.036 | -0.32/ p=0.006 |
| Change from baseline to end of treatment in CeD PRO Gastrointestinal Domain | -0.11/ p=0.548 | -0.12/ p=0.485 | -0.22/ p=0.289 | -0.17/ p=0.356 |

| | | | | |
|---|---|---|---|---|
| Abbreviations: ANCOVA = analysis of covariance; CeD GSRS = Celiac Disease domains of the Gastrointestinal Symptoms Rating Scale; CeD PRO = Celiac Disease Patient Reported Outcome; LSM = least squares mean; MMRM = mixed model for repeated measures. | | | | |

There was no clinically meaningful difference in anti-tTG (IgA and IgG) changes from baseline to end of treatment between 0.5 mg, 1 mg, and 2 mg of Larazotide acetate and placebo, despite the fact that a higher proportion of subjects in the Larazotide acetate 0.5 mg dose group reported that they had knowingly ingested food or beverages containing gluten (35%) during the study compared with the other treatment groups (27% in the placebo group, 25% in the Larazotide acetate 1 mg group, and 22% in the Larazotide acetate 2 mg group) or that 59% of the 0.5 mg Larazotide acetate group reported that they had ingested something they thought was gluten-free but later learned was not compared with 52%, 39%, and 38% of the placebo and Larazotide acetate 1 mg and 2 mg groups, respectively, as measured by the GFDCQ.

Larazotide acetate was well tolerated and an acceptable safety profile was demonstrated. The overall incidence and type of treatment-emergent adverse events reported in subjects treated with Larazotide acetate were generally similar to placebo. There were no clinically significant differences in hematology or chemistry values, ECG results, or vital signs between the placebo and Larazotide acetate treatment groups.

In conclusion, the results of this clinical study demonstrate that Larazotide acetate, a first-in-class oral peptide and Tight Junction Regulator, reduced gastrointestinal symptoms in subjects with celiac disease who were attempting to follow a GFD better than a GFD alone, with an acceptable safety and tolerability profile. In addition, comprehensive validation of the CeD PRO was completed in this trial.

### EQUIVALENTS

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth and as follows in the scope of the appended claims.

Those skilled in the art will recognize, or be able to ascertain, using no more than routine experimentation, numerous equivalents to the specific embodiments described specifically herein. Such equivalents are intended to be encompassed in the scope of the following claims.

### INCORPORATION BY REFERENCE

All patents and publications referenced herein are hereby incorporated by reference in their entireties.

### REFERENCES

Ciclitira PJ, Ellis HJ, Lundin KE. Gluten-free diet--what is toxic? Best Pract Res Clin Gastroenterol. 2005;19(3):359-71.
Dimenäs E. Methodological aspects of evaluation of quality of life in upper gastrointestinal disease. Scand J Gastroenterol. 1993a;28(suppl 199):18-21.
Dimenäs E, Glise H, Hallerbäck B, Hernqvist H, Svedlund J, Wiklund I. Quality of life in patients with upper gastrointestinal symptoms, an improved evaluation of treatment regimens? Scand J Gastroenterol. 1993b;28(8):681-7.
Drago S, El Asmar R, Di Pierro M, Grazia Clemente M, Tripathi A, Sapone A, et al. Gliadin, zonulin and gut permeability: effects on celiac and non-celiac intestinal mucosa and intestinal cell lines. Scand J Gastroenterol. 2006; 41(4): 408-19.
Dubé C, Rostom A, Sy R, Cranney A, Saloojee N, Garritty C, et al. The prevalence of celiac disease in average-risk and at-risk Western European populations: a systematic review. Gastroenterology. 2005 Apr;128(4 Suppl 1):S57-67.
Fasano A. Regulation of intercellular tight junctions by zonula occludens toxin and its eukaryotic analogue zonulin. Ann NY Acad Sci. 2000a;915:214-22.
Fasano A, Not T, Wang W, Uzzau S, Berti I, Tommasini A, et al. Zonulin, a newly discovered modulator of intestinal permeability, and its expression in coeliac disease. Lancet. 2000b;355(9214):1518-9.
Green PH, Fleischauer AT, Bhagat G, Goyal R, Jabri B, Neugut AI. Risk of malignancy in patients with celiac disease. Am J Med. 2003;115(3):191-5.
Green PH, Cellier C. Celiac disease. N Engl J Med. 2007;357:1731-43.
Gopalakrishnan S, Durai M, Kitchens K, Tamiz AP, Somerville R, Ginski M, et al. Larazotide acctatc regulates epithelial tight junctions in vitro and in vivo. Peptides. 2012a;35:86-94.
Gopalakrishnan S, Tripathi A, Tamiz AP, Alkan SS, Pandey NB. Larazotide acetate promotes tight junction assembly in epithelial cells. Peptides. 2012b;35: 95-101.
Hall JN, Rubin GP, Charnock A. Intentional and inadvertent non-adherence in adult coeliac disease. a cross-sectional survey. Appetite. 2013;68:56-62.
Lewis SJ, Heaton KW. Stool form scale as a useful guide to intestinal transit time. Scand. J. Gastroenterol. 1997;32 (9):920-4.
Norris JM, Barriga K, Hoffenberg EJ, Taki I, Miao D, Haas JE, et al. Risk of celiac disease autoimmunity and timing of gluten introduction in the diet of infants at increased risk of disease. JAMA. 2005;293(19):2343-51.
Rostom A, Murray JA, Kagnoff MF. American Gastroenterological Association (AGA) Institute technical review on the diagnosis and management of celiac disease. Gastroenterology. 2006 Dec;131(6):1981-2002.
Wang W, Uzzau S, Goldblum SE, Fasano A. Human zonulin, a potential modulator of intestinal tight junctions. J Cell Sci. 2000;113: 4435-40.

### Disclosed items:

1. A method for treating celiac disease, comprising: administering a composition containing about 0.25 mg to about 1 mg of Larazotide to a symptomatic celiac disease patient, the composition releasing the Larazotide in the duodenum and jejunum, and the composition being administered about 3 times per day for at least about 8 weeks.
2. The method of item 1, wherein the composition contains about 0.5 mg of Larazotide.
3. The method of item 1, wherein the composition contains about 0.25 mg, or about 0.3 mg, or about 0.4 mg of Larazotide.
4. The method of item 1, wherein the composition contains about 0.6 mg, about 0.7 mg, about 0.75 mg, about 0.8 mg, about 0.9 mg, or about 1 mg of Larazotide.
5. The method of any one of items 1 to 4, wherein the Larazotide is Larazotide acetate.
6. The method of any one of items 1 to 5, wherein the patient is on a gluten-free diet, including only unintentional exposure to gluten.
7. The method of any one of items 1 to 6, wherein the patient has celiac disease that is nonresponsive or refractory to gluten-free diet.
8. The method of any one of items 1 to 7, wherein the symptoms are one or more of abdominal cramping, abdominal pain, bloating, gas, diarrhea, loose stools, nausea, vomiting, indigestion, reflux, constipation, vomiting, headache, and tiredness.
9. The method of item 8, wherein the patient is experiencing headaches or tiredness.
10. The method of item 7 or 8, wherein the patient is experiencing one or more CeD PRO abdominal domain symptoms selected from abdominal pain, abdominal cramping, bloating or gas, and/or one or more CeD PRO GI domain symptoms selected from abdominal cramping, abdominal pain, bloating, gas, diarrhea, loose stools, nausea, indigestion, reflux, constipation, and vomiting, and
   the patient is experiencing at least one CeD PRO non-GI domain symptom.
11. The method of any one of items 1 to 10, wherein the CeD PRO non-GI domain symptom(s) are one or more of headache or tiredness.
12. The method of any one of items 1 to 11, wherein the patient is experiencing dermatitis herpetiformis.
13. The method of any one of items 1 to 12, wherein the patient is experiencing one or more CeD PRO Symptomatic days of at least 2, 3, 4, 5, 6, or 7,
   wherein a CeD PRO Symptomatic days is a day where the mean of abdominal cramping, abdominal pain, bloating, and gas is scored as ≥ 2.5 or ≥ 3 out of a 0 to 10 scale, or a day where the mean of diarrhea and loose stool is scored as ≥ 2.5 or ≥ 3 out of a 0 to 10 scale, or a day where nausea is scored as ≥ 2.5 or ≥ 3 out of a 0 to 10 scale.
14. The method of item 13, wherein upon treatment the patient experiences one or more CeD PRO Improved Symptom days per week, wherein a CeD PRO Improved Symptom day is a day where the mean of abdominal cramping, abdominal pain, bloating, and gas is scored as ≤ 1.5 out of a 0 to 10 scale, and a day where the mean of diarrhea and loose stool is scored as ≤ 1.5 out of a 0 to 10 scale, and a day where nausea is scored as ≤ 1 out of a 0 to 1 0 scale.
15. The method of any one of items 1 to 14, wherein the patient scores at least 2, or at least 3, or at least 4, or at least 5, or at least 6 on the CeD Gastrointestinal Symptom Rating Scale (CeD GSRS) at the start of treatment using the 1-7 Likert scale.
16. The method of any one of items 1 to 14, wherein the patient scores at least 2, or at least 3, or at least 4, or at least 5, or at least 6, or 7 on the Total Gastrointestinal Symptom Rating Scale (GSRS) at the start of treatment using the 1-7 Likert scale.
17. The method of any one of items 1 to 16, wherein the patient experiences at least about 3, 4, 5, 6, 7, 8, 9, 10, or more bowel movements per day at the start of treatment.
18. The method of item 17, wherein the patient experiences more than about 12, more than about 14, more than about 16, or more than about 18 bowel movements per day at the start of treatment.
19. The method of any one of items 1 to 16, wherein the patient experiences at least about 4, at least about 5, at least about 6, at least about 7, or at least about 8, or at least about 9, or at least about 10 diarrhea or loose stools per day on the Bristol Form Scale (BSFS) at the start of treatment.
20. The method of any one of items 1 to 16, wherein the patient experiences ≥ 3 diarrhea or loose stools per day with a score of 5-7 as measured by the Bristol Form Scale (BSFS) at the start of treatment.
21. The method of any one of items 1 to 20, wherein the composition is administered for at least about 9 weeks, or at least about 10 weeks, or at least about 12 weeks.
22. The method of item 21, wherein the composition is administered for at least about six months.
23. The method of item 21, wherein the composition is administered for at least about one year.
24. The method of any one of items 1 to 23, wherein the composition is administered prior to meals.
25. The method of item 24, wherein the composition is administered about 60 minutes, or about 45 minutes, or about 30 minutes, or about 15 minutes, or about 10 minutes, or about 5 minutes prior to meals.
26. The method of any one of items 1 to 25, wherein the composition is a delayed release composition described in US Patent 8,168,594 (which is hereby incorporated by reference in its entirety), which releases Larazotide in the duodenum and jejunum.
27. The method of any one of items 1 to 26, wherein the composition further releases Larazotide in the ileum and/or the colon.

## Claims

1. A composition for use in treating celiac disease in a symptomatic celiac disease patient, the composition containing about 0.25 mg to about 1 mg of Larazotide, the composition releasing the Larazotide in the duodenum and jejunum, wherein the composition is to be administered about 3 times per day for at least about 8 weeks.

2. The composition for the use of claim 1, wherein the composition contains about 0.25 mg, or about 0.3 mg, or about 0.4 mg, or about 0.5 mg, about 0.6 mg, about 0.7 mg, about 0.75 mg, about 0.8 mg, about 0.9 mg, or about 1 mg of Larazotide, optionally wherein the Larazotide is Larazotide acetate.

3. The composition for the use of claim 1 or claim 2, wherein the patient is on a gluten-free diet, including only unintentional exposure to gluten, optionally wherein the patient has celiac disease that is nonresponsive or refractory to gluten-free diet.

4. The composition for the use of any one of claims 1 to 3, wherein the symptoms are one or more of abdominal cramping, abdominal pain, bloating, gas, diarrhea, loose stools, nausea, vomiting, indigestion, reflux, constipation, vomiting, headache, and tiredness, optionally wherein the patient is experiencing headaches or tiredness.

5. The composition for the use of claim 4, wherein the patient is experiencing one or more CeD PRO abdominal domain symptoms selected from abdominal pain, abdominal cramping, bloating or gas, and/or one or more CeD PRO GI domain symptoms selected from abdominal cramping, abdominal pain, bloating, gas, diarrhea, loose stools, nausea, indigestion, reflux, constipation, and vomiting, and the patient is experiencing at least one CeD PRO non-GI domain symptom, optionally wherein the CeD PRO non-GI domain symptom(s) are one or more of headache or tiredness.

6. The composition for the use of any one of claims 1 to 5, wherein the patient is experiencing dermatitis herpetiformis.

7. The composition for the use of any one of claims 1 to 6, wherein the patient is experiencing one or more CeD PRO Symptomatic days of at least 2, 3, 4, 5, 6, or 7,
wherein a CeD PRO Symptomatic days is a day where the mean of abdominal cramping, abdominal pain, bloating, and gas is scored as > 2.5 or > 3 out of a 0 to 10 scale, or a day where the mean of diarrhea and loose stool is scored as > 2.5 or > 3 out of a 0 to 10 scale, or a day where nausea is scored as > 2.5 or > 3 out of a 0 to 10 scale,
optionally wherein upon treatment the patient experiences one or more CeD PRO Improved Symptom days per week, wherein a CeD PRO Improved Symptom day is a day where the mean of abdominal cramping, abdominal pain, bloating, and gas is scored as < 1.5 out of a 0 to 10 scale, and a day where the mean of diarrhea and loose stool is scored as < 1.5 out of a 0 to 10 scale, and a day where nausea is scored as < 1 out of a 0 to 1 0 scale.

8. The composition for the use of any one of claims 1 to 7, wherein the patient scores at least 2, or at least 3, or at least 4, or at least 5, or at least 6 on the CeD Gastrointestinal Symptom Rating Scale (CeD GSRS) at the start of treatment using the 1-7 Likert scale.

9. The composition for the use of any one of claims 1 to 7, wherein the patient scores at least 2, or at least 3, or at least 4, or at least 5, or at least 6, or 7 on the Total Gastrointestinal Symptom Rating Scale (GSRS) at the start of treatment using the 1-7 Likert scale.

10. The composition for the use of any one of claims 1 to 9, wherein the patient experiences at least about 3, 4, 5, 6, 7, 8, 9, 10, or more bowel movements per day at the start of treatment, optionally wherein the patient experiences more than about 12, more than about 14, more than about 16, or more than about 18 bowel movements per day at the start of treatment.

11. The composition for the use of any one of claims 1 to 9, wherein the patient experiences at least about 4, at least about 5, at least about 6, at least about 7, or at least about 8, or at least about 9, or at least about 10 diarrhea or loose stools per day on the Bristol Form Scale (BSFS) at the start of treatment.

12. The composition for the use of any one of claims 1 to 9, wherein the patient experiences > 3 diarrhea or loose stools per day with a score of 5-7 as measured by the Bristol Form Scale (BSFS) at the start of treatment.

13. The composition for the use of any one of claims 1 to 12, wherein the composition is to be administered for at least about 9 weeks, or at least about 10 weeks, or at least about 12 weeks, optionally wherein the composition is to be administered for at least about six months or for at least about one year.

14. The composition for the use of any one of claims 1 to 13, wherein the composition is to be administered prior to meals, optionally wherein the composition is to be administered about 60 minutes, or about 45 minutes, or about 30 minutes, or about 15 minutes, or about 10 minutes, or about 5 minutes prior to meals.

15. The composition for the use of any one of claims 1 to 14, wherein the composition is a delayed release composition that releases Larazotide in the duodenum and jejunum.

16. The composition for the use of any one of claims 1 to 15, wherein the composition further releases Larazotide in the ileum and/or the colon.


SEQUENCE LISTING

<110>  ALBA THERAPEUTICS CORPORATION

<120>  METHODS OF TREATING CELIAC DISEASE WITH LARAZOTIDE

<130>  237-034T1

<140>
<141>  2015-04-03

<150>  EP 15 772 716.5
<151>  2015-04-03

<150>  PCT/US2015/024313
<151>  2015-04-03

<150>  US 61/975,128
<151>  2014-04-04

<160>  1

<170>  PatentIn version 3.5

<210>  1
<211>  8
<212>  PRT
<213>  Artificial Sequence

<220>
<223>  Synthetic peptide

<400>  1

Gly Gly Val Leu Val Gln Pro Gly
1               5